# EUROPÄISCHE PATENTSCHRIFT

(11) **EP 3 900 680 B1**
(45) Veröffentlichungstag und Bekanntmachung des Hinweises auf die Patenterteilung: **29.06.2022**
(21) Anmeldenummer: 20171303.9
(22) Anmeldetag: 24.04.2020
(51) Int. Cl.: A61F 2/36, A61F 2/40, A61F 2/46, B29C 33/30, B29C 33/00, B29C 33/40, B29L 31/00, A61F 2/30, A61B 17/56

(54) **VORRICHTUNG UND VERFAHREN ZUR HERSTELLUNG VON SPACERN MIT VARIABLEM KOPF**
DEVICE AND METHOD FOR PRODUCING SPACERS WITH VARIABLE HEAD
DISPOSITIF ET PROCÉDÉ DE FABRICATION D'ENTRETOISES À TÊTE VARIABLE

(43) Veröffentlichungstag der Anmeldung: 27.10.2021
(73) Patentinhaber: Heraeus Medical GmbH, 61273 Wehrheim (DE)
(72) Erfinder: VOGT, Sebastian Dr., 61273 Wehrheim (DE); KLUGE, Thomas Dr., 61273 Wehrheim (DE)
(74) Vertreter: Schultheiss & Sterzel Patentanwälte PartG mbB

(56) Entgegenhaltungen:
- EP-A1- 2 522 310
- EP-A1- 2 617 393
- EP-A1- 2 787 928
- EP-B1- 2 617 393
- EP-B1- 2 787 928
- WO-A1-2018/203150
- US-A1- 2013 344 186

## Beschreibung

Die Erfindung betrifft eine Vorrichtung zum Herstellen eines Spacers durch Aushärten von Knochenzementteig wie in Anspruch 1 definiert. Zusätzliche Merkmale sind in den abhängigen Ansprüchen definiert. Der Spacer ist als temporärer Platzhalter dazu vorgesehen, im medizinischen Anwendungsbereich ein Gelenk oder einen Teil eines Gelenks umfassend eine artikulierende Oberfläche eines Gelenkkopfs temporär zu ersetzen. Bevorzugt ist der Spacer zum temporären Ersetzen eines Hüftgelenks oder eines Schultergelenks geeignet und vorgesehen. Dementsprechend ist die Vorrichtung bevorzugt zum Herstellen eines Hüftgelenkspacers oder eines Schultergelenkspacers vorgesehen. Die Erfindung betrifft auch ein Verfahren zur Herstellung eines solchen Spacers mit einer solchen Vorrichtung. Das erfindungsgemäße Verfahren ist in Anspruch 13 definiert. Zusätzliche Merkmale sind in den abhängigen Ansprüchen definiert.

Gegenstand der Erfindung ist somit eine Gießform zur Herstellung von einteiligen Knochenzementspacern, insbesondere zur Herstellung von einteiligen Hüft- und Schulterspacern, wobei der Durchmesser der Spacerkopfs der zu gießenden Hüft- und Schulterspacer erfindungsgemäß stufenlos entsprechend den anatomischen Verhältnissen des jeweiligen Patienten einstellbar ist. Hüft- und Schulterspacer sind als temporäre Platzhalter (Spacer) im Rahmen von zweizeitigen Revisionen von infizierten Hüft- und Schultertotalgelenkendoprothesen für die Interimsphase bestimmt. Die Gießform ist für die Herstellung von Hüft- und Schulterspacern mit niedrigviskosem und hochviskosem Polymethylmethacrylat-Knochenzementteig geeignet. Weitere Gegenstände der Erfindung sind Verfahren zur Herstellung von Hüft- und Schulterspacern mit einem stufenlos einstellbaren Durchmesser des Spacerkopfs.

Gelenk-Endoprothesen, wie Hüftgelenk-Endoprothesen und Schultergelenk-Endoprothesen, werden in großem Umfang weltweit implantiert. Leider kommt es in einem geringen Prozentsatz vor, dass Gelenk-Endoprothesen von mikrobiellen Keimen, besonders Grampositiven Bakterien als auch Gram-negativen Bakterien und in sehr geringem Umfang von Hefen und Pilzen, besiedelt werden. Diese mikrobiellen Keime, hauptsächlich typische Hautkeime wie Staphylococcus aureus und Staphylococcus epidermidis, können während einer chirurgischen Operation (OP) in den Patienten gelangen. Daneben ist es auch möglich, dass mikrobielle Keime hämatogen Gelenk-Endoprothesen erreichen. Bei einer Besiedlung von Gelenk-Endoprothesen mit mikrobiellen Keimen wird das umliegende Knochen- und Weichgewebe mit infiziert und von den mikrobiellen Keimen geschädigt.

Stand der Technik sind vor allem zwei Behandlungsverfahren für infizierte Gelenk-Endoprothesen, die einzeitige septische Revision und die zweizeitige septische Revision. Bei der einzeitigen Revision wird innerhalb einer OP zuerst die infizierte Gelenk-Endoprothesen entfernt, anschließend radikal debridiert und dann wird eine Revisions-Gelenk-Endoprothese implantiert.

Bei zweizeitigen septischen Revisionen wird in einer ersten OP zuerst die infizierte Gelenk-Endoprothese entfernt, dann wird debridiert und anschließend erfolgt die Implantation eines Spacers. Ein Hüftgelenkspacer besteht aus einem Schaft, einem Kragen, einem Hals und einem Kugelkopf und ist den Hüftgelenk-Endoprothesen in Form und Größe nachgebildet. Analog ist ein Schultergelenkspacer einer Schultergelenk-Endoprothesen in Form und Größe nachgebildet. Der Spacer wird mit Knochenzement an dem jeweiligen Knochen verankert, also beispielsweise bei Hüftgelenkspacern am proximalen Femur beziehungsweise im Femurkanal verankert. Der Spacer verbleibt bis zu mehreren Wochen im Patienten bis die Entzündung abgeklungen ist und die klinischen Entzündungsmarker zurückgegangen sind. Dann wird in einer zweiten OP der Spacer entfernt und nach erneutem Debridement eine Revisions-Gelenk-Endoprothese implantiert.

Im Rahmen von zweizeitigen septischen Wechseloperationen von Hüft- und Schultertotalgelenkendoprothesen sind Spacer als temporäre Platzhalter in der Interimsphase von wesentlicher Bedeutung. Bei der intraoperativen Herstellung dieser Spacer kann vom medizinischen Personal je nach vorliegenden Antibiogramm der für die Infektionen ursächlichen mikrobiellen Keime ein oder mehrere speziell auf die Keime abgestimmtes Antibiotikum oder mehrere Antibiotika dem Polymethylmethacrylat-Knochenzement zugesetzt werden.

Bei Spacern werden dem Zementpulver vor der eigentlichen Spacerherstellung Antibiotika zugesetzt. Mit diesem antibiotisch modifizierten Knochenzementpulver wird anschließend durch Beimischen von Monomerflüssigkeit ein Knochenzementteig hergestellt und aus diesem Knochenzementteig werden Spacer gegossen, die dann durch Polymerisation mit Hilfe der dem Zementpulver zugesetzten Monomerflüssigkeit aushärten. Der Knochenzementteig schließt dabei die Antibiotika im Wesentlichen ein. Die in den oberflächennahen Bereichen befindlichen Antibiotika-Partikel werden bei Einwirkung von Körperflüssigkeiten, wie Wundsekret, herausgelöst. Die Wirkstofffreisetzung ist initial am höchsten und nimmt dann im Verlauf von mehreren Tagen ab.

Die US 2010/0042213 A1 offenbart eine Hüftgelenkprothese mit einem Reservoir einer Flüssigkeit im Inneren des Implantats. Aus der WO 2017/178951 A1 ist ein Hüftspacer mit Vertiefungen bekannt, wobei in den Vertiefungen eine Substanz zur Behandlung des Knochens eingebracht werden kann. In dem Patent US 6 245 111 B1 wird eine Hüftgelenksprothese vorgeschlagen, deren Oberflächen mit einem Antibiotikum beschichtet sind. Das Patent US 5 681 289 offenbart eine Einrichtung zum Verteilen eines flüssigen Wirkstoffs mit Hilfe einer Blase im Inneren der Einrichtung. Keine der genannten Prothesen ist zum Erzeugen eines Spülkreislaufs geeignet. In der EP 1 991 170 B1 und der US 2011/0015754 A1 werden ein Hüftgelenkspacer beschrieben, die Wirkstoffe enthalten. Die US 2019/0290833 A1 offenbart einen spülbaren Hüftgelenkspacer, mit dem ein Flüssigkeitskreislauf erzeugbar ist. In der WO 2016/205077 A1 und der US 8 900 322 B2 werden weitere Spacer mit einer Spülfunktion beschrieben.

Es ist bekannt, mit Antibiotika ausgerüstete Spacer zu verwenden. Spacer können einerseits vom OP-Personal während der OP selbst aus PMMA-Knochenzementpulver, Antibiotika und Monomerflüssigkeit hergestellt werden, zum Beispiel mit einer Spacerform, wie sie beispielsweise in den Patenten DE 10 2015 104 704 B4 oder EP 2 617 393 B1 beschrieben sind, und andererseits ist es auch üblich, industriell aus Knochenzement vorgefertigte Hüftgelenkspacer einzusetzen.

Für die intraoperative Herstellung von Spacern mit Polymethylmethacrylat-Knochenzement sind Kunststoffgießformen üblich. Kunststoffgießformen zur intraoperativen Herstellung von einteiligen Hüftspacern werden in der US 6 361 731 B1 beschrieben. Diese Gießformen sind transparent und haben zwei separate Einfüllöffnungen. Dadurch kann auch hochviskoser Knochenzementteig mit geringem Druck in die Gießform eingebracht werden, weil die Fließwege des Knochenzementteigs relativ kurz sind. Bei der Verwendung von nicht hochviskosem Knochenzementteig besteht die Gefahr, dass Knochenzementteig nach erfolgter Füllung der Gießform vor der einsetzenden Aushärtung wieder aus den Einfüllöffnungen herausfließt. Diese Gießformen werden mit unterschiedlichen Spacerkopfdurchmessern angeboten. Der Durchmesser des Spacerkopfs ist nicht variabel einstellbar. Der medizinische Anwender kann nur zwischen vorgegebenen Spacerkopfgrößen auswählen. Wünschenswert wäre es, wenn der medizinische Anwender möglichst mit einer Gießform für den Schaft zwischen verschiedenen Spacerkopfgrößen wählen könnte.

In einer Weiterentwicklung wurden in den Patentschriften US 7 789 646 B2, US 8 480 389 B2 und US 8 801 983 B2 mehrteilige Gießformen für die Herstellung modularer Hüftspacer vorgeschlagen. Diese modularen Hüftspacer bestehen aus einem Spacerkopf und einem separaten Schaft. Es stehen dazu Gießformen für den Spacerkopf mit unterschiedlichen Spacerkopfdurchmessern zur Verfügung. Das bedeutet, die Gießform des Schafts wird mit der Gießform des Spacerkopfs, welche den ausgewählten Durchmesser besitzt, verbunden. Die so zusammengesetzte Gießform ist dann einteilig und besitzt an der Einfüllöffnung ein Gewinde zur Verbindung der Gießform mit einer Zementkartusche. In einer anderen Variante gemäß der US 7 637 729 B2 werden eine Gießform für die Herstellung des Schafts und eine separate Gießform für den Spacerkopf verwendet. Nach erfolgter Aushärtung und Entformung werden die beiden Spacerkomponenten zusammengesteckt. Im Patent US 7 789 646 B2 wird eine modulare Gießform beschrieben, bei der mit einem Stöpsel die Einfüllöffnung der Gießform verschlossen werden kann, wenn der Zementteig in die Gießform eingebracht wurde. Zuvor muss jedoch die Gießform von der Zementkartusche abgeschraubt werden. Bei Verwendung von niedrigviskosem Zementteig ist es daher möglich, wenn die Gießform ungünstig gehalten wird, dass Zementteig während der Trennung der Gießform von der Zementkartusche ausläuft, bevor der Stöpsel eingeschraubt ist. Bei Verwendung von nicht hochviskosem Knochenzementteig ist es daher möglich, wenn die Gießform ungünstig gehalten wird, dass Knochenzementteig während der Trennung der Gießform von der Knochenzementkartusche ausläuft, bevor der Stöpsel eingeschraubt beziehungsweise eingesteckt ist. In jedem Fall können ungewollt Lufteinschlüsse in der Spacerform dadurch entstehen, dass sich der Knochenzementteig von der Innenwandung der Gießform löst.

In der US 2007/0222114 A1 wird eine Hüftspacerform beschrieben. Diese Spacerform besteht aus einer Vielzahl von Formsegmenten, die miteinander verbunden werden. Durch die Vielzahl der Segmente kann die Spacerform recht genau an die anatomischen Gegebenheiten des Patienten angepasst werden. Die Spacerform-Segmente werden mittels Schraubschellen aneinandergefügt. Durch Kanäle in der Spacerform wird ein PMMA-Knochenzementteig (Polymethylmethacrylat-Knochenzementteig) eingeführt. Durch den komplexen Aufbau der Gießform ist es sehr aufwändig, die Spacerform-Segmente zusammenzufügen und nach erfolgter Aushärtung des PMMA-Knochenzementteigs den Hüftspacer zu entnehmen.

In der WO 2009/073 781 A2 wird eine Spacerform für einen Hüftspacer vorgeschlagen, die aus zwei Teilen besteht, die in Bezug zueinander verschoben werden können, um die Länge des Schafts anpassen zu können. Eine weitere Gießform ist in der EP 2 522 310 A1 offenbart. Diese Vorrichtung besteht aus wenigstens zwei Teilen, wobei in einem ersten Teil eine Einschubabschnitt und im zweiten Teil eine Einschubaufnahme angeordnet sind. Beide Teile sind in einander steckbar und bilden eine Gießform für die Herstellung des Schafts des Hüftspacers. In der EP 2 787 928 A1 wird eine komplexe Gießform beschrieben. Diese ermöglicht die Herstellung von Hüftspacern mit unterschiedlichen Kugelköpfen. Die Fixierung der Elemente der Gießform erfolgt über Verbindungselemente.

Die Aufgabe der vorliegenden Erfindung besteht also darin, die Nachteile des Stands der Technik zu überwinden. Insbesondere besteht die Aufgabe der Erfindung in der Entwicklung einer kostengünstigen Vorrichtung zum Herstellen eines einteiligen Spacers mit variablem Spacerkopf durch Aushärten von Knochenzementteig und in der Entwicklung eines einfach und kostengünstig durchführbaren Verfahrens zum Herstellen eines einteiligen Spacers mit variablem Spacerkopf durch Aushärten von Knochenzementteig, mit dem vom medizinischen Personal im OP unter Verwendung von Knochenzementteig, insbesondere von Polymethylmethacrylat-Knochenzement, einteilige Spacer, insbesondere Hüft- und Schulterspacer, hergestellt werden können. Hüft- und Schulterspacer sind ähnlich aufgebaut. Sie bestehen aus einem Schaft und einem Spacerkopf sowie einem Hals, der den Schaft und den Spacerkopf verbindet.

Daher soll eine Vorrichtung aufweisend eine Gießform entwickelt werden, die grundsätzlich für die Herstellung von Hüft- und Schulterspacern mit variablen Spacerkopfdurchmessern geeignet ist. Ein Metallkern kann zur mechanischen Stabilisierung im Inneren des Hüft- und Schulterspacer angeordnet sein beziehungsweise werden. Es soll möglich sein, sowohl mit (Polymethylmethacrylat-)Knochenzementteig niedriger beziehungsweise nicht hoher Viskosität als auch mit hoher Viskosität Spacer herzustellen. Für die vollständige Füllung einer Gießform mit einem hochviskosem Knochenzementteig ist ein hoher Einpressdruck erforderlich. Die Gießform der Vorrichtung soll mit dem hohen Einpressdruck einsetzbar sein. Die Gießform soll daher möglichst einem Druck von 10 N/cm² zum einen standhalten und zum anderen mit einem solchen Druck bedienbar sein.

Die Vorrichtung und insbesondere die Gießform der Vorrichtung soll so beschaffen sein, dass Knochenzementteig beziehungsweise fluider Knochenzementteig aus einer Knochenzementkartusche in eine Gießform eingepresst werden kann. Bei Verwendung von nicht hochviskosem Knochenzementteig soll nach Füllung der Gießform der Knochenzementteig nicht aus der Gießform ausfließen. Dazu ist es notwendig, die Gießform so zu gestalten, dass ein Auslaufen von nicht hochviskosem Knochenzementteig aus der Gießform während der Trennung der Gießform von der Zementkartusche sicher verhindert wird. Dieses Verschließen soll möglich sein, ohne dass in der Wandung der Gießform Öffnungen für komplex aufgebaute Ventile notwendig sind. Öffnungen in der Wandung der Gießform können zur Undichtigkeit der Gießform führen, wenn der Knochenzementteig unter hohem Druck in die Gießform eingepresst wird. Weiterhin soll der Angussbereich der Gießform so gestaltet sein, dass einerseits eine leichte Befüllung der Gießform mit Knochenzementteig möglich ist und dass andererseits nach erfolgter Aushärtung des Knochenzementteigs die Angussreste leicht entfernt werden können.

Die Aufgaben der Erfindung werden gelöst durch eine Vorrichtung zum Herstellen eines Spacers durch Aushärten von Knochenzementteig, wobei der Spacer dazu vorgesehen ist, im medizinischen Bereich ein Gelenk oder einen Teil eines Gelenks umfassend eine artikulierende Oberfläche eines Kopfs des Gelenks temporär zu ersetzen, insbesondere eines Hüftgelenks oder eines Schultergelenks temporär zu ersetzen, die Vorrichtung aufweisend eine Gießform zum Formen des Spacers aus Knochenzementteig, wobei die Gießform eine Schaftform zum Formen eines Schafts und eines Halses aufweist und die Gießform eine Hohlform mit einer kugelflächenförmigen Innenfläche zum Formen einer Gleitfläche eines Kopfs des Spacers aufweist, wobei die Schaftform und die Hohlform einen gemeinsamen Innenraum begrenzen, so dass der Kopf eines mit der Gießform geformten Spacers über den Hals mit dem Schaft einteilig verbunden ist,
wenigstens eine Einfüllöffnung zum Einpressen eines Knochenzementteigs in die Gießform,
wenigstens ein Entlüftungselement, das den gemeinsamen Innenraum der Gießform gasdurchlässig mit der Umgebung der Gießform verbindet,
wobei die Schaftform bei einem Einpressen von Knochenzementteig in die Gießform formstabil ist, während die Hohlform bei dem Einpressen von Knochenzementteig in die Gießform zumindest im Bereich der kugelflächenförmigen Innenfläche durch einen vom eingepressten Knochenzementteig vermittelten Druck expandierbar ist.

Bevorzugt weist die kugelflächenförmige Innenfläche eine radiale Abweichung von einer perfekten Kugeloberfläche von höchstens 10% auf, besonders bevorzugt von höchstens 1%. Ferner kann die kugelflächenförmige Innenfläche ein Kugelflächensegment umfassen, das einen Winkelausschnitt von zumindest 45° umfasst, bevorzugt von zumindest 90° umfasst, besonders bevorzugt zumindest eine Halbkugel umfasst. Eine Halbkugel hat einen Winkelausschnitt von 180°.

Es kann vorgesehen sein, dass die kugelflächenförmige Innenfläche eine halbkugelflächenförmige Innenfläche ist.

Der Durchmesser der nicht expandierten kugelflächenförmigen Innenfläche der Hohlform kann zwischen 10 mm und 60 mm betragen, vorzugsweise zwischen 30 mm und 50 mm.

Der Kopf des Gelenks, der zumindest partiell, insbesondere vollständig, von dem Spacer ersetzt werden soll, ist vorzugsweise ein Femurkopf oder ein Humeruskopf.

Die Schaftform kann zweiteilig aufgebaut sein, wobei die beiden Teile der Schaftform miteinander verbunden oder miteinander verbindbar sind, wobei vorzugsweise die beiden Teile der Schaftform fluiddicht miteinander verbunden oder verbindbar sind. Dies gilt gegebenenfalls bis auf die wenigstens eine Einfüllöffnung, die von beiden Teilen der Schaftform begrenzt sein kann.

Alternativ kann die Schaftform auch einteilig sein.

Unter einem Knochenzementteig beziehungsweise einem fluiden Knochenzementteig ist ein gemischter (also zum Einsatz bereiter) Knochenzementteig zu verstehen, der eine zähflüssige Konsistenz hat. Die Viskosität eines Knochenzements entspricht vorzugsweise der von Honig oder hat eine noch zähflüssigere Konsistenz, das heißt eine noch höhere Viskosität. Die Begriffe fluider Knochenzement und Knochenzementteig werden synonym verwendet.

Die Gießform ist innen in Bezug auf den Hohlraum und den Innenraum hohl.

Es kann bevorzugt auch vorgesehen sein, dass die Hohlform einen Innenraum aufweist, der eine Negativform eines Gelenkkopfs nachbildet, insbesondere eines Hüftgelenkkopfs oder Schultergelenkkopfs nachbildet.

Es kann ferner vorgesehen sein, dass die Gießform zweiteilig, dreiteilig, vierteilig oder mehrteilig ist, wobei vorzugsweise die Teile der Gießform über Flansche und/oder Gewinde flüssigkeitsdicht aneinander befestigbar sind. Besonders bevorzugt ist die Gießform zweiteilig (Hohlform und Schaftform), dreiteilig (Hohlform und zweiteilige Schaftform) oder vierteilig (Hohlform, zweiteilige Schaftform und einen in der Zylinderachse verstellbaren Hohlzylinder als Adapterelement zum Formen eines Halses des Spacers).

Die Gießform soll einem Druck von 10 N/cm² standhalten, um auch hochviskosen Knochenzementteig verwenden zu können.

Vorzugsweise soll die Hohlform mit einem hydrostatischen Druck von zumindest 1 N/cm² expandierbar sein, besonders bevorzugt mit einem hydrostatischen Druck von zumindest 5 N/cm² expandierbar sein.

In der vorliegenden Patentanmeldung werden die Richtungsbezeichnungen "proximal", "distal" und "lateral" sowie die Ebenen-bezeichnungen "Sagittalebene", "Frontalebene" und "Transversalebene" in Bezug auf den Spacer oder die Gießform so verwendet, wie dies bei dem im Patienten eingesetzten Zustand als anatomische Hauptrichtung oder als Körperebene zu verstehen wäre. Dabei bedeutet "proximal" zum Körperzentrum hin und "distal" vom Körperzentrum entfernt.

Der Schaft ist zur Verbindung mit einem Knochen (im Fall von Hüftgelenkspacern mit dem Femur und im Fall von Schultergelenkspacern mit dem Humerus) vorgesehen und ist vorzugsweise zu diesem Zweck in ein proximales Ende des präparierten Knochens beziehungsweise in den Knochenkanal einführbar.

Bevorzugt kann vorgesehen sein, dass die Vorrichtung zur Herstellung eines Spacers, insbesondere eines Hüftgelenkspacers oder Schultergelenkspacers, zur Anwendung zumindest eines antibiotischen und/oder antimykotischen Wirkstoffs geeignet ist.

Bevorzugt ist der Spacer einteilig aus einem biokompatiblen Knochenzementteig, wie Polymethylmethacrylat (PMMA), zu fertigen, wobei besonders bevorzugt das PMMA wenigsten ein aus dem PMMA lösbares Antibiotikum und/oder Antimykotikum enthält.

Durch die wenigstens eine Entlüftungsöffnung kann Luft oder Gas aus dem Inneren der Gießform entweichen, wenn der Knochenzementteig eingefüllt wird. Dadurch können Lufteinschlüsse und dadurch Unebenheiten an der Oberfläche des Spacers vermieden werden und gleichzeitig ein gleichmäßig wirkender Druck des Knochenzementteigs im Inneren der Hohlform sichergestellt werden.

Die Vorrichtung kann durch die Gießform selbst realisiert sein.

Es kann vorgesehen sein, dass das wenigstens eine Entlüftungselement für Gase durchlässig ist und für Knochenzementteig undurchlässig ist, insbesondere für Polymethylmethacrylat-Knochenzementteig (PMMA-Knochenzementteig) undurchlässig ist.

Es kann auch vorgesehen sein, dass das wenigstens eine Entlüftungselement in der Hohlform angeordnet ist oder das wenigstens eine Entlüftungselement mehrere Entlüftungselemente sind, wobei zumindest eines der mehreren Entlüftungselemente in der Hohlform angeordnet ist und zumindest eines der mehreren Entlüftungselemente in der Schaftform angeordnet ist.

Hierdurch wird sichergestellt, dass sich in der Hohlform keine Lufteinschlüsse bilden, die einen Teil des Drucks des Knochenzementteigs aufnehmen können und beim Aushärten expandieren können, was zu einer Veränderung der gewünschten Größe und Form der Gleitfläche führen würde. Zudem wird die Gleitfläche durch den Lufteinschluss unterbrochen und muss nach dem Aushärten gefüllt beziehungsweise repariert werden.

Gemäß einer Weiterbildung kann vorgesehen sein, dass die Hohlform zumindest im Bereich der kugelflächenförmigen Innenfläche durch den vom eingepressten Knochenzementteig vermittelten Druck radial expandierbar ist, bevorzugt radial und gleichförmig expandierbar ist.

Ferner kann vorgesehen sein, dass die Hohlform zumindest im Bereich der kugelflächenförmigen Innenfläche durch den vom eingepressten Knochenzementteig vermittelten Druck elastisch expandierbar ist, bevorzugt gummielastisch expandierbar ist.

Hierdurch kann eine gleichmäßige und stufenlose Einstellung der Größe der Gleitfläche beziehungsweise des Kopfs des Spacers ermöglicht werden. Damit kann der Kopf des Spacers besonders einfach an die für einen Patienten geeignete Behandlungssituation angepasst werden.

Unter dem Begriff "gummielastisch" wird verstanden, dass die kugelflächenförmige Innenfläche der Hohlform durch Druckbeaufschlagung auf die innere Oberfläche durch Polymethylmethacrylat-Knochenzementteig ohne Zerreißen der Wandung der Hohlform durch Dehnung der Wandung der Hohlform zumindest im Bereich der kugelflächenförmigen Innenfläche um den Faktor 3 vergrößert werden kann. Bevorzugt wird unter dem Begriff "gummielastisch" verstanden, dass das Volumen der kugelflächenförmigen Innenfläche der Hohlform durch Druckbeaufschlagung auf die innere Oberfläche durch Polymethylmethacrylat-Knochenzementteig durch Dehnung der Wandung der Hohlform ohne Zerreißen der Wandung der Hohlform im Bereich der kugelflächenförmigen Innenfläche mindestens um den Faktor 5,5 vergrößert werden kann.

Des Weiteren kann vorgesehen sein, dass die Schaftform und die Hohlform über einen Flansch oder ein Adapterelement miteinander flüssigkeitsdicht verbunden oder verbindbar sind, wobei bevorzugt ein expandierbarer Teil der Hohlform, der die kugelflächenförmige Innenfläche umfasst, mit einer ringförmigen Halterung auf einem Flansch der Schaftform oder des Adapterelements befestigt oder befestigbar ist, so dass eine randseitige Ringscheibe des expandierbaren Teils zwischen der ringförmigen Halterung und dem Flansch angeordnet ist und die Verbindung abdichtet, wobei besonders bevorzugt die ringförmige Halterung mit dem Flansch der Schaftform verschraubt oder verschraubbar ist.

Hierdurch können die Schaftform und die Hohlform, die unterschiedliche mechanische Eigenschaften aufweisen müssen, aneinander flüssigkeitsdicht befestigt werden, so dass der Knochenzementteig nicht zwischen der Schaftform und der Hohlform austreten kann.

Flüssigkeitsdicht bedeutet, dass der nicht ausgehärtete also fluide Knochenzementteig, und bevorzugt auch eine flüssige Monomerflüssigkeit als Ausgangskomponente des Knochenzements, nicht zwischen der Hohlform und der Schaftform hindurch ausfließen oder hindurchdringen können. Allgemein soll flüssigkeitsdicht vorliegend so verstanden werden, dass zumindest der Knochenzementteig nicht durch eine flüssigkeitsdichte Verbindung austreten kann.

Es kann vorgesehen sein, dass das Adapterelement in einem Winkel zwischen 80° und 100° relativ zu einer Schaftachse des Schafts der Schaftform absteht, bevorzugt in einem Winkel von 90° relativ zu einer Schaftachse des Schafts der Schaftform absteht.

Die Hohlform besitzt vorzugsweise einen umlaufenden Rand, der zwischen dem Adapterelement und einem Befestigungselement angeordnet ist oder anzuordnen ist.

Auch kann vorgesehen sein, dass die Vorrichtung ein Befestigungselement zur Befestigung der Hohlform an der Schaftform aufweist, wobei bevorzugt das Befestigungselement lösbar ist und besonders bevorzugt das Befestigungselement mehrere Schrauben sind oder mehrere Schrauben aufweist.

Hierdurch kann die Hohlform and der Schaftform befestigt werden, um die Gießform für den Spacer bereitzustellen. Damit können auch unterschiedliche Hohlformen, die sich in ihrer Form voneinander unterscheiden, an der Schaftform befestigt werden oder umgekehrt unterschiedliche Schaftformen an einer Hohlform befestigt werden. Hiermit kann eine noch größere Variabilität der Vorrichtung erreicht werden. Die Hohlformen können sich beispielsweise durch eine mehr oder weniger von der idealen Kugelform abweichende Form voneinander unterscheiden. Die Schaftformen können sich beispielsweise durch unterschiedliche Längen und/oder Durchmesser des Schafts voneinander unterscheiden.

Es kann vorgesehen sein, dass das Befestigungselement zumindest teilweise mit der Schaftform verbunden ist.

Des Weiteren kann vorgesehen sein, dass das Befestigungselement Klemmplatten aufweist, wobei bevorzugt das Befestigungselement zusätzlich Schrauben, Flügelschrauben, Muttern und/oder Gewindestangen aufweist.

Hiermit lässt sich auf konstruktiv einfache Weise eine dichte Verbindung zwischen der Schaftform und der Hohlform erzeugen.

Als Schrauben werden besonders Torx-Schrauben bevorzugt, die mit in den OPs üblichen Antriebsvorrichtungen geschraubt werden können.

Ferner kann vorgesehen sein, dass die wenigstens eine Einfüllöffnung auf einer von der Gießform abgewandten Seite mit einem Anschluss zum flüssigkeitsdichten Verbinden einer Knochenzementkartusche verbunden ist, wobei bevorzugt der Anschluss zum druckdichten Verbinden einer Knochenzementkartusche geeignet ist, wobei besonders bevorzugt der Anschluss ein Gewinde aufweist und ganz besonders bevorzugt ein umlaufende Dichtung und/oder eine umlaufende Dichtfläche oder eine umlaufende Dichtkante aufweist.

Hiermit wird sichergestellt, dass der Knochenzementteig aus einer Knochenzementkartusche in die Gießform eingepresst werden kann und mit der Knochenzementkartusche über den Knochenzentteig ein Druck auf die Hohlform ausgeübt werden kann, um die Hohlform zu expandieren.

Es kann auch vorgesehen sein, dass sich die Hohlform zumindest im Bereich der kugelflächenförmigen Innenfläche durch Einpressen von weiterem Knochenzementteig in die bereits vollständig mit Knochenzementteig gefüllte Gießform ausdehnt, während die Schaftform keinen zusätzlichen Knochenzementteig aufnimmt und formstabil bleibt.

Hiermit wird sichergestellt, dass nur der Kopf des Spacers durch das Einpressen von Knochenzementteig umgeformt wird.

Dass die Schaftform keinen zusätzlichen Knochenzementteig aufnimmt soll nicht bedeuten, dass sie sich nicht elastisch verformen kann.

Zumindest 95% der Volumenzunahme der Gießform durch Einpressen von Knochenzementteig soll erfindungsgemäß bevorzugt in der Hohlform stattfinden, besonders bevorzugt zumindest 99% der Volumenzunahme.

Gemäß einer besonders bevorzugten Weiterbildung der vorliegenden Erfindung kann vorgesehen sein, dass die Vorrichtung ferner aufweist
einen Ventilsitz, der im Bereich der wenigstens einen Einfüllöffnung mit der Gießform verbunden ist, wobei der Ventilsitz eine bereichsweise geschlossene Kopfseite mit mindestens einer ersten Durchführung aufweist, wobei die mindestens eine erste Durchführung in die wenigstens eine Einfüllöffnung mündet,
einen Ventilkörper, der drehbar gegen den Ventilsitz gelagert ist und der eine Dichtfläche aufweist, wobei die Dichtfläche in Richtung der bereichsweise geschlossenen Kopfseite des Ventilsitzes ausgerichtet ist, wobei in der Dichtfläche mindestens eine zweite Durchführung angeordnet ist,
wobei der Ventilsitz und der Ventilkörper zusammen ein Ventil bilden, wobei das Ventil durch Drehen des Ventilkörpers gegen den Ventilsitz reversibel in eine geöffnete Stellung und reversibel in eine geschlossene Stellung überführbar ist, wobei in der geöffneten Stellung des Ventils die mindestens eine erste Durchführung des Ventilsitzes und die mindestens eine zweite Durchführung des Ventilkörpers zumindest bereichsweise übereinanderliegen und eine für Knochenzementteig durchlässige Verbindung durch das Ventil in die Gießform bereitstellen, wobei in der geschlossenen Stellung des Ventils die mindestens eine erste Durchführung des Ventilsitzes durch die Dichtfläche des Ventilkörpers abgedeckt ist, wobei die wenigstens eine Einfüllöffnung der Gießform in der geschlossenen Stellung des Ventils für Knochenzementteig abgedeckt ist.

Durch das Ventil gelingt es auf konstruktiv besonders einfache Weise, dass der Knochenzementteig abgetrennt werden kann und gleichzeitig der Knochenzementteig innerhalb der Gießform unter Druck gehalten werden kann, um die Hohlform in einem expandierten Zustand zu halten. Das Ventil ermöglicht es also, den Anguss abzutrennen, während gleichzeitig über den Knochenzementteig ein Druck auf die Hohlform aufrechterhalten werden kann, um die Hohlform in der gewünschten Ausdehnung zu halten. Durch einen in einem Ventilsitz drehbaren Ventilkörper gelingt es überraschend auch, eine Vorrichtung mit einer Gießform bereitzustellen, bei der der Anguss mit dem Ventilkörper abgeschert beziehungsweise weitgehend abgeschert werden kann und gleichzeitig bei bestehendem Druck aus einer Knochenzementkartusche die Gießform derart zu verschließen oder die verbleibenden Kanäle zumindest so weit einzuschnüren, dass der Knochenzementteig in der Gießform weiter unter Druck gehalten werden kann, um sich an die Innenseiten der Gießform anzupressen und um die Hohlform expandiert zu halten, wobei gleichzeitig verhindert wird, dass Knochenzementteig aus der Gießform durch die wenigstens eine Einfüllöffnung wieder herausgedrückt wird. Die Vorrichtung ermöglicht auch, die Gießform mit den Inhalten mehrerer Knochenzementkartuschen nacheinander zu befüllen, ohne dass der Knochenzementteig wieder durch die wenigstens eine Einfüllöffnung aus der Gießform herausfließen kann. Hierdurch gelingt es, auch mit Knochenzementkartuschen, die nur geringe Volumina des Knochenzements bereitstellen, Spacer mit großem Volumen herzustellen.

Für Knochenzementteig abgedeckt bedeutet, dass der Knochenzementteig im Ventil zumindest derart stark am Fließen gehindert wird, dass er nicht durch das Ventil hindurchfließen kann, bevor er aushärtet. Hierzu reicht es für normal viskose Knochenzementteige bereits aus, wenn der Knochenzementteig nicht in gerader Linie durch das Ventil strömen kann und die freien Leitungsquerschnitte kleiner als 1 mm sind. Knochenzementteige sind viskose oder hochviskose Fluide, wie durch den Zusatz "Teig" angedeutet. Die Viskosität eines Knochenzementteigs beträgt mindestens 10 Pa s, was der Viskosität von flüssigem Honig entspricht. Zudem härtet der Knochenzementteig innerhalb von wenigen Minuten aus, so dass anschließend kein Durchtritt mehr möglich ist. Es kann bevorzugt vorgesehen sein, dass der Knochenzementteig eine Viskosität von mindestens 10 Pa s aufweist.

Es kann vorgesehen sein, dass die Dichtfläche bis auf die mindestens eine zweite Durchführung geschlossen ist.

Es kann auch vorgesehen sein, dass der Ventilsitz flüssigkeitsundurchlässig mit einer Gießformwand der Gießform verbunden ist.

Ferner kann vorgesehen sein, dass der Ventilsitz an einer Stirnseite eines durch die Gießform begrenzten Hohlraums als Scheibe ausgebildet ist, insbesondere als ebene Scheibe ausgebildet ist.

Bevorzugt kann auch vorgesehen sein, dass der Ventilsitz und der Ventilkörper hohlzylinderförmig sind.

Die Begriffe "geöffneter Zustand" und "geschlossener Zustand" des Ventils beziehungsweise des Ventilkörpers relativ zum Ventilsitz und die Begriffe "geöffnete Stellung" und "geschlossene Stellung" des Ventils beziehungsweise des Ventilkörpers relativ zum Ventilsitz werden synonym verwendet.

Es kann bevorzugt vorgesehen sein, dass die bereichsweise geschlossene Kopfseite des Ventilsitzes und die Dichtfläche des Ventilkörpers Scheiben sind oder scheibenförmig sind.

Es kann bevorzugt vorgesehen sein, dass der Ventilsitz die wenigstens eine Einfüllöffnung der Gießform begrenzt.

Bei erfindungsgemäßen Vorrichtungen mit Ventil kann vorgesehen sein, dass das Ventil auf der von der Gießform abgewandten Seite mit einem Anschluss zum flüssigkeitsdichten Verbinden einer Knochenzementkartusche verbunden ist oder das Ventil einen solchen Anschluss aufweist.

Hiermit kann die Gießform mit einer Knochenzementkartusche gefüllt werden und die Hohlform durch einen mit dem Knochenzementteig vermittelten Druck expandiert werden.

Es kann vorgesehen sein, dass der Ventilsitz nicht verdrehbar gegen die Gießform mit der Gießform verbunden ist, bevorzugt der Ventilsitz fest und/oder starr mit der Gießform verbunden ist.

Hierdurch kann das Ventil der Vorrichtung bequem von außen bedient werden, um eine Knochenzementkartusche zu wechseln oder diese zu lösen.

Es kann auch vorgesehen sein, dass das Ventil durch Drehen oder Kippen einer an den Anschluss angeschlossenen Zementkartusche bedienbar ist, wobei hierzu vorzugsweise der Anschluss an dem Ventilkörper angeordnet ist.

Ferner kann vorgesehen sein, dass das Ventil manuell bedienbar ist, bevorzugt manuell von außerhalb der Vorrichtung manuell bedienbar ist, wobei besonders bevorzugt der Ventilkörper manuell gegen den Ventilsitz drehbar ist und durch die Drehung das Ventil von der geschlossenen Stellung in die geöffnete Stellung und von der geöffneten Stellung in die geschlossene Stellung überführbar ist.

Hierdurch kann die Vorrichtung bequem von außen bedient werden. Zudem kann so die Knochenzementkartusche getauscht werden.

Des Weiteren kann vorgesehen sein, dass der Ventilsitz ein Innengewinde an der Innenseite aufweist und der Ventilkörper ein passendes Außengewinde an der Außenseite aufweist, so dass der Ventilkörper in den Ventilsitz schraubbar ist und/oder

Durch diese Maßnahme kann eine hohe Dichtwirkung an der Verbindung zwischen dem Ventilkörper und dem Ventilsitz erreichet werden. Zudem kann das Ventil so einfach und kostengünstig zusammengebaut werden.

Ferner kann vorgesehen sein, dass der Anschluss zum flüssigkeitsdichten Verbinden einer Knochenzementkartusche ein Innengewinde im Ventilkörper oder ein Außengewinde am Ventilkörper umfasst, wobei vorzugsweise ein Adapterelement der Knochenzementkartusche oder an der Knochenzementkartusche ein zu dem Innengewinde oder zu dem Außengewinde passendes Gegengewinde aufweist.

Hierdurch kann zum Einen eine stabile und flüssigkeitsdichte Verbindung am Anschluss hergestellt werden und zum anderen kann die Drehung bei der Schraubbewegung genutzt werden, um zu Beginn oder nach Ende der Schraubbewegung den Ventilkörper gegen den Ventilsitz zu drehen und somit das Ventil vom geöffneten in den geschlossenen Zustand zu überführen oder um das Ventil vom geschlossenen in den geöffneten Zustand zu überführen.

Durch die Verwendung geeigneter Gewinde kann insbesondere eine zusätzliche Sicherungsfunktion der Vorrichtung dadurch erzielt werden, dass ein Lösen der Knochenzementkartusche nur bei geschlossenem Ventil möglich ist und ein Öffnen des Ventils nur bei angeschlossener Knochenzementkartusche möglich ist.

Bei erfindungsgemäßen Vorrichtungen mit Ventilelement kann vorgesehen sein, dass das Innengewinde im Ventilkörper oder das Außengewinde am Ventilkörper ein Rechtsgewinde ist und das Ventil durch eine gleichsinnige Rechtsdrehung des Ventilkörpers von der geschlossenen in die geöffnete Stellung überführbar ist und das Ventil durch eine gegensinnige Linksdrehung des Ventilkörpers von der geöffneten in die geschlossene Stellung überführbar ist oder
das Innengewinde im Ventilkörper oder das Außengewinde am Ventilkörper ein Linksgewinde ist und das Ventil durch eine gleichsinnige Linksdrehung des Ventilkörpers von der geschlossenen in die geöffnete Stellung überführbar ist und das Ventil durch eine gegensinnige Rechtsdrehung des Ventilkörpers von der geöffneten in die geschlossene Stellung überführbar ist oder
das Innengewinde des Ventilsitzes und das Innengewinde und das Außengewinde des Ventilkörpers alle Linksgewinde oder alle Rechtsgewinde sind, wobei vorzugsweise auch ein Außengewinde eines Adapterelements zum flüssigkeitsdichten Verbinden einer Knochenzementkartusche an den Anschluss den gleichen Drehsinn hat.

Auch diese Maßnahmen zielen darauf ab, dass ein Schließen des Ventils beim Ausschrauben der Knochenzement-Kartusche automatisch erfolgt und ein Öffnen des Ventils bei Einschrauben der Knochenzement-Kartusche automatisch erfolgt.

Bei Vorrichtungen mit dem Ventil kann vorgesehen sein, dass die mindestens eine erste Durchführung des Ventilsitzes in der geschlossenen Stellung des Ventils mit der Dichtfläche des Ventilkörpers abgedeckt ist, wobei bevorzugt die bereichsweise geschlossene Kopfseite des Ventilsitzes und die Dichtfläche des Ventilkörpers maximal 2 mm voneinander beabstandet sind, besonders bevorzugt maximal 1 mm voneinander beabstandet sind, ganz besonders bevorzugt maximal 0,5 mm voneinander beabstandet sind.

Hierdurch kann sichergestellt werden, dass der in die Gießform eingefüllte Knochenzementteig (der fluide Knochenzement) nicht wieder aus der Gießform durch das Ventil herausgedrückt werden kann, wenn das Ventil geschlossen ist. Wenn der Knochenzementteig mit diesen Dicken beziehungsweise mit diesen Querschnitten im Bereich des Angusses aushärtet, kann er nach dem Aushärten des Spacers leicht manuell abgebrochen oder durchgeschnitten werden und muss nicht mit einer Säge aufgetrennt werden. Daher sind Angüsse mit solchen Dicken unschädlich, da sie den OP-Betrieb während einer OP nicht nennenswert aufhalten.

Bei Vorrichtungen mit dem Ventil kann ferner vorgesehen sein, dass der Ventilkörper um eine Drehachse gegen den Ventilsitz drehbar gelagert ist, wobei die Drehachse senkrecht zur Dichtfläche des Ventilkörpers verläuft oder wobei die Drehachse entlang einer Rotationssymmetrieachse der Dichtfläche des Ventilkörpers verläuft.

Hiermit wird erreicht, dass der durch das Ventil fließende Knochenzementteig von dem Ventilkörper durch die Drehung abgeschnitten beziehungsweise abgedreht werden kann. Dies ermöglicht eine glatte Schnittfläche und einen geringen Kraftaufwand beim Abscheren. Ferner kann auch eine Drehung der Knochenzementkartusche zum Abscheren des Knochenzementteigs verwendet werden. Es wird bevorzugt, dass die Drehachse entlang der Rotationssymmetrieachse der Dichtfläche des Ventilkörpers verläuft. Wenn die Drehachse senkrecht zur Dichtfläche des Ventilkörpers verläuft, kann das Ventil nach Art eines Zapfhahns (beispielsweise für Bier) aufgebaut sein.

Es kann auch vorgesehen sein, dass der Ventilkörper um eine Drehachse gegen den Ventilsitz drehbar gelagert ist, wobei die Drehachse in Richtung der Einfüllöffnung ausgerichtet ist.

Es kann ferner vorgesehen sein, dass sich der Ventilkörper um einen Winkel von maximal 280° gegen den Ventilsitz verdrehen lässt, bevorzugt von maximal 180°, besonders bevorzugt von maximal 100° gegen den Ventilsitz verdrehen lässt, ganz besonders bevorzugt von bis zu 90° gegen den Ventilsitz verdrehen lässt.

Bevorzugt können jeweils zwei Durchführungen in dem Ventilsitz und in dem Ventilkörper angeordnet sein, wobei zwei Durchführungen bevorzugt um 180° versetzt um den Mittelpunkt von Scheiben des Ventilsitzes und des Ventilkörpers angeordnet sind, wobei die Scheiben die bereichsweise geschlossene Kopfseite des Ventilsitzes und die Dichtfläche des Ventilkörpers bilden.

Bei Vorrichtungen mit dem Ventil kann des Weiteren vorgesehen sein, dass der Ventilkörper den Anschluss zum flüssigkeitsdichten Verbinden einer Knochenzementkartusche aufweist oder mit einem solchen Anschluss fest verbunden ist.

Hierdurch kann der Ventilkörper mit Hilfe einer angeschlossenen Knochenzementkartusche bedient werden.

Bei Vorrichtungen mit dem Ventil kann auch vorgesehen sein, dass die Summe aller Öffnungen der mindestens einen ersten Durchführung in der geschlossenen Kopfseite höchstens so groß ist wie die geschlossene Oberfläche der Kopfseite und dass die Summe aller Öffnungen der mindestens einen zweiten Durchführung in der Dichtfläche höchstens so groß ist wie die geschlossene Oberfläche der Dichtfläche.

Hierdurch wird sichergestellt, dass ein stabiles und für den Knochenzementteig undurchlässiges Verschließen des Ventils durch Drehen des Ventilkörpers gegen den Ventilsitz möglich ist.

Bei Vorrichtungen mit dem Ventil kann bevorzugt vorgesehen sein, dass die mindestens eine erste Durchführung in der bereichsweise geschlossenen Kopfseite die gleiche Größe und Gestalt hat wie die mindestens eine zweite Durchführung in der Dichtfläche.

Ebenfalls bevorzugt kann vorgesehen sein, dass die mindestens eine erste Durchführung in der bereichsweise geschlossenen Kopfseite zwei erste Durchführungen sind und die mindestens eine zweite Durchführung in der Dichtfläche zwei zweite Durchführungen sind, wobei bevorzugt die zwei ersten Durchführungen in gegenüberliegend angeordneten Kreissegmentvierteln bezüglich der Drehachse des Ventilkörpers im Ventilsitz angeordnet sind und die zwei zweiten Durchführungen in gegenüberliegend angeordneten Kreissegmentvierteln bezüglich der Drehachse des Ventilkörpers in der Dichtfläche angeordnet sind.

Durch diese beiden Maßnahmen kann ein ausreichender Strömungsquerschnitt für den zähflüssigen Knochenzementteig bereitgestellt werden und es können einseitige Belastungen des Ventils vermieden werden, die ansonsten zu einer Undichtigkeit des Ventils führen könnten.

Bei Vorrichtungen mit dem Ventil kann ferner vorgesehen sein, dass an der Dichtfläche des Ventilkörpers ein Bund angeordnet ist, der sich auf einem Rand des Ventilsitzes abstützt oder an der bereichsweise geschlossenen Kopfseite des Ventilsitzes ein Bund angeordnet ist, der sich auf einem Rand des Ventilkörpers abstützt.

Hierdurch kann eine stabile Führung des Ventilkörpers am Ventilsitz erreicht werden. Ferner kann dadurch exakt die Position der mindestens einen zweiten Durchführung bei gegebener Gewindelänge des Ventilkörpers bezüglich der mindestens einen ersten Durchführung definiert werden.

Dabei kann vorgesehen sein, dass ein radial ausgerichteter Hebel an der Mantelfläche des Ventilkörpers neben dem Bund angeordnet ist.

Bei Vorrichtungen mit dem Ventil kann auch vorgesehen sein, dass an dem Ventilkörper ein Hebel angeordnet ist, der eine radiale Erstreckung bezüglich der Drehachse des Ventilkörpers aufweist, wobei vorzugsweise der Hebel durch eine Aussparung in der Gießform oder in dem Ventilsitz ragt, wobei die Aussparung in der Gießform gegebenenfalls im Bereich der Verbindung zum Ventilsitz angeordnet ist, wobei die Aussparung derart dimensioniert ist, dass sich das Ventil durch Drehen des Ventilkörpers im Ventilsitz mittels des Hebels von der geöffneten Stellung in die geschlossene Stellung und umgekehrt überführen lässt, wobei besonders bevorzugt die Aussparung derart dimensioniert ist, dass sich der Ventilkörper maximal um 90° gegen den Ventilsitz drehen lässt.

Hiermit kann das Ventil bequem von außen manuell bedient werden. Mit diesem Hebel kann der Ventilkörper von der geöffneten Stellung in die geschlossene Stellung des Ventils gedreht werden.

Bei Vorrichtungen mit dem Ventil kann ferner vorgesehen sein, dass der Ventilkörper und der Ventilsitz aus Kunststoff gefertigt sind, insbesondere aus thermoplastischem Kunststoff gefertigt sind, wobei bevorzugt der Ventilsitz mit einer Wandung der Gießform verklebt oder verschweißt ist.

Hierdurch ist das Ventil und damit die Vorrichtung kostengünstig und als hygienisches Wegwerfprodukt zu fertigen.

Bevorzugt kann vorgesehen sein, dass der Ventilsitz an seiner Außenseite Rippen besitzt, die eine formschlüssige Verbindung mit der Gießform eingehen beziehungsweise eingehen können.

Auch kann vorgesehen sein, dass die Vorrichtung ein Adapterelement aufweist, das mit einer Knochenzementkartusche verbunden ist oder verbindbar ist, wobei das Adapterelement lösbar und formschlüssig mit dem Anschluss verbunden oder verbindbar ist, so dass ein Innenraum der Knochenzementkartusche über das Adapterelement für Knochenzementteig durchlässig mit der Einfüllöffnung verbunden oder verbindbar ist. Wenn die Vorrichtung das Ventil aufweist, kann vorgesehen sein, dass der Innenraum der Knochenzementkartusche über das Adapterelement für Knochenzementteig durchlässig mit der mindestens einen zweiten Durchführung im Ventilkörper des Ventils verbunden oder verbindbar ist.

Es kann vorgesehen sein, dass die Vorrichtung eine Knochenzementkartusche zum Mischen von Knochenzement-Ausgangskomponenten und zum Austragen von gemischtem Knochenzementteig aus der Knochenzementkartusche heraus aufweist, bevorzugt eine Knochenzementkartusche zum Mischen von Polymethylmethacrylat-Knochenzement-Ausgangskomponenten und zum Austragen von gemischtem Polymethylmethacrylat-Knochenzementteig aus der Knochenzementkartusche heraus aufweist, wobei besonders bevorzugt die Knochenzementkartusche die Knochenzement-Ausgangskomponenten zur Herstellung des Knochenzements in voneinander getrennten Bereichen beinhaltet.

Hierdurch wird die Vorrichtung weiter komplettiert, da mit der Vorrichtung dann auch der Knochenzementteig bereitgestellt werden kann, der zum Ausformen des Spacers in die Gießform gefüllt wird und mit dem die Hohlform der Gießform expandiert wird, indem mit der Knochenzementkartusche der hierfür notwendige Druck auf den Knochenzementteig ausgeübt wird.

Des Weiteren kann vorgesehen sein, dass die Gießform ausgehend von einem Innenraum der Gießform mindestens drei oder vier Kavitäten zur Aufnahme von Haltestiften aufweist, wobei bevorzugt die Kavitäten in der Schaftform angeordnet sind und besonders bevorzugt die Schaftform zweiteilig oder dreiteilig ist und die Kavitäten in Rändern oder in Längsflanschen zumindest eines Teils der zweiteiligen Schaftform angeordnet sind.

Mit diesen Kavitäten kann ein Metallkern als Armierung in der Gießform und damit in dem Spacer angeordnet und genau platziert werden.

Auch kann vorgesehen sein, dass die Vorrichtung einen Metallkern aufweist, der in der Gießform anzuordnen ist, wobei bevorzugt der Metallkern Bohrungen zur Aufnahme von Haltestiften besitzt, wobei besonders bevorzugt die Bohrungen innerhalb des Teils des Metallkerns angeordnet sind, die in der Schaftform anzuordnen sind.

Vorzugsweise besteht der Metallkern aus einem biokompatiblen Metall oder aus einer biokompatiblen Metalllegierung, besonders bevorzugt aus chirurgischem Stahl.

Es kann auch vorgesehen sein, dass die Vorrichtung zumindest drei oder vier Haltestifte zur Halterung des Metallkerns in der Gießform aufweist.

Der Metallkern dient der Stabilisierung des Spacers und damit einer besseren Haltbarkeit des behandelten Gelenks.

Durch die Haltestifte wird er Metallkern in einer definierten Position innerhalb der Gießform gehalten. Dadurch wird die Dicke des Knochenzementmantels um den Metallkern definiert. Die Haltestifte sind vorzugsweise aus einem biokompatiblen Kunststoff gefertigt. Besonders eignet sich hierbei Polymethylmethacrylat. Haltestifte aus Polymethylmethacrylat verbinden sich mit dem Knochenzementteig irreversibel. Nach Aushärtung des Knochenzementteigs werden die aus dem Spacer herausragenden Haltestifte einfach abgeschnitten. Die im Inneren des Spacer befindlichen Reste der Haltestifte verbleiben dort.

Bevorzugt kann auch vorgesehen sein, dass die kugelflächenförmige Innenfläche der Hohlform im nicht expandierten Zustand einen Durchmesser von mindestens 35 mm oder von mindestens 40 mm hat, bevorzugt zwischen 40 mm und 50 mm hat, und im maximal expandierten Zustand einen Durchmesser von höchstens 70 mm hat, bevorzugt zwischen 60 mm und 70 mm, wobei der Durchmesser der kugelflächenförmigen Innenfläche der Hohlform im nicht expandierten Zustand kleiner ist als der Durchmesser der kugelflächenförmigen Innenfläche der Hohlform im expandierten Zustand.

Der maximal expandierte Zustand ist der Zustand, der erreichbar ist, wenn der Druck auf den Knochenzementteig mit Hilfe einer üblichen Knochenzementkartusche erzeugt wird. Eine übliche Knochenzementkartusche ist beispielsweise eine Palacos^{®} Knochenzementkartusche, wie sie von der Firma Heraeus Medical GmbH erhältlich ist.

Hierdurch lassen sich Spacer mit Köpfen erzeugen, wie sie im Bereich von Hüftspacern üblich sind. Dabei ist bevorzugt, dass auch bei der kleinsten Größe ein expandierten Zustand der Hohlform notwendig ist, damit die Hohlform auch bei der kleinsten Größe einen Druck auf den Knochenzementteig in der Gießform ausübt, mit dem die gewünschte Form erreicht wird und mit dem Lufteinschlüsse aus dem Knochenzementteig durch das wenigstens eine Entlüftungselement aus die Gießform herausgedrückt werden kann. Ferner ist es so möglich, mit der Vorrichtung Hüftspacer mit den üblichen Spacerkopfdurchmessern im Bereich von 46 mm bis 65 mm herzustellen. Schulterspacer mit Spacerkopfdurchmessern von 40 mm bis 50 mm sind ebenfalls herstellbar.

Des Weiteren kann vorgesehen sein, dass die Hohlform zumindest im Bereich der kugelflächenförmigen Innenfläche aus einem gummielastischen Material besteht, vorzugsweise aus Kautschuk, Silikonkautschuk, einem Synthesekautschuk oder einem Ethylen-Propylen-Dien-Kautschuk (EPDM) besteht.

Ethylen-Propylen-Dien-Kautschuke (EPDM) sind Terpolymere aus Ethylen, Propylen und einem nicht näher festgelegten Dien.

Die Materialien sind besonders gut für die Hohlform beziehungsweise den Bereich der kugelflächenförmigen Innenfläche der Hohlform geeignet, um eine elastische Verformung zu ermöglichen. Daneben kommen alle sonstigen gummielastischen, biokompatiblen Kunststoffe als Material für die Bildung der Hohlform in Betracht.

Es kann auch vorgesehen sein, dass die wenigstens eine Einfüllöffnung ein Absperrelement enthält, das in einem geschlossenen Zustand ein Ausfließen des Knochenzementteigs aus der Gießform durch die wenigstens eine Einfüllöffnung verhindert.

Hierdurch kann der Druck des Knochenzementteigs in der Gießform beim Aushärten aufrechterhalten werden und damit die Hohlform im expandierten Zustand gehalten werden, wobei gleichzeitig die wenigstens eine Einfüllöffnung mit dem Absperrelement verschlossen ist. Dadurch kann die Ausbildung eines massiven Angusses verhindert werden.

Ferner kann vorgesehen sein, dass die Schaftform ein in der Länge variables Adapterelement umfasst, mit dem die Länge des Halses des Spacers, der den Schaft mit dem Kopf des Spacers verbindet, variierbar ist, wobei vorzugsweise das Adapterelement durch eine Schraubverbindung in der Länge variierbar ist.

Hiermit kann die Länge des Halses des Spacers variiert und eingestellt werden und damit an die individuelle Behandlungssituation angepasst werden.

Auch kann vorgesehen sein, dass die Vorrichtung mehrere formstabile Gegenformen aufweist, die zur Aufnahme der Hohlform geeignet sind, wobei die formstabilen Gegenformen eine unterschiedlich weite Expansion der Hohlform ermöglichen, so dass die Expansion der Hohlform zumindest in dem Bereich der kugelflächenförmigen Innenfläche durch die formstabilen Gegenformen bis hin zu unterschiedlichen Durchmessern begrenzt ist, wenn die Hohlform in die jeweilige formstabile Gegenform eingesetzt ist, wobei bevorzugt die formstabilen Gegenformen durch zumindest eine Blisterpackung oder eine Kunststoffschale mit einer oder mit mehreren Vertiefungen als die formstabilen Gegenformen realisiert ist.

Des Weiteren kann vorgesehen sein, dass die Vorrichtung eine Prüflehre oder eine Schieblehre zum Messen des aktuellen Durchmessers der kugelflächenförmigen Innenfläche der Hohlform aufweist, wobei die Prüflehre oder die Schieblehre außen an der Hohlform anlegbar oder angeordnet ist und wobei vorzugsweise der Durchmesser der kugelflächenförmigen Innenfläche der Hohlform unmittelbar ablesbar ist.

Durch diese Maßnahmen wird die Anwendung der Vorrichtung vereinfacht. Der Anwender kann so den gewünschten Durchmesser des Kopfs des Spacers bestimmen oder einstellen. Durch die formstabilen Gegenformen kann der Anwender einen Spacer mit einem Kopf mit dem gewünschten Durchmesser oder der gewünschten Form erzeugen, ohne dabei sehr genau auf den auf den Knochenzementteig ausgeübten Druck achten zu müssen.

Die der vorliegenden Erfindung zugrundeliegenden Aufgaben werden auch gelöst durch ein Verfahren zur Herstellung eines Spacers zum temporären Ersetzen eines Gelenks oder eines Teils eines Gelenks, insbesondere eines Hüftgelenks oder eines Schultergelenks, umfassend eine artikulierende Oberfläche des Gelenks, wobei das Verfahren mit einer oben genannten Vorrichtung durchgeführt wird, das Verfahren aufweisend die folgenden chronologischen Schritte:
A) Einpressen von Knochenzementteig durch die wenigstens eine Einfüllöffnung in die Gießform und gleichzeitig Verdrängen von Luft aus der Gießform durch das wenigstens eine Entlüftungselement durch das Einpressen des Knochenzementteigs;
B) Weiteres Einpressen von Knochenzementteig durch die wenigstens eine Einfüllöffnung in die Gießform, wobei durch das Einpressen des Knochenzementteigs die Hohlform zumindest im Bereich der kugelflächenförmigen Innenfläche expandiert, während die Schaftform formstabil bleibt;
C) Aushärten des Knochenzementteigs in der Gießform; und
D) Entnehmen des so geformten und ausgehärteten Spacers aus der Gießform.

Dabei kann vorgesehen sein, dass der Knochenzementteig die Luft aus der Gießform durch das wenigstens eine Entlüftungselement herausdrückt, bis der Knochenzementteig auf einen für Knochenzementteig undurchlässigen Filter, insbesondere einen für Gas durchlässigen aber für Knochenzementteig undurchlässigen Porenfilter in dem wenigstens einen Entlüftungselement trifft.

Das Verfahren wird nicht zur unmittelbaren medizinischen Therapie eingesetzt. Lediglich der mit dem erfindungsgemäßen Verfahren hergestellte Spacer kann zu therapeutischen Maßnahmen oder zur medizinischen Behandlung eingesetzt werden. Ein Eingriff am Körper findet bei der Herstellung des Spacers nicht statt.

Der Spacer ist für medizinische Anwendungen vorgesehen. Das erfindungsgemäße Verfahren umfasst nicht die Implantation bei einem Patienten sondern lediglich das Ausformen des Spacers. Nach Schritt D) kann der Spacer entgratet, geglättet, geschliffen, gereinigt, poliert und/oder bereichsweise aufgeraut werden.

Zum Entnehmen des geformten und ausgehärteten Spacers aus der Gießform in Schritt D) kann die Gießform nach Schritt C) geöffnet werden.

Es kann vorgesehen sein, dass vor Schritt A) ein flüssigkeitsdichtes Verbinden einer Knochenzementkartusche mit einem Anschluss der Vorrichtung erfolgt, wobei der Anschluss mit der wenigstens einen Einfüllöffnung flüssigkeitsdurchlässig verbunden ist und in Schritt A) der Knochenzementteig aus der Knochenzementkartusche in die Gießform gepresst wird.

Hierdurch kann die Knochenzementkartusche zum Befüllen der Gießform und zum Aufbauen des Drucks auf den Knochenzementteig in der Gießform verwendet werden, um den Hohlraum mit dem Knochenzementteig zu expandieren.

Dabei kann vorgesehen sein, dass eine Vorrichtung mit einem wie zuvor beschriebenen Ventil verwendet wird, wobei das Einpressen von Knochenzementteig in Schritt A) durch das Ventil in der geöffneten Stellung hindurch in die Gießform erfolgt, wobei nach Schritt B) und vor Schritt C) ein Schritt B1) erfolgt:
B1) Drehen des Ventilkörpers gegen den Ventilsitz und dadurch Überführen des Ventils in die geschlossene Stellung und Abscheren des Knochenzementteigs an der mindestens einen ersten Durchführung in der bereichsweise geschlossenen Kopfseite des Ventilsitzes durch die Drehung des Ventilkörpers gegen den Ventilsitz, wobei vorzugsweise anschließend eine Knochenzementkartusche von einem Anschluss gelöst wird, der mit der wenigstens einen Einfüllöffnung flüssigkeitsdurchlässig verbunden ist.

Hiermit kann der Druck, der von dem Knochenzementteig auf die Hohlform ausgeübt wird, auch dann gehalten werden, wenn das Ventil geschlossen ist und keine Knochenzementkartusche angeschlossen ist. Dadurch kann zum einen ein leicht zu durchtrennender dünner Anguss erzeugt werden und zum anderen wird verhindert, dass Knochenzementteig wieder aus der Gießform herausfließt und dadurch der Kopf des Spacers einen zu geringen Durchmesser erhält.

Ferner kann vorgesehen sein, dass nach Schritt B1) und vor Schritt C) die folgenden Zwischenschritte erfolgen:
B2) Flüssigkeitsdichtes Verbinden einer neuen Knochenzementkartusche mit dem Anschluss der Vorrichtung, wobei in der neuen Knochenzementkartusche Knochenzementteig oder Ausgangskomponenten zur Herstellung des Knochenzementteigs enthalten ist oder sind; B3) Drehen des Ventilkörpers gegen den Ventilsitz und dadurch Überführen des Ventils in die geöffnete Stellung;
B4) Einpressen des Knochenzementteigs aus der neuen Knochenzementkartusche durch das Ventil in der geöffneten Stellung hindurch in die Gießform;
B5) Drehen des Ventilkörpers gegen den Ventilsitz und dadurch Überführen des Ventils in die geschlossene Stellung und Abscheren des Knochenzementteigs an der mindestens einen ersten Durchführung in der bereichsweise geschlossenen Kopfseite des Ventilsitzes durch die Drehung des Ventilkörpers gegen den Ventilsitz; und
B6) Lösen der neuen Knochenzementkartusche von dem Anschluss;
   wobei vorzugsweise die Schritte B2) bis B6) einmal oder mehrfach mit jeweils neuen Knochenzementkartuschen, die Knochenzementteig oder dessen Ausgangskomponenten enthalten, wiederholt werden, bis die Gießform vollständig mit Knochenzementteig gefüllt ist und darüber hinaus bis die Hohlform zumindest im Bereich der kugelflächenförmigen Innenfläche mit Hilfe des Knochenzementteigs bis zur gewünschten Größe expandiert ist.

Hierdurch kann eine Gießform mit großem Volumen mit mehreren Knochenzementkartuschen enthaltend geringe Volumina des Knochenzementteigs gefüllt werden. Dies ist beispielsweise für die Herstellung von großvolumigen Hüftgelenkspacern vorteilhaft.

Es kann vorgesehen sein, dass vor Schritt A), und vorzugsweise vor dem Anschließen der Knochenzementkartusche, der Knochenzementteig in der Knochenzementkartusche aus einer Monomerflüssigkeit und aus einem Zementpulver gemischt wird, wobei bevorzugt gegebenenfalls vor Schritt B3), vorzugsweise vor Schritt B2), der Knochenzementteig in der neuen Knochenzementkartusche aus einer Monomerflüssigkeit und aus einem Zementpulver gemischt wird.

Hierdurch kann zur Herstellung des Spacers ein frisch gemischter Knochenzementteig verwendet werden. Insbesondere PMMA-Knochenzementteige lassen sich im gemischten Zustand nur schwer oder gar nicht über Zeiträume von mehr als wenigen Minuten lagern. Zudem können dem Knochenzementteig so auch kurz vor der Herstellung des Spacers dem Knochenzementteig zur Behandlung geeignete pharmazeutische Wirkstoffe, wie Antibiotika und Antimykotika, beigemengt werden.

Es kann auch vorgesehen sein, dass zum flüssigkeitsdichten Verbinden der Knochenzementkartusche und/oder der neuen Knochenzementkartusche mit dem Anschluss die Knochenzementkartusche oder die neue Knochenzementkartusche in den Anschluss hineingedreht oder eingeschraubt wird und zum Lösen der Knochenzementkartusche und/oder der neuen Knochenzementkartusche von dem Anschluss die Knochenzementkartusche oder die neue Knochenzementkartusche aus dem Anschluss herausgedreht oder herausgeschraubt wird.

Neben dem Schrauben kann die Knochenzementkartusche beispielsweise mit einem Bajonettverschluss mit dem Anschluss verbunden werden.

Durch das Eindrehen oder Einschrauben der Knochenzementkartusche in den Anschluss kann eine flüssigkeitsdichte Verbindung zwischen dem Anschluss und der Knochenzementkartusche bereitgestellt werden. Zudem kann durch die Drehung auch eine Drehung des Ventilkörpers gegen den Ventilsitz erfolgen beziehungsweise induziert werden.

Ferner kann vorgesehen sein, dass das Drehen des Ventilkörpers gegen den Ventilsitz durch ein Schrauben des Ventilkörpers in dem Ventilsitz erfolgt oder durch ein manuelles Drehen des Ventilkörpers gegen Ventilsitz erfolgt, wobei bevorzugt das manuelle Drehen durch Bedienen eines sich radial von Ventilkörper weg erstreckenden Hebels erfolgt, der sich durch eine Aussparung in der Gießform oder in dem Ventilsitz erstreckt.

Hiermit kann das Ventil auf einfache Art und Weise vom Anwender bedient werden.

Des Weiteren kann vorgesehen sein, dass das Einpressen des Knochenzementteigs aus der Knochenzementkartusche oder der neuen Knochenzementkartusche durch Eindrücken eines Kolbens in einen Innenraum der Knochenzementkartusche erfolgt.

Hierdurch kann der Knochenzementteig auf einfache Weise aus der Knochenzementkartusche durch das geöffnete Ventil in die Gießform eingepresst werden.

Auch kann vorgesehen sein, dass vor Schritt A), und vorzugsweise vor dem Anschließen der Knochenzementkartusche, ein Metallkern innerhalb der Gießform angeordnet wird, wobei bevorzugt der Metallkern über mehrere Haltestifte von einer Innenwand der Schaftform beabstandet wird, wobei besonders bevorzugt die mehreren Haltestifte in Bohrungen im Metallkern und in Kavitäten zur Aufnahme von Haltestiften in der Innenwand der Schaftform befestigt werden.

Hierdurch kann der Spacer mit Hilfe der Vorrichtung mit einer innenliegenden Armierung aufgebaut werden. Der Knochenzementteig umfließt dabei den in der Gießform angeordneten Metallkern.

Bevorzugt kann ferner vorgesehen sein, dass die Hohlform der Gießform in eine von mehreren formstabile Gegenformen eingesetzt wird, wobei die formstabilen Gegenformen eine unterschiedlich weite Expansion der Hohlform ermöglichen, so dass die Expansion der Hohlform zumindest in dem Bereich der kugelflächenförmigen Innenfläche durch die verwendete formstabile Gegenform bis hin zu einem bestimmten Durchmessern begrenzt wird, während der Knochenzementteig zum Expandieren der Hohlform in die Gießform gepresst wird.

Des Weiteren kann vorgesehen sein, dass eine Prüflehre oder eine Schieblehre zum Messen des aktuellen Durchmessers der kugelflächenförmigen Innenfläche der Hohlform dazu verwendet wird, den aktuellen Durchmesser der kugelflächenförmigen Innenfläche der Hohlform abzulesen, wobei vorzugsweise das Einpressen des Knochenzementteigs in die Gießform gestoppt wird, wenn ein gewünschter Durchmesser erreicht wird.

Durch diese Maßnahmen wird die Anwendung des Verfahrens vereinfacht. Der Anwender kann so den gewünschten Durchmesser des Kopfs des Spacers bestimmen oder einstellen. Durch die formstabilen Gegenformen kann der Anwender einen Spacer mit einem Kopf mit dem gewünschten Durchmesser oder der gewünschten Form erzeugen, ohne dabei sehr genau auf den auf den Knochenzementteig ausgeübten Druck achten zu müssen.

Der Erfindung liegt die überraschende Erkenntnis zugrunde, dass es durch die expandierbare Hohlform gelingt, eine Gießform mit variabler Kopfgröße zur Herstellung eines Spacers bereitzustellen, so dass mit der Gießform Spacer mit unterschiedlichen Köpfen, insbesondere mit Köpfen mit unterschiedlichen Durchmessern, hergestellt werden können. Der Durchmesser kann dabei stufenlos eingestellt werden. Der Durchmesser des Kopfs des Spacers kann durch einen (insbesondere manuell) mit dem Knochenzementteig vermittelten Druck eingestellt werden. Die Anwendung der Vorrichtung ist dadurch besonders einfach und intuitiv.

Ein besonderer Vorteil der vorliegenden Erfindung besteht darin, dass mit einer Hüft- oder Schulterspacergießform durch Expandieren der Gießform im Kopfbereich Spacer mit unterschiedlichen Kopfdurchmessern hergestellt werden können, ohne dass separate Kopfgießformen für jeden gewünschten Kopfdurchmesser notwendig sind, die aufwendig mit der Schaftform für den Spacerschaft verbunden werden müssen. Der Kopfdurchmesser der Spacer kann kontinuierlich vom Anwender durch Einpressen von Polymethylmethacrylat-Knochenzement eingestellt werden. Das bedeutet, mit einer einzigen Gießform können Hüft- und Schulterspacer mit allen anatomisch möglichen Kopfdurchmessern hergestellt werden. Bei konventionellen Spacergießformen gibt es üblicherweise mindestens vier unterschiedliche Kopfgrößen. Diese können durch eine einzelne Gießform ersetzt werden. Der Herstellungs- und auch der Logistikaufwand sinkt dadurch erheblich.

Für Vorrichtungen mit Ventil ergeben sich zusätzliche Vorteile. Knochenzementteig, insbesondere nicht hochviskoser Knochenzementteig, kann durch den Verschluss beziehungsweise das geschlossene Ventil nicht aus der Gießform fließen. Ein Zusammenziehen der Hohlform und dadurch eine Verringerung des Durchmessers des Spacerkopfs wird dadurch verhindert. Eine Ausbildung von Fehlstellen im Spacer infolge des Auslaufens von Knochenzementteig wird ebenso verhindert. Weiterhin wird durch die erfindungsgemäßen Maßnahmen der noch in der Knochenzementkartusche befindliche Rest des Knochenzementteigs vom Knochenzementteig in der Gießform getrennt. Nach beendeter Aushärtung des Knochenzementteigs ist es daher nicht mehr notwendig, den Anguss mechanisch beispielsweise durch Sägen abzutrennen. Eventuell verbliebene dünne Verbindungen können leicht abgebrochen oder abgeschnitten werden. Dadurch wird der Arbeits- und Zeitaufwand für das OP-Personal gesenkt.

Der Anguss des Spacers wird durch die wenigstens eine Einfüllöffnung mit dem Ventilsitz und dem Ventilkörper gebildet. Durch das Verdrehen des Ventilkörpers gegenüber dem Ventilsitz aus der geöffneten Stellung in die geschlossene Stellung des Ventils wird die Gießform für Knochenzementteig undurchlässig verschlossen. Das bedeutet, der aus dem Ventilsitz und dem Ventilkörper gebildete Anguss beziehungsweise die dem Anguss formgebende Teile haben gleichzeitig eine Ventilfunktion. Komplexe zusätzliche Ventile sind nicht notwendig.

Die manuelle Drehung des Ventilkörpers gegen den Ventilsitz kann durch einen Hebel an der Außenseite des Ventilkörpers erfolgen. Vorteilhaft kann die Drehung auch dadurch erfolgen, dass beim Herausdrehen der Knochenzementkartusche der Ventilkörper von der Knochenzementkartusche mit gedreht wird. Dabei ist es jedoch notwendig, dass ein Anschlag die Drehbewegung des Ventilkörpers gegenüber dem Ventilsitz begrenzt, damit der Verschluss sicher erfolgen kann und damit der Ventilkörper nicht aus dem Ventilsitz komplett herausgedreht werden kann.

Ein mit der Vorrichtung hergestellter Spacer kann vorteilhaft im Rahmen von zweizeitigen septischen Revisionen verwendet werden, bei denen eine Infektion mit zwei oder mehreren mikrobiellen Keimen und insbesondere mit problematischen Keimen vorliegt.

Eine beispielhafte erfindungsgemäße Vorrichtung kann zusammengesetzt sein aus
a) einer formstabilen hohlen Schaftform, die den Schaft und den Hals des Spacers abbildet,
b) einem Port (als die wenigstens eine Einfüllöffnung) zum Einpressen von Polymethylmethacrylat-Knochenzementteig,
c) einer gummielastischen, zumindest halbkugelförmigen Hohlform, die mit der formstabilen hohlen Schaftform der Gießform verbunden oder verbindbar ist,
d) wenigstens einem Entlüftungselement, das den Innenraum der formstabilen hohlen Schaftform und der gummielastischen, zumindest halbkugelförmigen Hohlform mit der umgebenden Atmosphäre verbindet, wobei das wenigstens eine Entlüftungselement für Gase durchlässig und für pastösen Polymethylmethacrylat-Knochenzement undurchlässig ist, und
e) wobei der Durchmesser der gummielastischen, zumindest halbkugelförmigen Hohlform, durch das Volumen des in die Gießform eingepressten Polymethylmethacrylat-Knochenzementteig stufenlos unter Ausdehnung der gummielastischen, zumindest halbkugelförmigen Hohlform vergrößert wird beziehungsweise vergrößerbar ist.

Die erfindungsgemäße Vorrichtung beziehungsweise die beispielhafte Gießform der Vorrichtung kann auch gegeneinander bewegliche Hohlzylinder zur Formen eines Halses des Spacers aufweisen, wobei der Hals der Kopf und den Schaft des Spacers miteinander verbindet und durch eine relative Positionierung der beiden gegeneinander beweglichen Hohlzylinder die Länge des Halses und damit der Abstand zwischen dem Kopf und dem Schaft des Spacers eingestellt werden kann.

Eine solche Vorrichtung kann beispielsweise aufweisen:
a) eine formstabile hohle Schaftform, die den Schaft und den Hals des Spacers abbildet, wobei der Hals durch einen ersten Hohlzylinder der Schaftform formbar ist, wobei der ersten Hohlzylinder ein Innengewinde besitzt,
b) einen Port (als die wenigstens eine Einfüllöffnung) zum Einpressen von Polymethylmethacrylat-Knochenzementteig an der formstabilen hohlen Schaftform,
c) eine gummielastische zumindest halbkugelförmige Hohlform, die mit einer ringförmigen formstabilen Auflage verbunden ist, wobei an der gegenüberliegenden Unterseite ein zweiter Hohlzylinder angeordnet ist, der mit der gummielastischen zumindest halbkugelförmigen Hohlform flüssigkeitsdurchlässig verbunden ist und wobei der zweite Hohlzylinder ein Außengewinde besitzt, wobei
d) der zweite Hohlzylinder in den ersten Hohlzylinder geschraubt ist oder schraubbar ist, wodurch der Abstand der hohlen gummielastischen zumindest halbkugelförmigen Hohlform von der Längsachse der Schaftform variiert werden kann, und wobei
   d) der Durchmesser der gummielastischen zumindest halbkugelförmigen Hohlform durch das Volumen des in die Gießform eingepressten Polymethylmethacrylat-Knochenzementteigs stufenlos unter Ausdehnung vergrößert werden kann.

Ein beispielhaftes erfindungsgemäßes Verfahren zur Herstellung von Spacern mit der erfindungsgemäßen Vorrichtung kann die folgenden nacheinander ablaufenden Schritte umfassen:
a) Bereitstellen der Gießform,
b) Mischen eines Knochenzementpulvers mit einer Monomerflüssigkeit in einer Knochenzementkartusche bis sich ein Knochenzementteig gebildet hat,
c) Verbinden der Knochenzementkartusche mit dem Port,
d) Einpressen des Polymethylmethacrylat-Knochenzementteigs unter gleichzeitiger Verdrängung der Luft aus der Gießform in die umgebende Atmosphäre,
e) weiteres Einpressen von Polymethylmethacrylat-Knochenzementteig unter Ausdehnung der gummielastischen, zumindest halbkugelförmigen Hohlform, bis der gewünschte Durchmesser erreicht ist,
f) Aushärten des Polymethylmethacrylat-Knochenzementteigs, und
g) Entnahme des Spacers aus der Gießform.

Ein alternatives beispielhaftes erfindungsgemäßes Verfahren zur Herstellung von Spacern mit der erfindungsgemäßen Vorrichtung kann die folgenden nacheinander ablaufenden Schritte umfassen:
a) Mischen eines Knochenzementpulvers mit einer Monomerflüssigkeit in einer Knochenzementkartusche bis sich ein Knochenzementteig gebildet hat,
b) Verbinden der Knochenzementkartusche mit dem Port,
c) Schrauben eines zweiten Hohlzylinders der Gießform in einen ersten Hohlzylinders der Gießform, bis der gewünschte Abstand zwischen der gummielastischen zumindest halbkugelförmigen Hohlform und der Längsachse des Schaftform eingestellt ist,
d) Einpressen des Polymethylmethacrylat-Knochenzementteigs unter gleichzeitiger Verdrängung der Luft aus der Gießform in die umgebende Atmosphäre,
e) weiteres Einpressen von Polymethylmethacrylat-Knochenzementteig unter Ausdehnung der gummielastischen, zumindest halbkugelförmigen Hohlform, bis der gewünschte Durchmesser erreicht ist,
f) Aushärten des Polymethylmethacrylat-Knochenzementteigs, und
g) Entnahme des Spacers aus der Gießform.

Im Folgenden werden weitere Ausführungsbeispiele der Erfindung anhand von achtundzwanzig schematisch dargestellten Figuren erläutert. Dabei zeigt:
Figur 1: eine schematische perspektivische Querschnittansicht einer beispielhaften ersten erfindungsgemäßen Vorrichtung zum Herstellen eines Hüftgelenkspacers;
Figur 2: eine schematische perspektivische Außenansicht der ersten erfindungsgemäßen Vorrichtung nach Figur 1;
Figur 3: eine schematische perspektivische Querschnittansicht der ersten erfindungsgemäßen Vorrichtung nach den Figuren 1 und 2 mit offenem Ventil;
Figur 4: eine schematische perspektivische Querschnittansicht der ersten erfindungsgemäßen Vorrichtung nach den Figuren 1 bis 3 mit geschlossenem Ventil;
Figur 5: eine schematische Querschnittansicht der ersten erfindungsgemäßen Vorrichtung mit offenem Ventil vor dem Einfüllen von Knochenzementteig in eine Gießform der Vorrichtung;
Figur 6: eine schematische Querschnittansicht der ersten erfindungsgemäßen Vorrichtung während dem Einfüllen von Knochenzementteig in die Gießform;
Figur 7: eine schematische Querschnittansicht der ersten erfindungsgemäßen Vorrichtung mit einer mit Knochenzementteig gefüllten Gießform;
Figur 8: eine schematische Querschnittansicht der ersten erfindungsgemäßen Vorrichtung mit einer mit Knochenzementteig gefüllten Gießform mit geschlossenem Ventil;
Figur 9: eine schematische Querschnittansicht der ersten erfindungsgemäßen Vorrichtung mit geschlossenem Ventil mit nicht expandierter Hohlform nach dem Entfernen einer Knochenzementkartusche und eines Adapterelements von der Gießform;
Figur 10: eine schematische Querschnittansicht der ersten erfindungsgemäßen Vorrichtung mit geschlossenem Ventil mit expandierter Hohlform;
Figur 11: eine schematische perspektivische Ansicht einer beispielhaften zweiten erfindungsgemäßen Vorrichtung zum Herstellen eines Schultergelenkspacers mit geöffneter zweiteiliger Schaftform;
Figur 12: eine schematische perspektivische Querschnittansicht auf die zweite erfindungsgemäße Vorrichtung nach Figur 11;
Figur 13: eine schematische perspektivische Querschnittansicht auf die zweite erfindungsgemäße Vorrichtung nach den Figuren 11 und 12 mit einem verlängertem Adapterelement;
Figur 14: eine schematische perspektivische Ansicht auf die zweite erfindungsgemäße Vorrichtung nach Figur 13 mit geöffneter zweiteiliger Schaftform und mit einem verlängertem Adapterelement;
Figur 15: eine schematische perspektivische Außenansicht auf die geschlossene zweite erfindungsgemäßen Vorrichtung nach den Figuren 11 bis 14;
Figur 16: eine schematische Querschnittansicht der zweiten erfindungsgemäßen Vorrichtung mit eingefülltem Knochenzementteig aus der Knochenzementkartusche;
Figur 17: eine schematische Querschnittansicht der zweiten erfindungsgemäßen Vorrichtung mit geschlossenem Ventil nach dem Entfernen einer Knochenzementkartusche und eines Adapterelements von der Gießform;
Figur 18: eine schematische perspektivische Außenansicht auf die geschlossene zweite erfindungsgemäßen Vorrichtung nach den Figuren 11 bis 17 mit verlängertem Adapterelement und durch gestrichelte Linien angedeuteten expandierten Hohlformabmessungen;
Figur 19: eine schematische perspektivische Querschnittansicht der zweiten erfindungsgemäßen Vorrichtung mit expandierter Hohlform und mit verlängertem Adapterelement;
Figur 20: eine perspektivische Ansicht auf einen Spacer, der mit einer zweiten erfindungsgemäßen Vorrichtung nach den Figuren 11 bis 19 hergestellt wurde;
Figur 21: eine perspektivische Ansicht auf einen Spacer mit verlängertem Hals, der mit einer zweiten erfindungsgemäßen Vorrichtung nach den Figuren 11 bis 19 hergestellt wurde;
Figur 22: eine schematische perspektivische Ansicht auf ein Ventil für eine erfindungsgemäße Vorrichtung im geöffneten Zustand;
Figur 23: eine schematische perspektivische Teilquerschnittansicht auf das Ventil nach Figur 22 im geöffneten Zustand;
Figur 24: eine schematische perspektivische Querschnittansicht durch das Ventil nach den Figuren 22 und 23 im geöffneten Zustand;
Figur 25: eine schematische perspektivische Ansicht auf das Ventil nach den Figuren 22 bis 24 im geschlossenen Zustand;
Figur 26: eine schematische perspektivische Teilquerschnittansicht auf das Ventil nach den Figuren 22 bis 25 im geschlossenen Zustand;
Figur 27: eine schematische perspektivische Querschnittansicht auf das Ventil nach den Figuren 22 bis 26 im geschlossenen Zustand; und
Figur 28: eine schematische Querschnittansicht des Ventils nach den Figuren 22 bis 27 im geschlossenen Zustand.

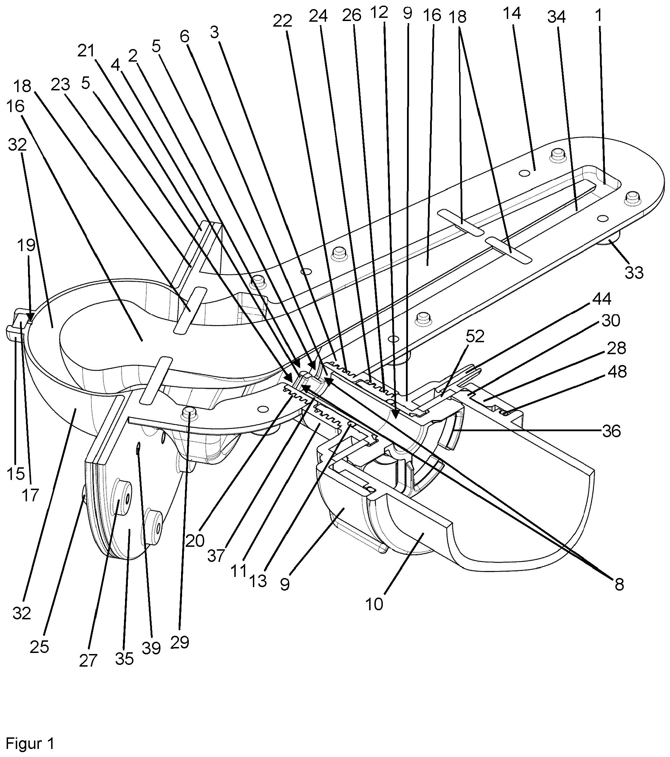

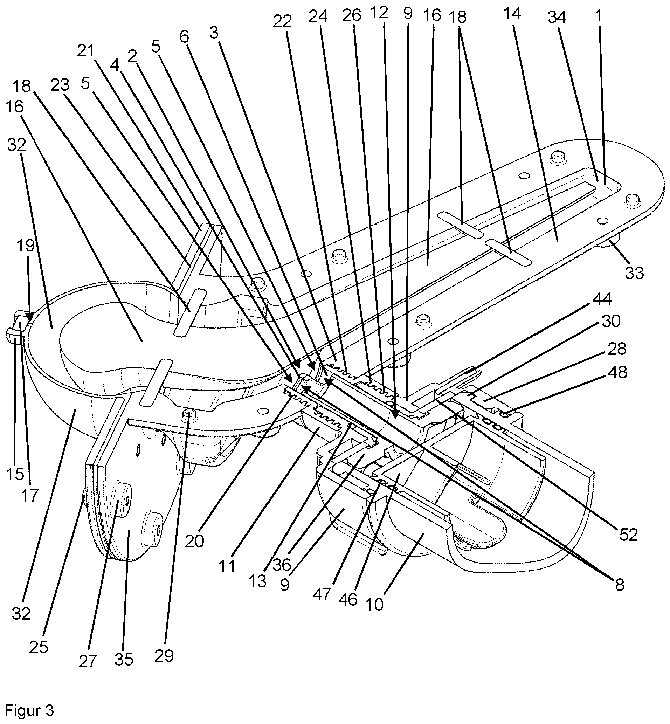

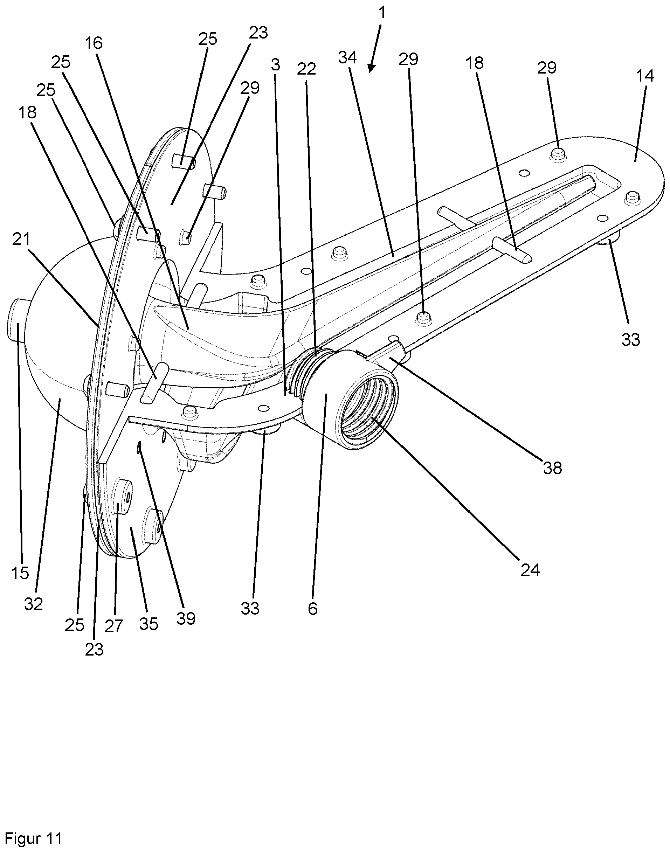

In den Figuren 1 bis 11 sind Abbildungen eines ersten Ausführungsbeispiels einer erfindungsgemäßen Vorrichtung zur Herstellung eines Hüftgelenkspacers und Teile davon in verschiedenen Ansichten gezeigt.

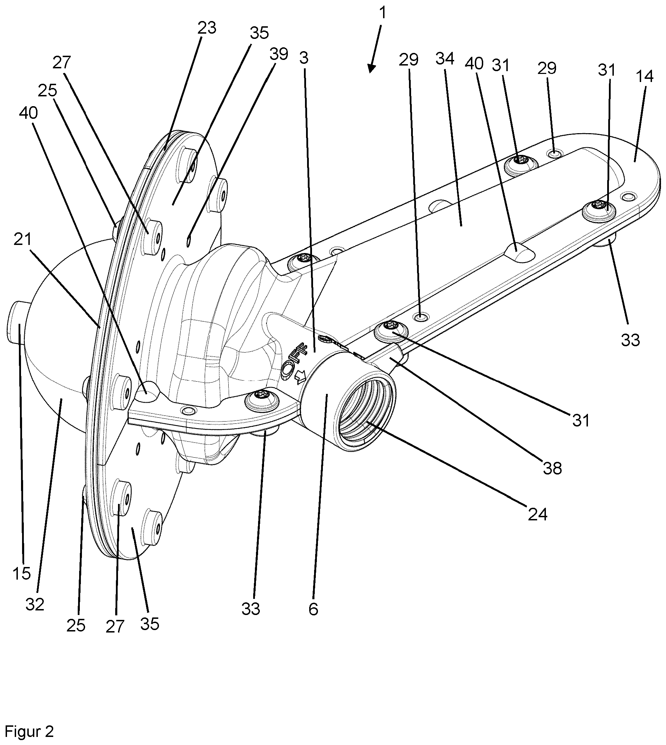

Die erste erfindungsgemäße Vorrichtung ist zur Herstellung eines Spacers für ein Hüftgelenk geeignet und vorgesehen. Die Vorrichtung umfasst eine Gießform 1. Die Gießform 1 kann mehrteilig, insbesondere dreiteilig, aufgebaut sein. In den Figuren 1 und 3 bis 11 ist die Gießform 1 offen oder geschnitten dargestellt, so dass der innere Aufbau der Vorrichtung erkennbar ist. Die Gießform 1 kann eine proximale Hohlform 32 zum Formen eines Kopfs des Spacers und eine distale Schaftform 34 zum Formen eines Schafts des Spacers aufweisen. Die formgebenden Teile der Hohlform 32 können einteilig sein und die formgebenden Teile der Schaftform 34 können zweiteilig sein (siehe Figur 2). In Figur 2 sind alle Teile der Gießform 1 dargestellt. Die Hohlform 32 kann eine kugelflächenförmige Innenfläche in Form einer Halbkugel aufweisen. Die kugelflächenförmige Innenfläche der Hohlform 32 dient als Negativform zum Ausformen einer Gleitfläche des Kopfs des Spacers. Die Hohlform 32 ist erfindungsgemäß expandierbar, um Gleitflächen mit unterschiedlichen Radien beziehungsweise Spacergelenkköpfe (Köpfe von Spacern) mit unterschiedlichen Durchmessern herstellen zu können, die von der kugelflächenförmigen Innenfläche der Hohlform 32 geformt werden.

An einer Seite der Gießform 1 kann eine Einfüllöffnung 2 zum Einfüllen von Knochenzementteig 50 ausgeformt sein, die durch eine in beiden Teilen der Schaftform 34 jeweils Halbkreisförmige zylindrische Öffnung definiert sein kann. Diese Einfüllöffnung 2 kann einen Ventilsitz 3 bilden, der als Teil der Schaftform 34 ausgeführt sein kann. Der Ventilsitz 3 kann also mit der Gießform 1 fest verbunden sein. Durch Einpressen des Knochenzementteigs 50 in die Gießform 1 kann die Hohlform 32 und damit die kugelflächenförmige Innenfläche der Hohlform stufenlos expandiert werden. Dadurch ist eine variable Einstellung des Durchmessers des Kopfs eines mit der Vorrichtung gefertigten Spacers möglich.

Der Ventilsitz 3 kann als ein Hohlzylinder ausgeführt sein, der auf einer in Richtung der Einfüllöffnung 2 ausgerichteten Kopfseite 4 bis auf zwei erste Durchführungen 5 geschlossen ist. Die beiden ersten Durchführungen 5 können viertelkreisförmig geformt sein und können vorzugsweise um 180° bezüglich der Zylinderachse des Ventilsitzes 3 gegeneinander verdreht beziehungsweise versetzt angeordnet sein. Im Inneren des Ventilsitzes 3 kann ein gegen den Ventilsitz 3 axial verdrehbarer Ventilkörper 6 angeordnet sein. Der Ventilkörper 6 kann eine in Richtung der Kopfseite 4 des Ventilsitzes 3 ausgerichtete Dichtfläche 7 beziehungsweise Oberfläche aufweisen. Der Ventilkörper 6 kann als abgestufter Hohlzylinder aufgebaut sein, dessen vorderer Teil in den Ventilsitz 3 eingeschraubt oder eingesteckt sein kann.

In der Dichtfläche 7 können zwei zweite Durchführungen 8 angeordnet sein. Die beiden zweiten Durchführungen 8 können analog den ersten Durchführungen 5 viertelkreisförmig geformt sein und können vorzugsweise um 180° bezüglich der Zylinderachse des Ventilkörpers 6 gegeneinander verdreht angeordnet sein. Der Ventilsitz 3 und der Ventilkörper 6 bilden zusammen ein Ventil der Vorrichtung. In den Ventilkörper 6 kann ein Adapterelement 9 zum flüssigkeitsdichten Verbinden einer Knochenzementkartusche 10 eingeschraubt sein oder eingeschraubt werden (siehe Figuren 1 und 3 bis 8). Die Knochenzementkartusche 10 und das Adapterelement 9 können Teil der erfindungsgemäßen Vorrichtung sein. Der Ventilkörper 6 kann an seiner offenen Seite, die von der Dichtfläche 7 abgewandt ist, als Anschluss 11 zum Verbinden des Adapterelements 9 ausgeformt sein.

Die Knochenzementkartusche 10 kann an ihrer Vorderseite ein Austragsrohr 37 mit einer Austragsöffnung 12 zum Austragen des Knochenzementteigs 50 aus der Knochenzementkartusche 10 aufweisen. Die Austragsöffnung 12 kann zusammen mit dem Austragsrohr 37 in dem Adapterelement 9 angeordnet sein und durch das Austragsrohr 37 begrenzt werden. Das Adapterelement 9 kann die Knochenzementkartusche 10 an ihrer Vorderseite bis auf die Austragsöffnung 12 und gegebenenfalls bis auf einen Vakuumanschluss 44 verschließen. Zur Abdichtung kann vorgesehen sein, dass in dem Adapterelement 9 eine Dichtung 13 in Form eines O-Rings aus Gummi angeordnet ist, die gegen das Austragsrohr 37 abdichtet. An dem ins Innere der Knochenzementkartusche 10 weisende Ende des Austragsrohrs 37 kann ein Mischer 36 mit mehreren Mischflügeln befestigt sein, mit dem der Knochenzementteig 50 im Inneren der Knochenzementkartusche 10 gemischt werden kann, bevor die Knochenzementkartusche 10 an das Ventil angeschlossen wird. Hierzu kann das Austragsrohr 37 axial linear und verdrehbar in der Adapterelement 9 beweglich gelagert sein.

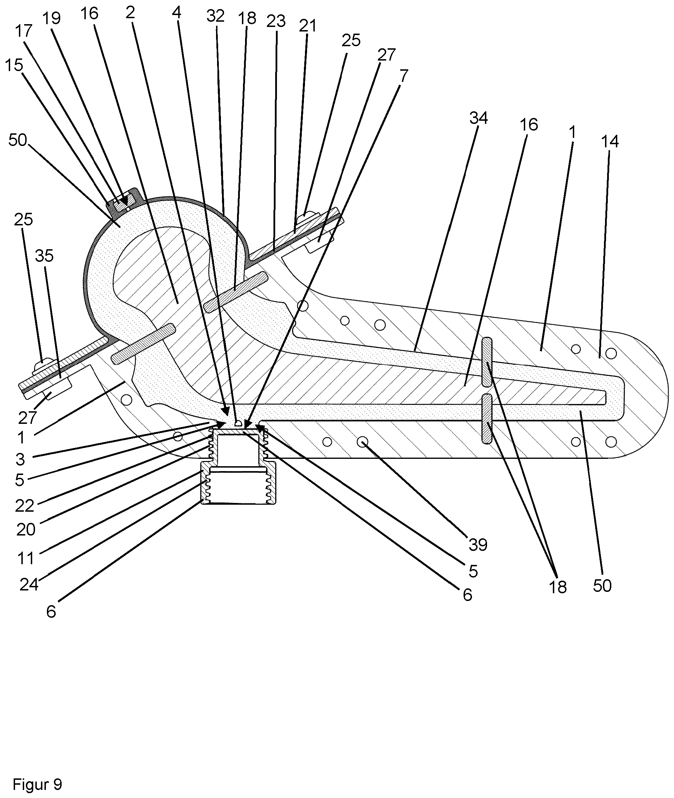

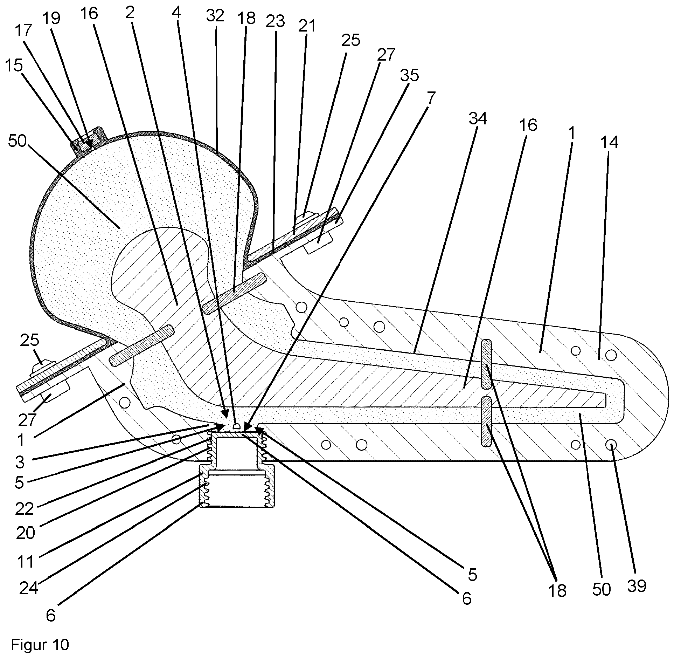

Die Hohlform 32 kann aus einem gummielastischen Kunststoff bestehen. Dadurch kann die Hohlform 32 mit Hilfe des Knochenzementteigs 50 expandiert werden, wie in den Figuren 9 und 10 zu erkennen ist. Figur 9 zeigt dabei eine nicht expandierte Hohlform 32 und Figur 10 eine expandierte Hohlform 32. Die Wandstärke der Hohlform 32 ist gleichmäßig dick, so dass sich die Hohlform 32 gleichmäßig ausdehnt, wenn über den Knochenzementteig 50 ein Druck im Inneren der Hohlform 32 ausgeübt wird.

Die Schaftform 34 kann kostengünstig aus Kunststofffolie gefertigt werden und ist formstabil, kann also im Gegensatz zur Hohlform 32 nicht oder nicht wesentlich durch einen vom Knochenzementteig 50 ausgeübten Druck im Inneren der Gießform 1 expandiert werden. Die Kunststofffolie kann mehrere Schichten aufweisen. Die beiden Teile der Schaftform 34 können über Flansche 14 flächenbündig aneinander befestigt werden. Ebenso können die Hohlform 32 und die Schaftform 34 über einen Flansch 35 der Schaftform 34 und über eine Ringscheibe 23 der Hohlform 32 miteinander flächenbündig verbunden werden. Durch Verbinden der Teile der Gießform 1 über die Flansche 14, 35 und die Ringscheibe 23 kann die Gießform 1 nach außen verschlossen werden. Die Ringscheibe 23 kann über eine segmentierte ringförmige Halterung 21 an den Flansch 35 geschraubt werden. Hierzu können Schrauben 25 in Stutzen 27 mit zu den Schrauben 25 passenden Innengewinden geschraubt werden. Ebenso können die beiden Teile der Schaftform 34 mit Schrauben 31 aneinander befestigt werden, die in Stutzen 33 mit zu den Schrauben 31 passenden Innengewinden geschraubt werden. Zur Vereinfachung der Positionierung der beiden Teile der Schaftform 34 aneinander und der Hohlform 32 an der Schaftform 34 können Stifte 29 vorgesehen sein, die in Ausnehmungen 39 an dem gegenüberliegenden Flansch 14 beziehungsweise Flansch 35 gesteckt werden können. Die Ringscheibe 23 dichtet die Verbindung der Hohlform 32 zur Schaftform 34 wie ein Dichtring ab.

Die Gießform 1 hat zumindest ein Entlüftungselement 15, das jeweils zumindest eine Entlüftungsöffnung 19 aufweisen kann. Zumindest ein Entlüftungselement 15 kann an der Hohlform 32 angeordnet sein, um Luft aus dem Inneren der Gießform 1 herausdrücken zu können. Durch die Entlüftungsöffnung 19 kann Luft oder Gas aus dem Inneren der geschlossenen Gießform 1 entweichen, wenn ein Knochenzementteig 50 durch die Einfüllöffnung 2 in die Gießform 1 eingefüllt wird. In dem Entlüftungselement 15 kann ein Porenfilter 17 angeordnet sein, der für Gase durchlässig ist aber für den Knochenzementteig 50 nicht durchlässig ist. Hierdurch wird verhindert, dass der Knochenzementteig 50 beim Einfüllen in die Gießform 1 durch die Entlüftungsöffnung 19 austreten kann und dadurch zum einen die Form des Kopfs des Spacers beeinträchtigt und zum anderen sich der Druck des Knochenzementteigs 50 in der Hohlform 32 durch Ausfließen von Knochenzementteig 50 aus der Hohlform 32 abbauen kann. Hierdurch wird sichergestellt, dass die Hohlform 32 in dem gewünschten expandierten Zustand verbleibt, während der Knochenzementteig 50 in der Gießform 1 auszuhärten beginnt. Es kann ausreichen, wenn die freie Querschnittsfläche der Entlüftungsöffnung 19 so klein ist, dass der Knochenzementteig 50 aufgrund seiner zähflüssigen Konsistenz nicht durch die Entlüftungsöffnung 19 entweichen kann.

Im Inneren der Gießform 1 kann ein Metallkern 16 platziert werden. Der Metallkern 16 kann aus chirurgischem Stahl oder aus Titan bestehen. Alternativ wäre es theoretisch auch möglich, den Metallkern 16 aus einem Kunststoff wie PMMA zu fertigen. Der Metallkern 16 kann über Haltestifte 18 mit der Schaftform 34 verbunden werden. Der Metallkern 16 kann mit Hilfe der Haltestifte 18 von der Innenwand der Gießform 1 beabstandet sein, so dass der Knochenzementteig 50 den Metallkern 16 vollständig umfließen kann. Der Metallkern 16 bewirkt eine Stabilisierung des Spacers. Die Haltestifte 18 können aus PMMA bestehen. Dieses kann sich mit einem Knochenzementteig 50 aus PMMA irreversibel verbinden.

Der Ventilsitz 3 kann an seiner Innenseite ein Innengewinde 20 aufweisen. An der der Dichtfläche 7 zugewandten vorderen Hälfte des Ventilkörpers 6 kann der Ventilkörper 6 an seiner Außenseite ein zum Innengewinde 20 des Ventilsitzes 3 passendes Außengewinde 22 aufweisen. Der Ventilkörper 6 kann mit seinem Außengewinde 22 in das Innengewinde 20 des Ventilsitzes 3 eingeschraubt werden.

Durch Einschrauben des Ventilkörpers 6 in den Ventilsitz 3 bis zum Anschlag können die ersten Durchführungen 5 und die zweiten Durchführungen 8 in Überdeckung zueinander gebracht werden. Das Ventil befindet sich dann im geöffneten Zustand. Ein Knochenzementteig 50 kann in diesem geöffneten Zustand durch die ersten Durchführungen 5 und durch die zweiten Durchführungen 8 aus der Knochenzementkartusche 10 in die Gießform 1 fließen. Durch eine Vierteldrehung (um 90°) des Ventilkörpers 6 gegen den Ventilsitz 3, das heißt durch ein Herausschrauben des Ventilkörpers 6 aus dem Ventilsitz 3, können die ersten Durchführungen 5 und die zweiten Durchführungen 8 gegeneinander versetzt werden, so dass die Dichtfläche 7 des Ventilkörpers 6 die ersten Durchführungen 5 des Ventilsitzes 3 abdeckt und die geschlossenen Bereiche der Kopfseite 4 des Ventilsitzes 3 die zweiten Durchführungen 8 des Ventilkörpers 6 abdeckt. Das Ventil befindet sich dann im geschlossenen Zustand. Durch den geringen Hub des Ventilkörpers 6 gegen den Ventilsitz 3 bei einer Vierteldrehung, ist der zwischen dem Ventilkörper 6 und dem Ventilsitz 3 entstehende Spalt so schmal (weniger als 1 mm breit), dass ein normaler und erst recht ein hochviskoser Knochenzementteig 50 nicht durch den Spalt dringen können. Dies gilt insbesondere, weil der Knochenzementteig 50 im Spalt von seiner eigentlichen Flussrichtung um 90° abgelenkt wird.

In der Rückseite des Ventilkörpers 6 kann im Anschluss 11 ein Innengewinde 24 angeordnet sein. Das Adapterelement 9 weist an seiner Vorderseite ein zum Innengewinde 24 passendes Außengewinde 26 auf. Das Adapterelement 9 kann so in den Anschluss 11 des Ventilkörpers 6 eingeschraubt werden. Hierdurch kann eine flüssigkeitsdichte Verbindung der Knochenzementkartusche 10 zum Ventilkörper 6 und damit in die Gießform 1 erzeugt werden. Das Innengewinde 20 des Ventilsitzes 3, das Außengewinde 22 des Ventilkörpers 6, das Innengewinde 24 des Ventilkörpers 6 und das Außengewinde 26 des Adapterelements 9 können alle den gleichen Drehsinn aufweisen, das heißt, dass alle diese Gewinde Rechtsgewinde oder Linksgewinde sind. Dadurch kann das Ventil durch Einschrauben des Adapterelements 9 in den Anschluss 11 und gleichsinniges Weiterdrehen des Adapterelements 9 geöffnet werden. Gleichzeitig dichtet auch der Ventilkörper 6 gegen den Ventilsitz 3 ab.

Das Adapterelement 9 kann über ein Rastmittel 28 am Adapterelement 9 mit einer Gegenrastung 30 an einer zylindrischen Wandung der Knochenzementkartusche 10 verbunden werden oder sein. Zur Abdichtung kann eine umlaufende Dichtung 48 vorgesehen sein, die die zylindrische Wandung der Knochenzementkartusche 10 gegen das Adapterelement 9 abdichtet.

Die Gießform 1 weist die Hohlform 32 zum Ausformen des Gelenkkopfs (Kopfs) oder einer Gleitfläche des Gelenkkopfs des Spacers und die Schaftform 34 zum Ausformen des Schafts eines Spacers auf. Des Weiteren kann in der Gießform 1 im Bereich der Einfüllöffnung 2 eine Aussparung für einen Hebel 38 des Ventilkörpers 6 angeordnet sein. Der Hebel 38 kann mit dem Ventilkörper 6 verbunden sein. Der Ventilkörper 6 kann mit dem Hebel 38 im Ventilsitz 3 gedreht werden. Die Aussparung ist vorzugsweise genau so groß, dass den Ventilkörper 6 nur um maximal eine Vierteldrehung gegen den Ventilsitz 3 gedreht werden kann. Dadurch kann das Ventil mit Hilfe des Hebels 38 manuell von außen vom geöffneten Zustand in den geschlossenen Zustand beziehungsweise vom geschlossenen Zustand in den geöffneten Zustand überführt werden.

Im Bereich der Flansche 14 können in der Schaftform 34 Formen 40 für Kavitäten angeordnet sein, in denen die Haltestifte 18 angeordnet werden können.

In dem Adapterelement 9 kann ein Vakuumanschluss 44 angeordnet sein, mit dem ein Innenraum der Knochenzementkartusche 10, in dem der Knochenzementteig 50 gemischt wird, evakuiert werden kann. Dadurch kann der Knochenzementteig 50 unter Vakuum gemischt werden.

Im zylindrischen Innenraum der Knochenzementkartusche 10 kann ein Kolben 46 zum Austreiben des Knochenzementteigs 50 aus der Knochenzementkartusche 10 durch das Ventil in die Gießform 1 angeordnet sein. Hierzu kann der Kolben 46 an seiner Außenseite zylindrisch geformt und über zwei umlaufende Dichtungen 47 gegen den zylindrischen Innenraum abgedichtet sein. Durch Vortreiben des Kolbens 46 kann der Knochenzementteig 50 aus der Austragsöffnung 12 der Knochenzementkartusche 10 in beziehungsweise durch das geöffnete Ventil gepresst werden.

In dem Adapterelement 9 kann eine Porenscheibe 52 angeordnet sein. Die Porenscheibe 52 ist für den Knochenzementteig 50 und seine Ausgangskomponenten undurchlässig. Der Vakuumanschluss 44 kann von der Porenscheibe 52 abgedeckt sein. Hierdurch wird verhindert, dass ein Knochenzementpulver als Ausgangskomponente des Knochenzementteigs 50 in den Vakuumanschluss 44 eindringen kann.

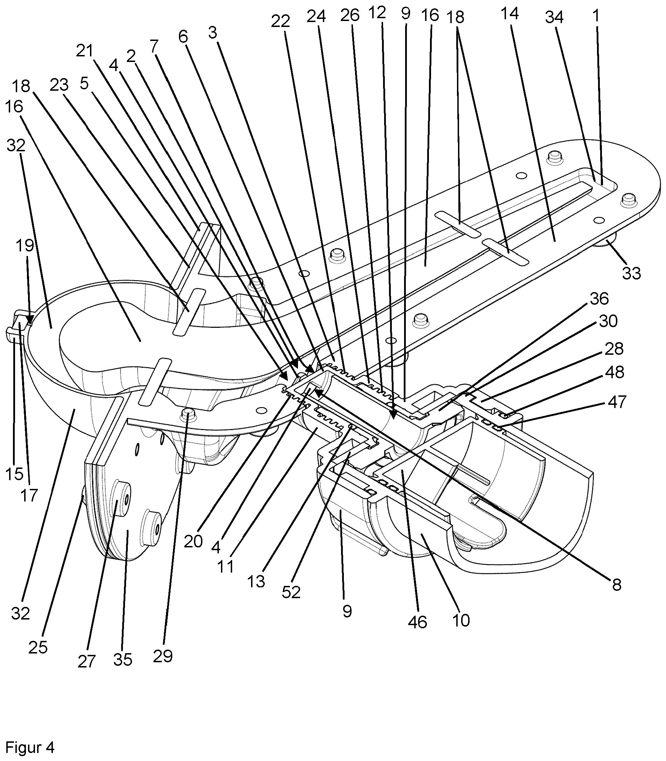

Der Ablauf eines erfindungsgemäßen Verfahrens ist in den Figuren 4 bis 10 anhand der ersten erfindungsgemäßen Vorrichtung dargestellt. Zunächst kann der Metallkern 16 mit den Haltestiften 18 in der Schaftform 34 positioniert werden. Hierzu können die Haltestifte 18 an einem Ende zwischen den beiden Teilen der Schaftform 34 in den durch die Formen 40 gebildeten Kavitäten angeordnet und gehalten und mit dem anderen Ende in passende Bohrungen im Metallkern 16 angeordnet werden. Zur Befestigung der beiden Teile der Schaftform 34 aneinander können die Flansche 14 der beiden Teile der Schaftform 34 zunächst über die Stifte 29 in den Ausnehmungen 39 positioniert und anschließend die beiden Teile der Schaftform 34 miteinander mit Hilfe der Schrauben 31 aneinandergeschraubt werden. Die Gießform 1 kann dann geschlossen werden, indem die Hohlform 32 an der Schaftform 34 befestigt wird. Hierzu kann die Ringscheibe 23 an den Flansch 35 angelegt werden. Die beiden halben Ringe der Halterung 21 können anschließend an die Ringscheibe 23 angelegt und über die Stifte 29 und die Ausnehmungen 39 positioniert werden. Danach kann durch anziehen der Schrauben 25 die Hohlform 32 an der Schaftform 34 befestigt werden, wobei die Ringscheibe 23 die Verbindung abdichtet. Die Gießform 1 ist dann mit dem Metallkern 16 darin verschlossen und kann bereitgestellt werden, um einen Spacer zu formen.

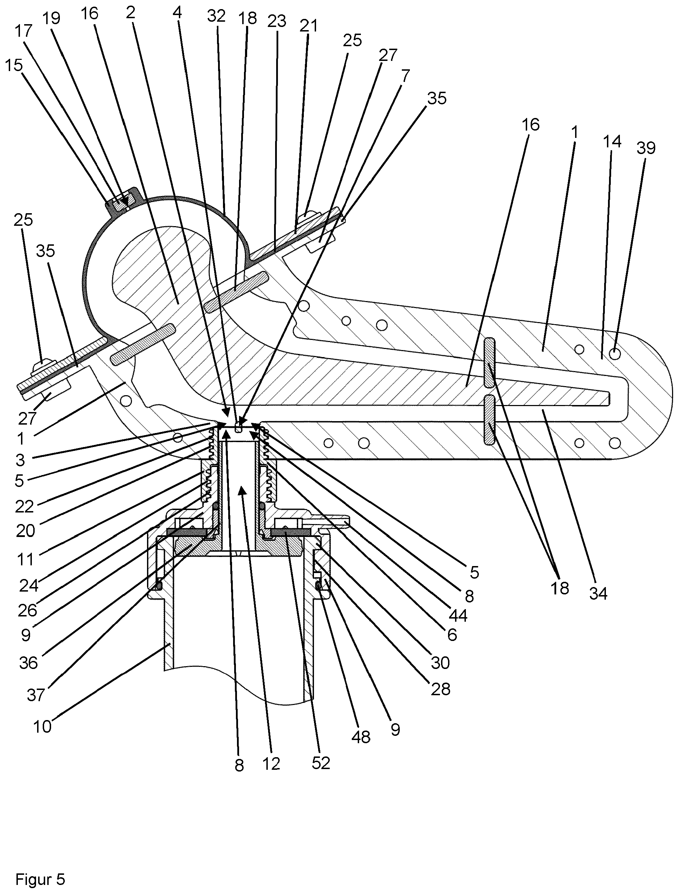

Ein Knochenzementteig 50 kann in der Knochenzementkartusche 10 unter Vakuum gemischt werden. Anschließend kann die Knochenzementkartusche 10 mit dem Adapterelement 9 in den Anschluss 11 des Ventilkörpers 6 geschraubt werden. Beim Einschrauben der Adapterelements 9 kann das Ventil in die geöffnete Stellung überführt werden, indem der Ventilkörper 6 bis zum Anschlag in den Ventilsitz 3 geschraubt wird. Diese Situation ist in Figur 1 und 5 dargestellt.

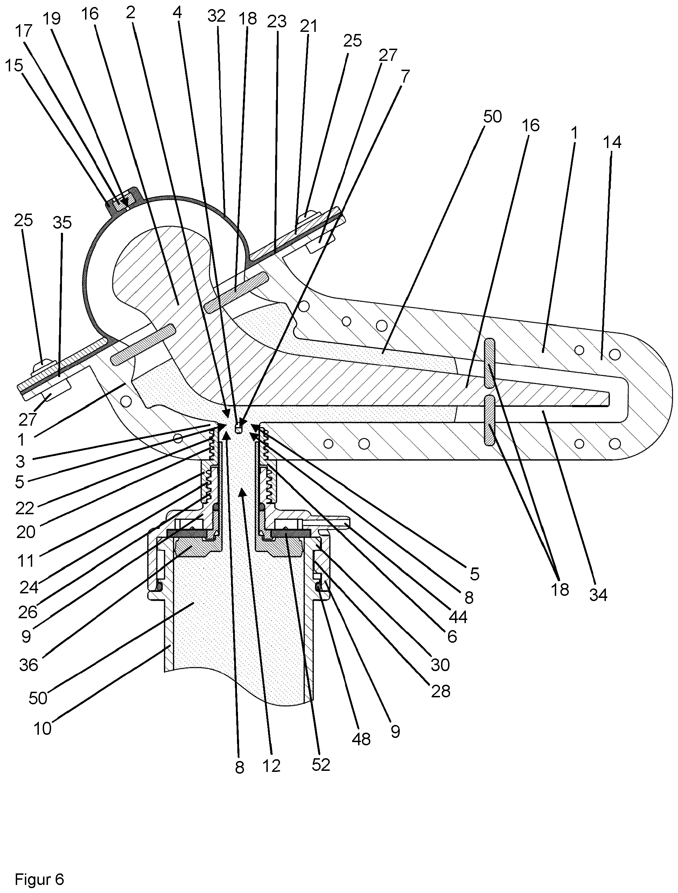

Anschließend wird der Knochenzementteig 50 in die Gießform 1 gepresst. Dies kann folgendermaßen geschehen: durch Vortreiben des Kolbens 46 kann der Knochenzementteig 50 aus der Knochenzementkartusche 10 durch das Ventil und durch die sich in Deckung befindlichen ersten Durchführungen 5 und zweiten Durchführungen 8 in die Gießform 1 gepresst werden. Diese Situation ist in Figur 6 gezeigt. Durch Schließen des Ventils durch manuelles Bedienen des Hebels 38 und dadurch Drehen des Ventilkörpers 6 um eine Vierteldrehung gegen den Ventilsitz 3 kann zwischendurch eine neue Knochenzementkartusche 10 angesetzt werden, falls das Volumen des Knochenzementteigs 50 einer einzigen Knochenzementkartusche 10 nicht ausreicht, um die Gießform 1 vollständig zu füllen. Dabei kann der in der Gießform 1 enthaltene Knochenzementteig 50 nicht wieder ausfließen, da die ersten Durchgänge 5 und die zweiten Durchgänge 8 in der geschlossenen Stellung des Ventils abgedeckt sind und der Spalt dazwischen nicht ausreicht, um den viskosen Knochenzementteig 50 hindurchfließen lassen zu können. Gleichzeitig wird ein Druck des Knochenzementteigs 50 im Inneren der Hohlform 32 durch das geschlossene Ventil aufrechterhalten.

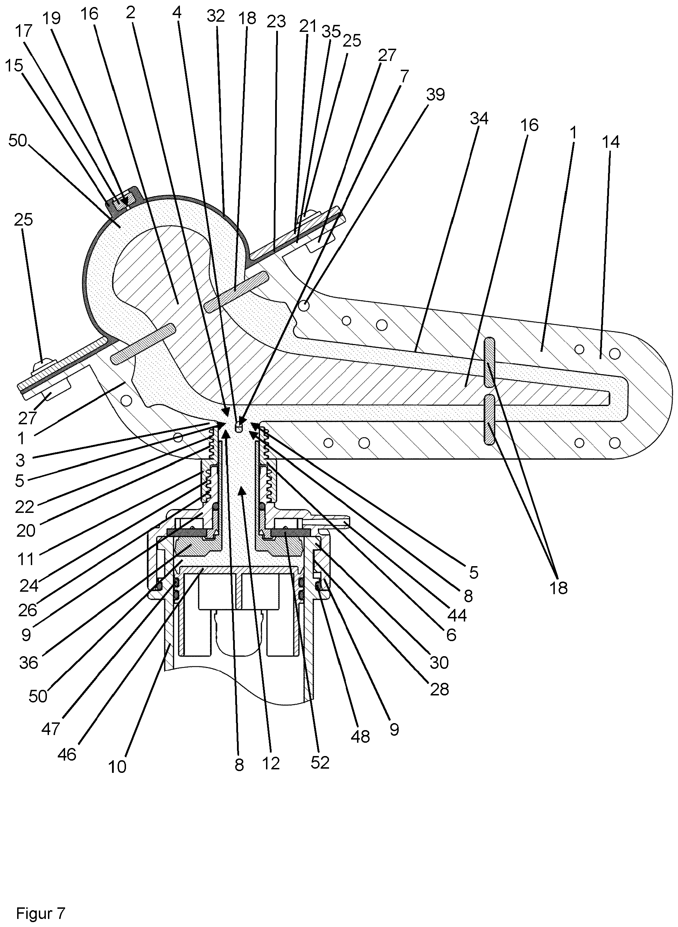

Irgendwann ist die Gießform 1 mit dem Knochenzementteig 50 gefüllt. Diese Situation ist in Figur 7 gezeigt. Luft oder Gas aus der Gießform 1 entweicht durch das Entlüftungselement 15 beziehungsweise durch die Entlüftungsöffnungen 19 in der Hohlform 32. Durch weiteres Einpressen von Knochenzementteig 50 kann die Hohlform 32 bis zur gewünschten Größe expandiert werden (siehe Figur 10 im Vergleich zur Figur 9). Die radiale Ausdehnung kann mit Hilfe einer Prüflehre oder einer Schieblehre (nicht gezeigt) zum Messen des aktuellen Durchmessers der Hohlform 32 bestimmt werden. Bei bekannter Wandstärke der Hohlform 32 ist dadurch auch der aktuelle Durchmesser der kugelflächenförmigen Innenfläche der Hohlform 32 bestimmbar. Alternativ oder zusätzlich kann auch eine formstabile Gegenform (nicht gezeigt) mit bekanntem und definiertem Durchmesser vorgesehen sein, in die die Hohlform 32 eingelegt werden kann, so dass sich die Hohlform 32 nur so weit ausdehnen kann, bis sie an der Gegenform anliegt. Hierdurch kann auf einfache Weise der gewünschte Durchmesser des Kopfs des mit der Vorrichtung hergestellten Spacers einstellen. Wenn die gewünschte radiale Ausdehnung kugelflächenförmigen Innenfläche der Hohlform 32 erreicht ist, kann das Ventil geschlossen werden.

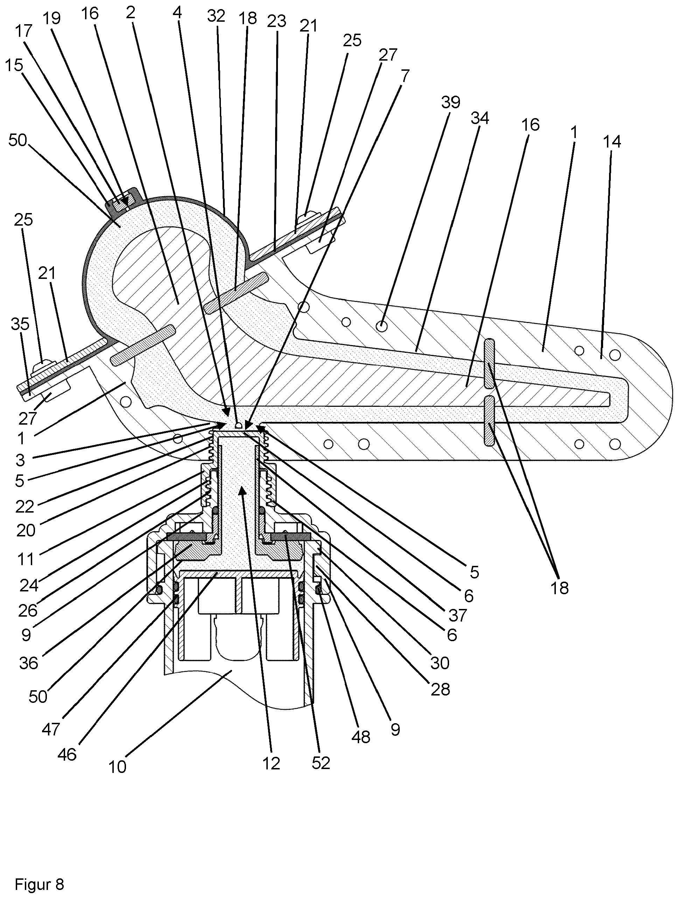

Durch Schließen des Ventils mit dem Hebel 38 kann der Knochenzementteig 50 abgeschert beziehungsweise abgeschnitten werden. Diese Situation ist in den Figuren 4 und 8 dargestellt. Die Knochenzementkartusche 10 kann abgeschraubt und entfernt werden. Eventuell verbliebene dünne Verbindungen können ohne weiteres abreißen oder abbrechen. Diese Situation ist in Figur 9 und 10 dargestellt.

In diesem Zustand härtet der Knochenzementteig 50 in der Gießform 1 aus. Die Größe beziehungsweise der Durchmesser des Kopfs des Spacers entspricht dabei dem Durchmesser der kugelflächenförmigen Innenfläche der expandierten Hohlform 32. Um Unebenheiten der Gleitfläche des Spacers zu vermeiden, ist es erfindungsgemäß bevorzugt, dass auch bei der Herstellung von Spacern mit Köpfen mit kleinstem Durchmesser die Hohlform 32 bereits geringfügig expandiert ist und dadurch der Knochenzementteig 50 in die Gießform 1 unter Druck steht, während er aushärtet.

Anschließend wird der so geformte Spacer aus der Gießform 1 entnommen. Die vorspringenden Haltestifte 18 können abgeschnitten werden. Ebenso kann ein durch den Ventilsitz 3 und die ersten Durchgänge 5 verursachter Anguss abgeschnitten und entfernt werden. Auch können Spitzen oder Unebenheiten, die durch die Entlüftungsöffnung 19 verursacht werden, entfernt werden. Die Oberfläche des Spacers kann poliert werden und/oder beschichtet werden, beispielsweise mit Antibiotika.

Anstatt einer Gießform 1 zum Formen eines Hüftgelenkspacers kann ohne weiteres auch eine Gießform zum Formen eines anderen Spacers verwendet werden.

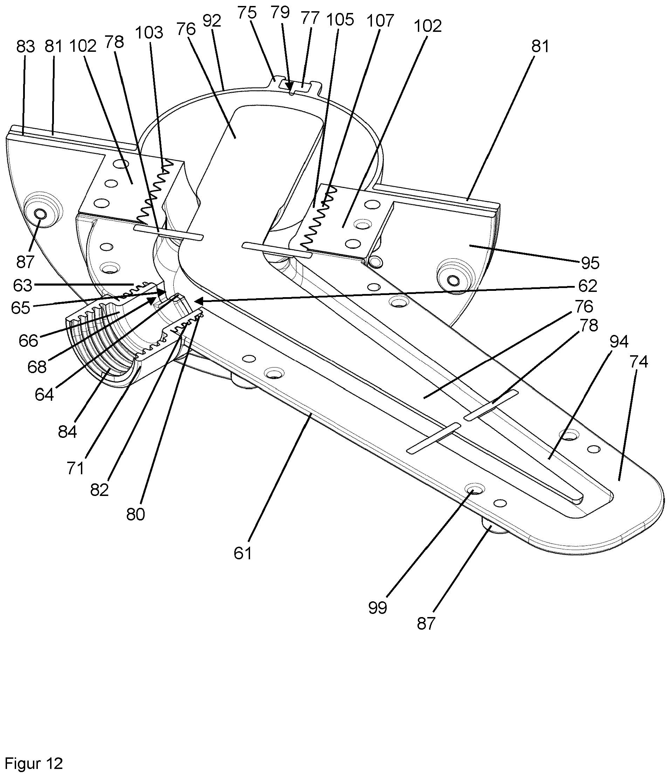

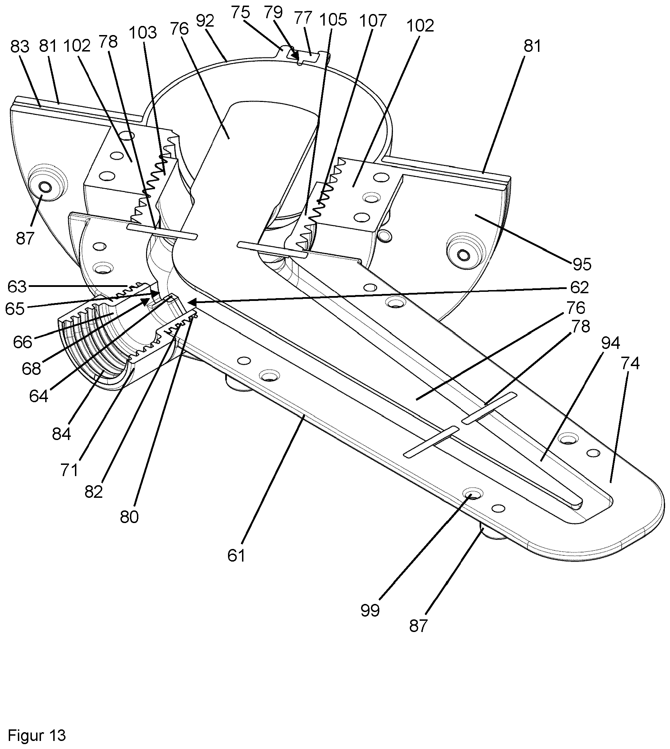

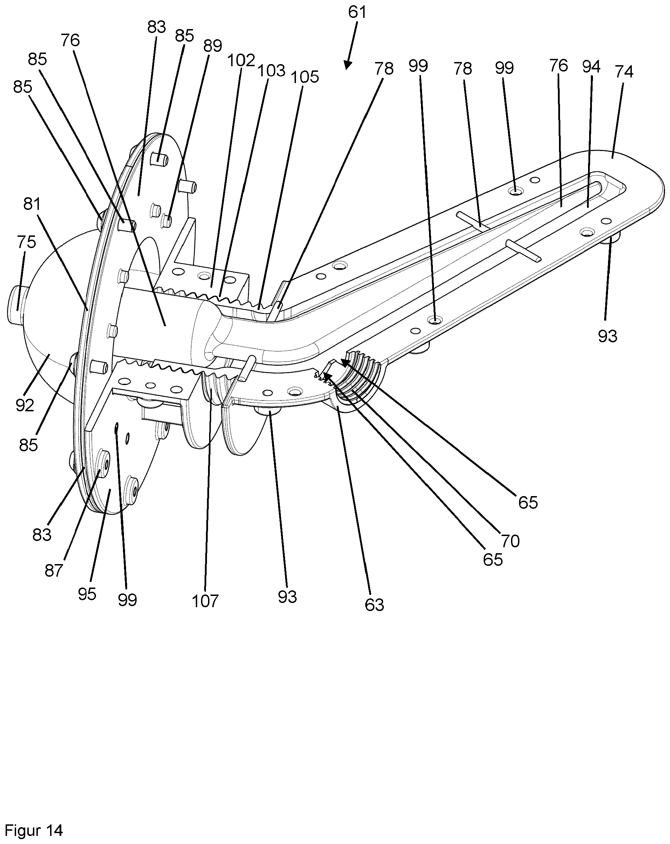

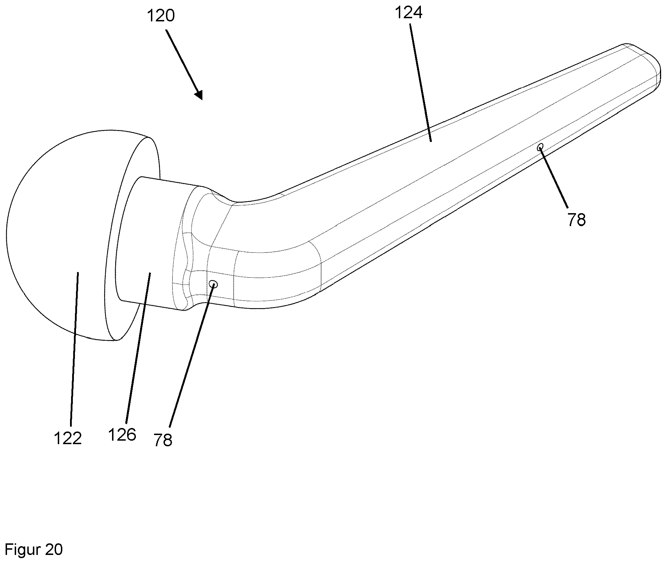

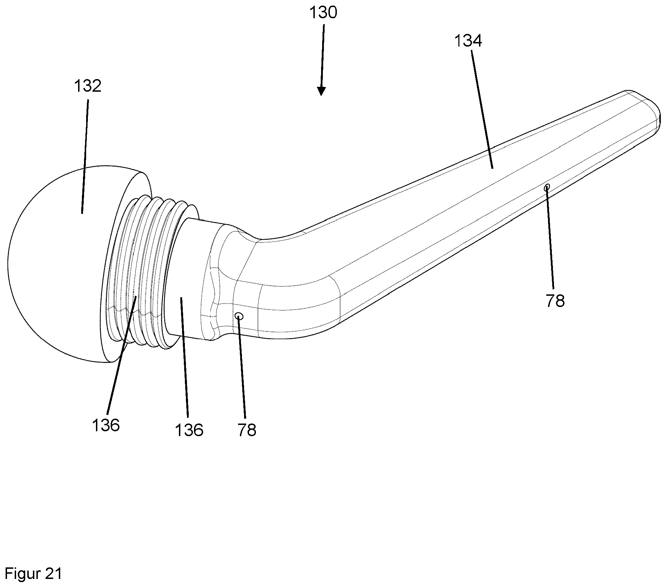

In den Figuren 12 bis 19 sind Abbildungen eines zweiten Ausführungsbeispiels einer erfindungsgemäßen Vorrichtung zur Herstellung eines Spacers für ein Schultergelenk und Teile davon in verschiedenen Ansichten gezeigt. Die Figuren 20 und 21 zeigen einen Schultergelenkspacer, der mit einer solchen zweiten erfindungsgemäßen Vorrichtung hergestellt wurde, als Resultat eines erfindungsgemäßen Verfahrens, dessen Verfahrensschritte in den Figuren 14 bis 21 gezeigt sind.

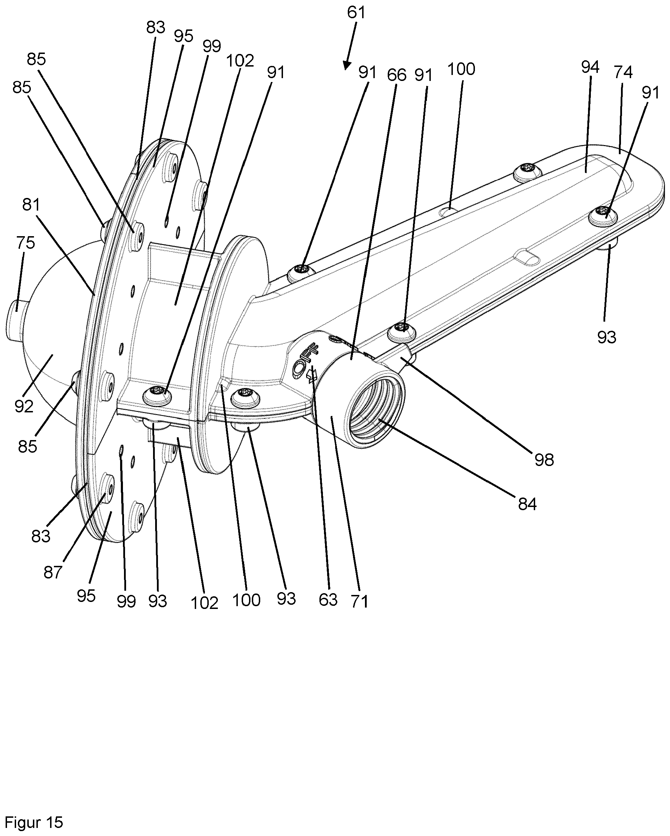

Die zweite erfindungsgemäße Vorrichtung ist zur Herstellung eines Spacers 120, 130 (siehe Figuren 20 und 21) für ein Schultergelenk geeignet und vorgesehen. Die Vorrichtung umfasst eine Gießform 61. Die Gießform 61 kann mehrteilig, insbesondere vierteilig, aufgebaut sein. In den Figuren 12 bis 14 und 16 bis 19é ist die Gießform 61 offen oder geschnitten dargestellt, so dass der innere Aufbau der Vorrichtung erkennbar ist. Die Gießform 61 kann eine proximale Hohlform 92 zum Formen eines Kopfs des Spacers und eine distale Schaftform 94 zum Formen eines Schafts des Spacers aufweisen. Die formgebenden Teile der Hohlform 92 können einteilig sein und die formgebenden Teile der Schaftform 94 können zweiteilig sein (siehe Figur 15). In Figur 15 sind alle Teile der Gießform 61 dargestellt. Die Hohlform 92 kann eine kugelflächenförmige Innenfläche in Form einer Halbkugel aufweisen. Die kugelflächenförmige Innenfläche der Hohlform 92 dient als Negativform zum Ausformen einer Gleitfläche des Kopfs des Spacers. Die Hohlform 92 ist erfindungsgemäß expandierbar, um Gleitflächen mit unterschiedlichen Radien beziehungsweise Spacergelenkköpfe (Köpfe von Spacern) mit unterschiedlichen Durchmessern herstellen zu können, die von der kugelflächenförmigen Innenfläche der Hohlform 92 geformt werden.

Im Unterschied zu der ersten erfindungsgemäßen Vorrichtung kann die Gießform 61 zusätzlich ein Adapterelement 102 mit einem Innengewinde 103 aufweisen. Das Adapterelement 102 kann mit seinem Innengewinde 103 auf einen Hohlzylinder 105 mit passendem Außengewinde 107 geschraubt sein. Der Stutzen 105 kann durch die beiden Teile der Schaftform 94 geformt werden. Das Adapterelement 102 kann zu einer variablen Beabstandung der Hohlform 92 von der Schaftform 94 dienen. Das Adapterelement 102 und die innere Form des Hohlzylinders 105 formen den Hals eines mit der zweiten erfindungsgemäßen Vorrichtung hergestellten Spacers 120, 130. Durch ein Schrauben des Adapterelements 102 gegen den Hohlzylinder 105 kann die Länge des Halses des Spacers verändert werden (siehe Figuren 20 und 21). Ein solches Adapterelement 102 mit einem Hohlzylinder 105 zum Einstellen der Länge des Halses des Spacers kann grundsätzlich auch bei der ersten erfindungsgemäßen Vorrichtung eingesetzt werden.

An einer Seite der Gießform 61 kann eine Einfüllöffnung 62 zum Einfüllen von Knochenzementteig 50 ausgeformt sein, die durch eine in beiden Teilen der Schaftform 94 jeweils Halbkreisförmige zylindrische Öffnung definiert sein kann. Diese Einfüllöffnung 62 kann einen Ventilsitz 63 bilden, der als Teil der Schaftform 94 ausgeführt sein kann. Der Ventilsitz 63 kann also mit der Gießform 61 fest verbunden sein. Durch Einpressen des Knochenzementteigs 50 in die Gießform 61 kann die Hohlform 92 und damit die kugelflächenförmige Innenfläche der Hohlform stufenlos expandiert werden. Dadurch ist eine variable Einstellung des Durchmessers des Kopfs eines mit der Vorrichtung gefertigten Spacers möglich.

Der Ventilsitz 63 kann als ein Hohlzylinder ausgeführt sein, der auf einer in Richtung der Einfüllöffnung 62 ausgerichteten Kopfseite 64 bis auf zwei erste Durchführungen 65 geschlossen ist. Die beiden ersten Durchführungen 65 können viertelkreisförmig geformt sein und können vorzugsweise um 180° bezüglich der Zylinderachse des Ventilsitzes 63 gegeneinander verdreht angeordnet sein. Im Inneren des Ventilsitzes 63 kann ein gegen den Ventilsitz 63 axial verdrehbarer Ventilkörper 66 angeordnet sein. Der Ventilkörper 66 kann eine in Richtung der Kopfseite 64 des Ventilsitzes 63 ausgerichtete Dichtfläche 67 beziehungsweise Oberfläche aufweisen. Der Ventilkörper 66 kann als abgestufter Hohlzylinder aufgebaut sein, dessen vorderer Teil in den Ventilsitz 63 eingeschraubt oder eingesteckt sein kann.

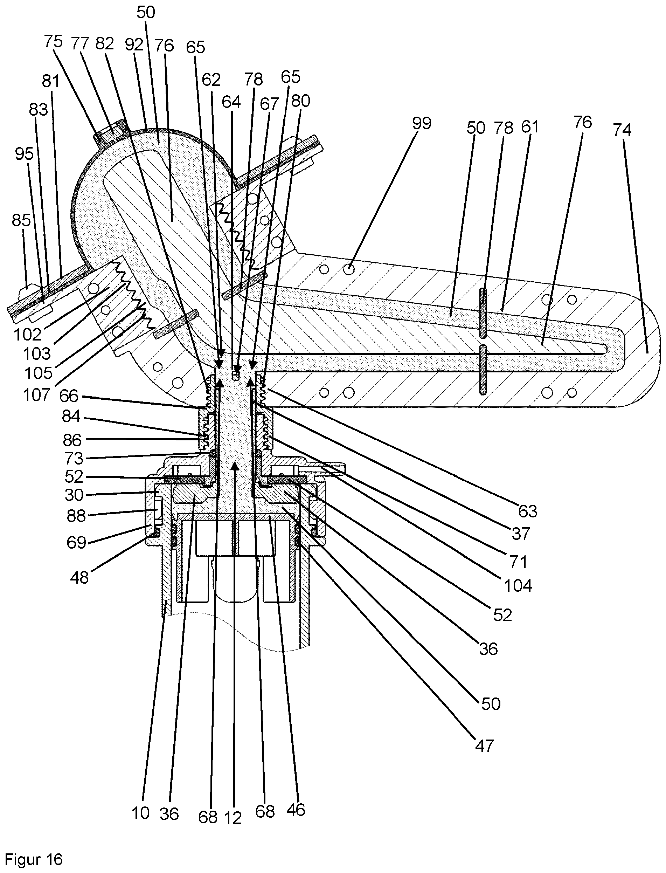

In der Dichtfläche 67 können zwei zweite Durchführungen 68 angeordnet sein. Die beiden zweiten Durchführungen 68 können analog den ersten Durchführungen 65 viertelkreisförmig geformt sein und können vorzugsweise um 180° bezüglich der Zylinderachse des Ventilkörpers 66 gegeneinander verdreht beziehungsweise versetzt angeordnet sein. Der Ventilsitz 63 und der Ventilkörper 66 bilden zusammen ein Ventil der Vorrichtung. In den Ventilkörper 66 kann ein Adapterelement 69 zum flüssigkeitsdichten Verbinden einer Knochenzementkartusche 10 eingeschraubt sein oder eingeschraubt werden (siehe Figur 16). Die Knochenzementkartusche 10 und das Adapterelement 69 können Teil der erfindungsgemäßen Vorrichtung sein. Der Ventilkörper 66 kann an seiner offenen Seite, die von der Dichtfläche 67 abgewandt ist, als Anschluss 71 zum Verbinden des Adapterelements 69 ausgeformt sein. Es kann die gleiche Knochenzementkartusche 10 verwendet werden, die auch in dem ersten Ausführungsbeispiel beschrieben wurde, um die Gießform 61 mit Knochenzementteig 50 zu befüllen. Daher werden für beide Ausführungen die gleichen Bezugszeichen verwendet.

Die Knochenzementkartusche 10 kann an ihrer Vorderseite ein Austragsrohr 37 mit einer Austragsöffnung 12 zum Austragen des Knochenzementteigs 50 aus der Knochenzementkartusche 10 aufweisen. Die Austragsöffnung 12 kann zusammen mit dem Austragsrohr 37 in dem Adapterelement 9 angeordnet sein und durch das Austragsrohr 37 begrenzt werden. Das Adapterelement 9 kann die Knochenzementkartusche 10 an ihrer Vorderseite bis auf die Austragsöffnung 12 und gegebenenfalls bis auf einen Vakuumanschluss 104 verschließen. Zur Abdichtung kann vorgesehen sein, dass in dem Adapterelement 9 eine Dichtung 73 in Form eines O-Rings aus Gummi angeordnet ist, die gegen das Austragsrohr 37 abdichtet. An dem ins Innere der Knochenzementkartusche 10 weisende Ende des Austragsrohrs 37 kann ein Mischer 36 mit mehreren Mischflügeln befestigt sein, mit dem der Knochenzementteig 50 im Inneren der Knochenzementkartusche 10 gemischt werden kann, bevor die Knochenzementkartusche 10 an das Ventil angeschlossen wird. Hierzu kann das Austragsrohr 37 axial linear und verdrehbar in der Adapterelement 9 beweglich gelagert sein.

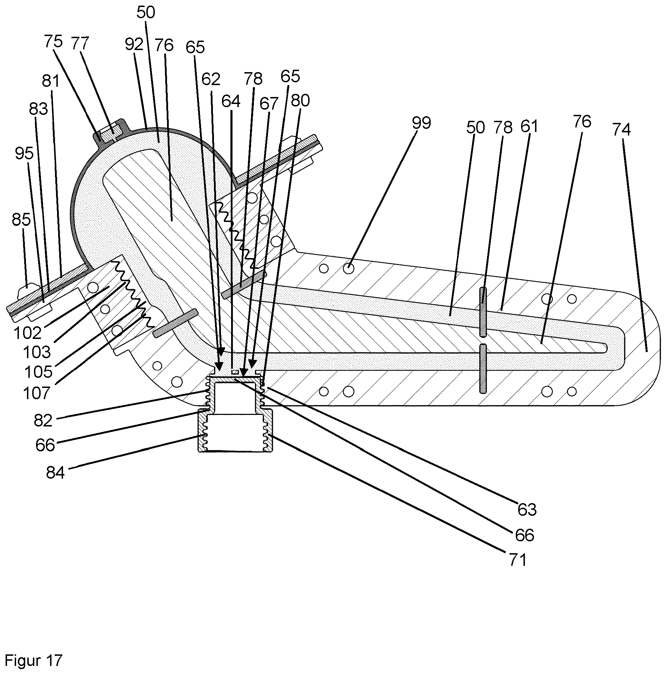

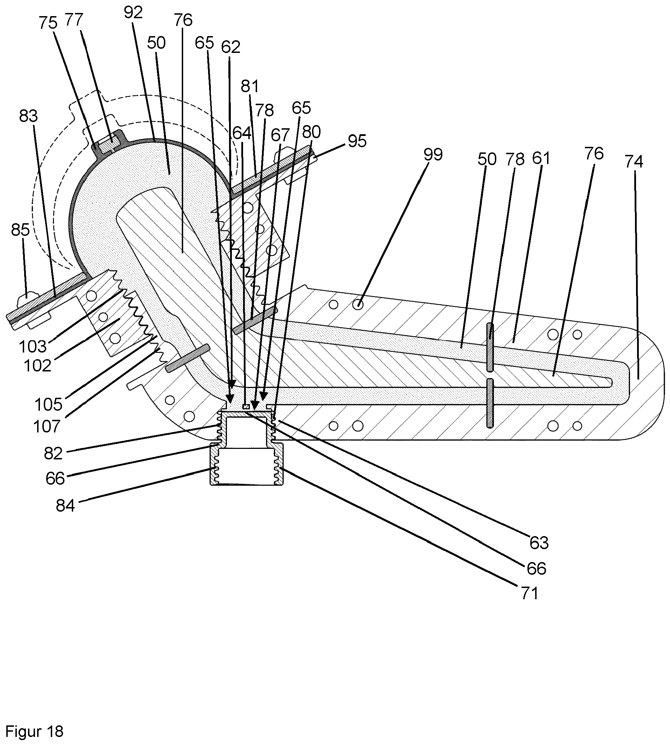

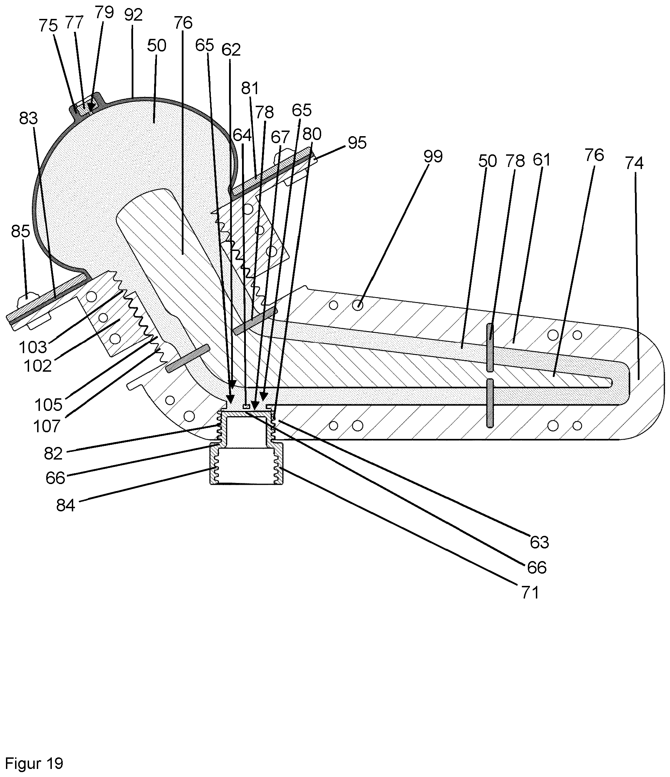

Die Hohlform 92 kann aus einem gummielastischen Kunststoff bestehen. Dadurch kann die Hohlform 92 mit Hilfe des Knochenzementteigs 50 expandiert werden, wie in den Figuren 17, 18 und 19 angedeutet ist. Figur 17 zeigt dabei eine nicht expandierte Hohlform 92, Figur 18 Abmessungen für zusätzliche mögliche expandierte Hohlformen 92 als gestrichelte Linien und Figur 19 eine expandierte Hohlform 92. Die Wandstärke der Hohlform 92 ist gleichmäßig dick, so dass sich die Hohlform 92 gleichmäßig ausdehnt, wenn über den Knochenzementteig 50 ein Druck im Inneren der Hohlform 92 ausgeübt wird.

Die Schaftform 94 und das Adapterelement 102 können kostengünstig aus Kunststofffolie gefertigt werden und sind formstabil, können also im Gegensatz zur Hohlform 92 nicht oder nicht wesentlich durch einen vom Knochenzementteig 50 ausgeübten Druck im Inneren der Gießform 61 expandiert werden. Die Kunststofffolie kann mehrere Schichten aufweisen. Die beiden Teile der Schaftform 94 können über Flansche 74 flächenbündig aneinander befestigt werden. Ebenso können die Hohlform 92 und das Adapterelement 102 über einen Flansch 95 des Adapterelements 102 und über eine Ringscheibe 83 der Hohlform 92 miteinander flächenbündig verbunden werden. Durch Verbinden der Teile der Gießform 61 über die Flansche 74, 95 und die Ringscheibe 83 und über das Innengewinde 103 und das Außengewinde 107 kann die Gießform 61 verbunden und nach außen verschlossen werden. Die Ringscheibe 83 kann über eine segmentierte ringförmige Halterung 81 an den Flansch 95 geschraubt werden. Hierzu können Schrauben 85 in Stutzen 87 mit zu den Schrauben 85 passenden Innengewinden geschraubt werden. Ebenso können die beiden Teile der Schaftform 94 mit Schrauben 91 aneinander befestigt werden, die in Stutzen 93 mit zu den Schrauben 91 passenden Innengewinden geschraubt werden. Zur Vereinfachung der Positionierung der beiden Teile der Schaftform 94 aneinander und der Hohlform 92 an dem Flansch 95 des Adapterelements 102 können Stifte 89 vorgesehen sein, die in Ausnehmungen 99 an dem gegenüberliegenden Flansch 74 beziehungsweise Flansch 95 gesteckt werden können. Die Ringscheibe 83 dichtet die Verbindung der Hohlform 92 zum Adapterelement 102 wie ein Dichtring ab.

Die Gießform 61 hat zumindest ein Entlüftungselement 75, das jeweils zumindest eine Entlüftungsöffnung 79 aufweisen kann. Zumindest ein Entlüftungselement 75 kann an der Hohlform 92 angeordnet sein, um Luft aus dem Inneren der Gießform 61 herausdrücken zu können. Durch die Entlüftungsöffnung 79 kann Luft oder Gas aus dem Inneren der geschlossenen Gießform 61 entweichen, wenn ein Knochenzementteig 50 durch die Einfüllöffnung 62 in die Gießform 61 eingefüllt wird. In dem Entlüftungselement 75 kann ein Porenfilter 77 angeordnet sein, der für Gase durchlässig ist aber für den Knochenzementteig 50 nicht durchlässig ist. Hierdurch wird verhindert, dass der Knochenzementteig 50 beim Einfüllen in die Gießform 61 durch die Entlüftungsöffnung 79 austreten kann und dadurch zum einen die Form des Kopfs des Spacers beeinträchtigt und zum anderen sich der Druck des Knochenzementteigs 50 in der Hohlform 92 durch Ausfließen von Knochenzementteig 50 aus der Hohlform 92 abbauen kann. Hierdurch wird sichergestellt, dass die Hohlform 92 in dem gewünschten expandierten Zustand verbleibt, während der Knochenzementteig 50 in der Gießform 61 auszuhärten beginnt. Es kann ausreichen, wenn die freie Querschnittsfläche der Entlüftungsöffnung 79 so klein ist, dass der Knochenzementteig 50 aufgrund seiner zähflüssigen Konsistenz nicht durch die Entlüftungsöffnung 79 entweichen kann.

Im Inneren der Gießform 61 kann ein Metallkern 76 platziert werden. Der Metallkern 76 kann aus chirurgischem Stahl oder aus Titan bestehen. Alternativ wäre es theoretisch auch möglich, den Metallkern 76 aus einem Kunststoff wie PMMA zu fertigen. Der Metallkern 76 kann über Haltestifte 78 mit der Schaftform 94 verbunden werden. Der Metallkern 76 kann mit Hilfe der Haltestifte 78 von der Innenwand der Gießform 61 beabstandet sein, so dass der Knochenzementteig 50 den Metallkern 76 vollständig umfließen kann. Der Metallkern 76 bewirkt eine Stabilisierung des Spacers 120, 130. Die Haltestifte 78 können aus PMMA bestehen. Dieses kann sich mit einem Knochenzementteig 50 aus PMMA irreversibel verbinden.

Der Ventilsitz 63 kann an seiner Innenseite ein Innengewinde 80 aufweisen. An der der Dichtfläche 67 zugewandten vorderen Hälfte des Ventilkörpers 66 kann der Ventilkörper 66 an seiner Außenseite ein zum Innengewinde 80 des Ventilsitzes 63 passendes Außengewinde 82 aufweisen. Der Ventilkörper 66 kann mit seinem Außengewinde 82 in das Innengewinde 80 des Ventilsitzes 63 eingeschraubt werden.

Durch Einschrauben des Ventilkörpers 66 in den Ventilsitz 63 bis zum Anschlag können die ersten Durchführungen 65 und die zweiten Durchführungen 68 in Überdeckung zueinander gebracht werden. Das Ventil befindet sich dann im geöffneten Zustand. Ein Knochenzementteig 50 kann in diesem geöffneten Zustand durch die ersten Durchführungen 65 und durch die zweiten Durchführungen 68 aus der Knochenzementkartusche 10 in die Gießform 61 fließen. Durch eine Vierteldrehung (um 90°) des Ventilkörpers 66 gegen den Ventilsitz 63, das heißt durch ein Herausschrauben des Ventilkörpers 66 aus dem Ventilsitz 63, können die ersten Durchführungen 65 und die zweiten Durchführungen 68 gegeneinander versetzt werden, so dass die Dichtfläche 67 des Ventilkörpers 66 die ersten Durchführungen 65 des Ventilsitzes 63 abdeckt und die geschlossenen Bereiche der Kopfseite 64 des Ventilsitzes 63 die zweiten Durchführungen 68 des Ventilkörpers 66 abdeckt. Das Ventil befindet sich dann im geschlossenen Zustand. Durch den geringen Hub des Ventilkörpers 66 gegen den Ventilsitz 63 bei einer Vierteldrehung, ist der zwischen dem Ventilkörper 66 und dem Ventilsitz 63 entstehende Spalt so schmal (weniger als 1 mm breit), dass ein normaler und erst recht ein hochviskoser Knochenzementteig 50 nicht durch den Spalt dringen können. Dies gilt insbesondere, weil der Knochenzementteig 50 im Spalt von seiner eigentlichen Flussrichtung um 90° abgelenkt wird.

In der Rückseite des Ventilkörpers 66 kann im Anschluss 71 ein Innengewinde 84 angeordnet sein. Das Adapterelement 69 weist an seiner Vorderseite ein zum Innengewinde 84 passendes Außengewinde 86 auf. Das Adapterelement 69 kann so in den Anschluss 71 des Ventilkörpers 66 eingeschraubt werden. Hierdurch kann eine flüssigkeitsdichte Verbindung der Knochenzementkartusche 10 zum Ventilkörper 66 und damit in die Gießform 61 erzeugt werden. Das Innengewinde 80 des Ventilsitzes 63, das Außengewinde 82 des Ventilkörpers 66, das Innengewinde 84 des Ventilkörpers 66 und das Außengewinde 86 des Adapterelements 69 können alle den gleichen Drehsinn aufweisen, das heißt, dass alle diese Gewinde Rechtsgewinde oder Linksgewinde sind. Dadurch kann das Ventil durch Einschrauben des Adapterelements 69 in den Anschluss 71 und gleichsinniges Weiterdrehen des Adapterelements 69 geöffnet werden. Gleichzeitig dichtet auch der Ventilkörper 66 gegen den Ventilsitz 63 ab.

Das Adapterelement 69 kann über ein Rastmittel 88 am Adapterelement 69 mit einer Gegenrastung 30 an einer zylindrischen Wandung der Knochenzementkartusche 10 verbunden werden oder sein. Zur Abdichtung kann eine umlaufende Dichtung 48 vorgesehen sein, die die zylindrische Wandung der Knochenzementkartusche 10 gegen das Adapterelement 69 abdichtet.

Die Gießform 61 kann die Hohlform 92 zum Ausformen des Gelenkkopfs (Kopfs) des Spacers und die Schaftform 94 zum Ausformen des Schafts eines Spacers aufweisen. Das Adapterelement 102 kann als Teil der Schaftform 94 angesehen werden. Die Schaftform ist dann dreiteilig. Des Weiteren kann in der Gießform 91 im Bereich der Einfüllöffnung 92 eine Aussparung für einen Hebel des Ventilkörpers 66 angeordnet sein. Der Hebel kann mit dem Ventilkörper 66 verbunden sein. Der Ventilkörper 66 kann mit dem Hebel im Ventilsitz 63 gedreht werden. Die Aussparung ist vorzugsweise genau so groß, dass den Ventilkörper 66 nur um maximal eine Vierteldrehung gegen den Ventilsitz 63 gedreht werden kann. Dadurch kann das Ventil mit Hilfe des Hebels manuell von außen vom geöffneten Zustand in den geschlossenen Zustand beziehungsweise vom geschlossenen Zustand in den geöffneten Zustand überführt werden.

Im Bereich der Flansche 74 können in der Schaftform 94 Formen 100 für Kavitäten angeordnet sein, in denen die Haltestifte 78 angeordnet werden können.

In dem Adapterelement 69 kann ein Vakuumanschluss 104 angeordnet sein, mit dem ein Innenraum der Knochenzementkartusche 10, in dem der Knochenzementteig 50 gemischt wird, evakuiert werden kann. Dadurch kann der Knochenzementteig 50 unter Vakuum gemischt werden.

Im zylindrischen Innenraum der Knochenzementkartusche 10 kann ein Kolben 46 zum Austreiben des Knochenzementteigs 50 aus der Knochenzementkartusche 10 durch das Ventil in die Gießform 61 angeordnet sein. Hierzu kann der Kolben 46 an seiner Außenseite zylindrisch geformt und über zwei umlaufende Dichtungen 47 gegen den zylindrischen Innenraum abgedichtet sein. Durch Vortreiben des Kolbens 46 kann der Knochenzementteig 50 aus der Austragsöffnung 12 der Knochenzementkartusche 10 in beziehungsweise durch das geöffnete Ventil gepresst werden.

In dem Adapterelement 69 kann eine Porenscheibe 52 angeordnet sein. Die Porenscheibe 52 ist für den Knochenzementteig 50 und seine Ausgangskomponenten undurchlässig. Der Vakuumanschluss 104 kann von der Porenscheibe 52 abgedeckt sein. Hierdurch wird verhindert, dass ein Knochenzementpulver als Ausgangskomponente des Knochenzementteigs 50 in den Vakuumanschluss 104 eindringen kann.

Der Ablauf eines erfindungsgemäßen Verfahrens ist in den Figuren 12 bis 21 anhand der zweiten erfindungsgemäßen Vorrichtung dargestellt. Zunächst kann der Metallkern 76 mit den Haltestiften 78 in der Schaftform 94 positioniert werden. Hierzu können die Haltestifte 78 an einem Ende zwischen den beiden Teilen der Schaftform 94 in den durch die Formen 100 gebildeten Kavitäten angeordnet und gehalten und mit dem anderen Ende in passende Bohrungen im Metallkern 76 angeordnet werden. Zur Befestigung der beiden Teile der Schaftform 94 aneinander können die Flansche 74 der beiden Teile der Schaftform 94 zunächst über die Stifte 79 in den Ausnehmungen 99 positioniert und anschließend die beiden über den Flansch 74 aneinander zu fügenden Teile der Schaftform 74 miteinander mit Hilfe der Schrauben 91 aneinandergeschraubt werden. Anschließend kann das Adapterelement 102 auf den Stutzen 105 geschraubt werden. Das Adapterelement 102 kann dabei so weit auf den Stutzen 105 geschraubt werden, dass sich die gewünschte Länge des Halses ergibt.

Hierzu können an der Außenseite des Adapterelements 102 Markierungen (nicht gezeigt) angeordnet sein, mit denen sich die Länge des Halses des mit der Vorrichtung herzustellenden Spacers ablesen lässt. Die Gießform 61 kann dann geschlossen werden, indem die Hohlform 92 an dem Adapterelement 102 befestigt wird. Hierzu kann die Ringscheibe 83 an den Flansch 95 angelegt werden. Die beiden halben Ringe der Halterung 81 können anschließend an die Ringscheibe 83 angelegt und über die Stifte 89 und die Ausnehmungen 99 positioniert werden. Danach kann durch anziehen der Schrauben 85 die Hohlform 92 an dem Adapterelement 102 befestigt werden, wobei die Ringscheibe 83 die Verbindung abdichtet. Die Gießform 61 ist dann mit dem Metallkern 76 darin verschlossen und kann bereitgestellt werden, um einen Spacer zu formen. Diese Situation ist in Figur 15 gezeigt.

Ein Knochenzementteig 50 kann in der Knochenzementkartusche 10 unter Vakuum gemischt werden. Anschließend kann die Knochenzementkartusche 10 mit dem Adapterelement 69 in den Anschluss 71 des Ventilkörpers 66 geschraubt werden. Beim Einschrauben der Adapterelements 69 kann das Ventil in die geöffnete Stellung überführt werden, indem der Ventilkörper 66 bis zum Anschlag in den Ventilsitz 63 geschraubt wird.

Anschließend wird der Knochenzementteig 50 in die Gießform 61 gepresst. Dies kann folgendermaßen geschehen: durch Vortreiben des Kolbens 46 kann der Knochenzementteig 50 aus der Knochenzementkartusche 10 durch das Ventil und durch die sich in Deckung befindlichen ersten Durchführungen 65 und zweiten Durchführungen 68 in die Gießform 61 gepresst werden. Diese Situation ist in Figur 16 gezeigt. Durch Schließen des Ventils durch Drehen des Ventilkörpers 66 um eine Vierteldrehung gegen den Ventilsitz 63 kann zwischendurch eine neue Knochenzementkartusche 10 angesetzt werden, falls das Volumen des Knochenzementteigs 50 einer einzigen Knochenzementkartusche 10 nicht ausreicht, um die Gießform 61 vollständig zu füllen. Dabei kann der in der Gießform 61 enthaltene Knochenzementteig 50 nicht wieder ausfließen, das die ersten Durchgänge 65 und die zweiten Durchgänge 68 in der geschlossenen Stellung des Ventils abgedeckt sind und der Spalt dazwischen nicht ausreicht, um den viskosen Knochenzementteig 50 hindurchfließen lassen zu können. Gleichzeitig wird ein Druck des Knochenzementteigs 50 im Inneren der Hohlform 92 durch das geschlossene Ventil aufrechterhalten.

Irgendwann ist die Gießform 61 mit dem Knochenzementteig 50 gefüllt. Luft oder Gas aus der Gießform 1 ist durch Entlüftungsöffnungen in der Gießform 61 entwichen. Diese Situation ist in Figur 16 gezeigt. Luft oder Gas aus der Gießform 61 entweicht durch das Entlüftungselement 75 beziehungsweise durch die Entlüftungsöffnungen 79 in der Hohlform 92. Durch weiteres Einpressen von Knochenzementteig 50 kann die Hohlform 92 bis zur gewünschten Größe expandiert werden (siehe Figuren 18 und 19 im Vergleich zur Figur 17). Die radiale Ausdehnung kann mit Hilfe einer Prüflehre oder einer Schieblehre (nicht gezeigt) zum Messen des aktuellen Durchmessers der Hohlform 92 bestimmt werden. Bei bekannter Wandstärke der Hohlform 92 ist dadurch auch der aktuelle Durchmesser der kugelflächenförmigen Innenfläche der Hohlform 92 bestimmbar. Alternativ oder zusätzlich kann auch eine formstabile Gegenform (nicht gezeigt) mit bekanntem und definiertem Durchmesser vorgesehen sein, in die die Hohlform 92 eingelegt werden kann, so dass sich die Hohlform 92 nur so weit ausdehnen kann, bis sie an der Gegenform anliegt. Hierdurch kann auf einfache Weise der gewünschte Durchmesser des Kopfs des mit der Vorrichtung hergestellten Spacers einstellen. Wenn die gewünschte radiale Ausdehnung kugelflächenförmigen Innenfläche der Hohlform 92 erreicht ist, kann das Ventil geschlossen werden.

Durch Schließen des Ventils kann der Knochenzementteig 50 abgeschert beziehungsweise abgeschnitten werden. Die Knochenzementkartusche 10 kann abgeschraubt und entfernt werden. Eventuell verbliebene dünne Verbindungen können ohne weiteres abreißen oder abbrechen. Diese Situation ist in den Figuren 17 und Figur 19 dargestellt.

In diesem Zustand härtet der Knochenzementteig 50 in der Gießform 61 aus. Diese Situation ist in Figur 19 gezeigt. Die Größe beziehungsweise der Durchmesser des Kopfs des Spacers entspricht dabei dem Durchmesser der kugelflächenförmigen Innenfläche der expandierten Hohlform 92. Um Unebenheiten der Gleitfläche des Spacers zu vermeiden, ist es erfindungsgemäß bevorzugt, dass auch bei der Herstellung von Spacern mit Köpfen mit kleinstem Durchmesser die Hohlform 92 bereits geringfügig expandiert ist und dadurch der Knochenzementteig 50 in die Gießform 61 unter Druck steht, während er aushärtet.

Anschließend wird der so geformte Spacer 120, 130 (siehe Figuren 20 und 21) aus der Gießform 1 entnommen. Die vorspringenden Haltestifte 78 können abgeschnitten werden. Ebenso kann ein durch den Ventilsitz 63 und die ersten Durchgänge 65 verursachter Anguss abgeschnitten und entfernt werden. Auch können Spitzen, die durch die Entlüftungsöffnungen verursacht werden, entfernt werden. Diese Situation ist in den Figuren 20 und 21 gezeigt.

Ein entnommene Spacer 120 ist in Figur 20 gezeigt. Der Spacer 120 weist einen Kopf 122 auf, der von der Hohlform 92 geformt wurde. Der Spacer 120 wurde durch Aushärten des Knochenzementteigs 50 in der Gießform 61 geformt, wie sie in Figur 17 gezeigt ist. Der Kopf 122 ist über einen Hals 126 mit einem Schaft 124 des Spacers 120 verbunden.

Ein alternativer Spacer 130 mit längerem Hals 136, der durch eine Verlängerung mit dem Adapterelement 102 geformt wurde (siehe Figuren 18 und 19), ist in Figur 21 gezeigt. Der Spacer 130 weist einen Kopf 132 auf, der von der Hohlform 92 geformt wurde. Der Spacer 130 wurde durch Aushärten des Knochenzementteigs 50 in der Gießform 61 geformt, wie sie in Figur 18 gezeigt ist. Der Kopf 132 ist über den Hals 136 mit einem Schaft 134 des Spacers 130 verbunden. Der Hals 134 weist in seinem proximalen Bereich die Form eines Gewindes auf, da er eine Negativform des Innengewindes 103 des Adapterelements 102 ist. Bei Bedarf kann die Oberfläche in diesem Bereich geglättet werden.

Die Oberfläche des Spacers 120, 130 kann poliert werden und/oder beschichtet werden, beispielsweise mit Antibiotika.

Anstatt einer Gießform 61 zum Formen eines Schultergelenkspacers kann ohne weiteres auch eine Gießform zum Formen eines anderen Spacers verwendet werden.

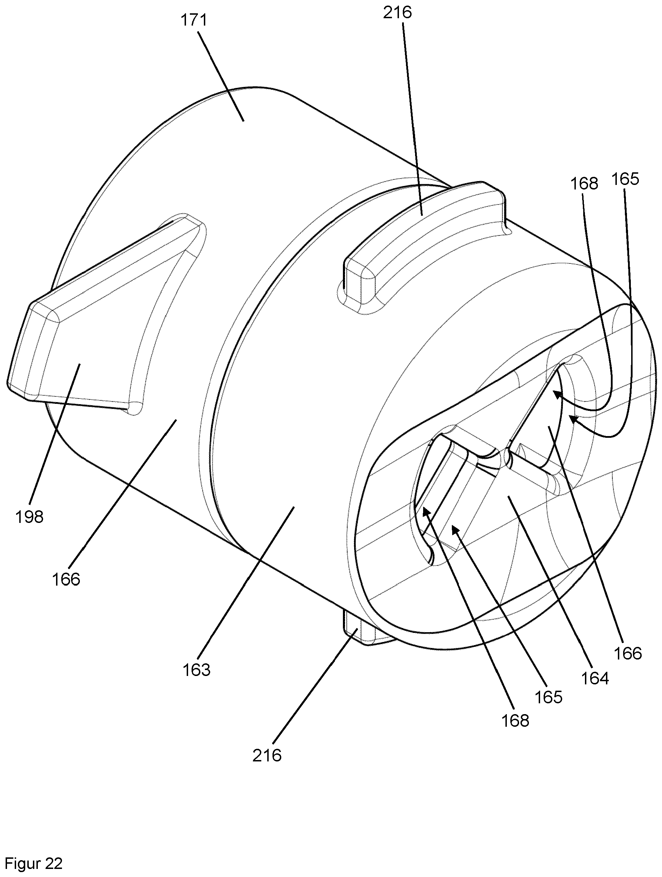

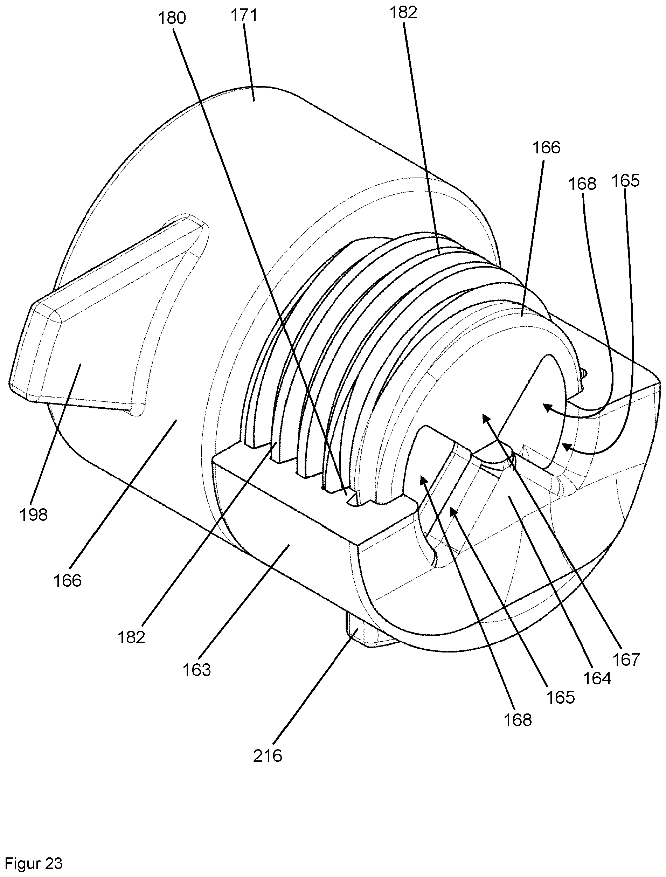

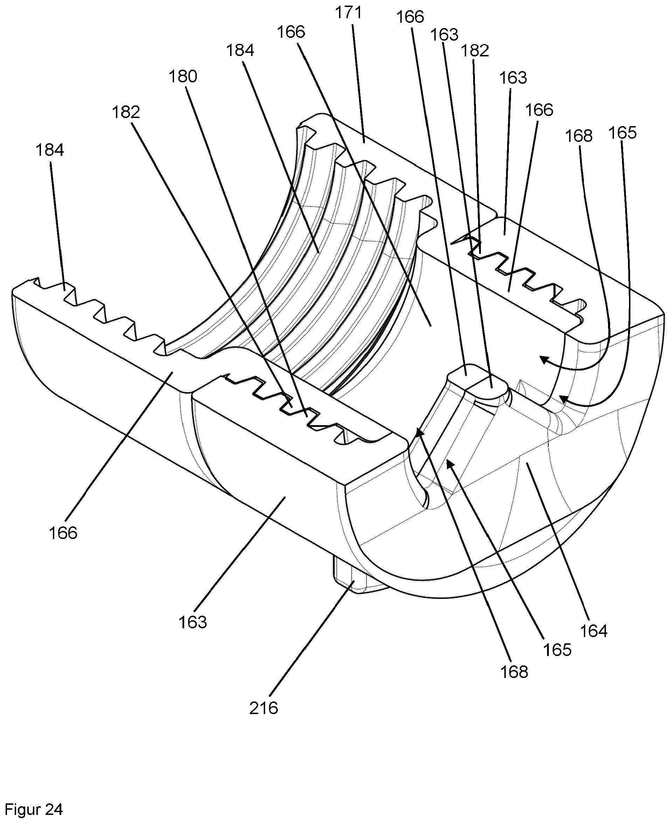

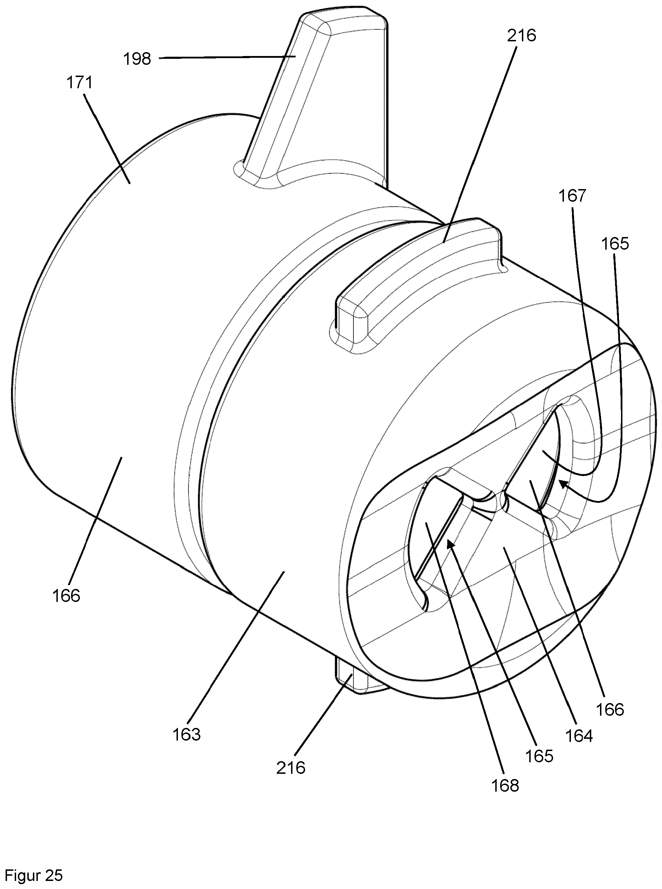

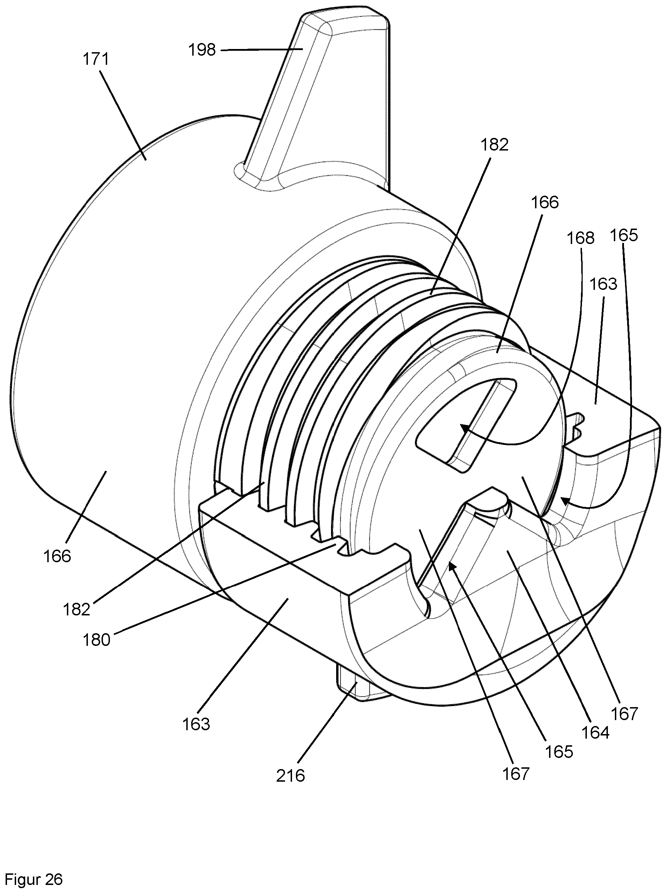

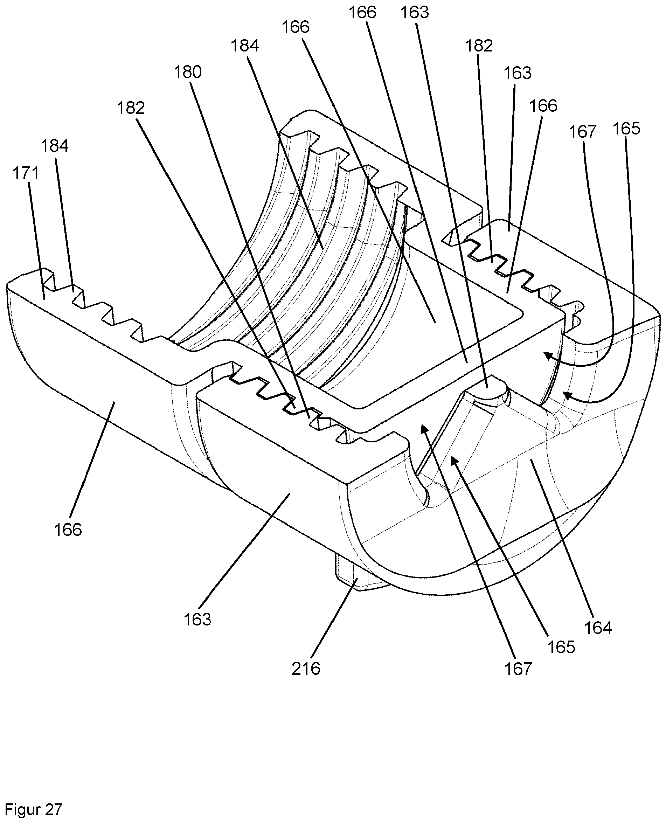

Die Figuren 22 bis 28 zeigen ein Ventil für eine erfindungsgemäße Vorrichtung zur Herstellung eines Spacers in geöffneter Stellung (Figuren 22 bis 24) und in geschlossener Stellung (Figuren 25 bis 28). Das Ventil entspricht den Ventilen der ersten erfindungsgemäßen Vorrichtung nach den Figuren 1 bis 11 und der zweiten erfindungsgemäßen Vorrichtung nach den Figuren 12 bis 19, ist aber auch mit anderen Gießformen zur Herstellung anderer Spacer einsetzbar.

Das Ventil hat einen Ventilsitz 163, der in einer Einfüllöffnung einer Gießform (nicht gezeigt) angeordnet werden kann. Der Ventilsitz 163 kann mit einem Teil der Gießform fest verbunden sein oder auch einteilig mit der Gießform ausgeführt sein. Zur besseren und dichteren Verbindbarkeit des Ventilsitzes 163 mit einer Gießform kann der Ventilsitz 163 an seiner äußeren Oberfläche eine Strukturierung aufweisen, beispielsweise Längsrillen, die parallel zur Zylinderachse einer zylindrischen Außenwand des Ventilsitzes 163 angeordnet sind.

Der Ventilsitz 163 kann als ein Hohlzylinder ausgeführt sein, der auf einer Kopfseite 164 bis auf zwei erste Durchführungen 165 geschlossen ist. Die beiden ersten Durchführungen 165 können viertelkreisförmig geformt sein und können vorzugsweise um 180° bezüglich der Zylinderachse des Ventilsitzes 163 gegeneinander verdreht angeordnet sein. Im Inneren des Ventilsitzes 163 kann ein gegen den Ventilsitz 163 axial verdrehbarer Ventilkörper 166 angeordnet sein. Der Ventilkörper 166 kann eine in Richtung der Kopfseite 164 des Ventilsitzes 163 ausgerichtete Dichtfläche 167 beziehungsweise Oberfläche aufweisen. Der Ventilkörper 166 kann als abgestufter Hohlzylinder aufgebaut sein, dessen vorderer Teil in den Ventilsitz 163 eingeschraubt oder eingesteckt sein kann.

In der Dichtfläche 167 können zwei zweite Durchführungen 168 angeordnet sein. Die beiden zweiten Durchführungen 168 können analog den ersten Durchführungen 165 viertelkreisförmig geformt sein und können vorzugsweise um 180° bezüglich der Zylinderachse des Ventilkörpers 166 gegeneinander verdreht beziehungsweise versetzt angeordnet sein. Der Ventilsitz 163 und der Ventilkörper 166 bilden zusammen das Ventil einer erfindungsgemäßen Vorrichtung. In den Ventilkörper 166 kann ein Adapterelement (nicht gezeigt) zum flüssigkeitsdichten Verbinden einer Knochenzementkartusche (nicht gezeigt) eingeschraubt werden. Der Ventilkörper 166 kann an seiner offenen Seite, die von der Dichtfläche 167 abgewandt ist, als Anschluss 171 zum Verbinden eines Adapterelements ausgeformt sein.

Der Ventilsitz 163 kann an seiner Innenseite ein Innengewinde 180 aufweisen. An der der Dichtfläche 167 zugewandten vorderen Hälfte des Ventilkörpers 166 kann der Ventilkörper 166 an seiner Außenseite ein zum Innengewinde 180 des Ventilsitzes 163 passendes Außengewinde 182 aufweisen. Der Ventilkörper 166 kann mit seinem Außengewinde 182 in das Innengewinde 180 des Ventilsitzes 163 eingeschraubt werden.

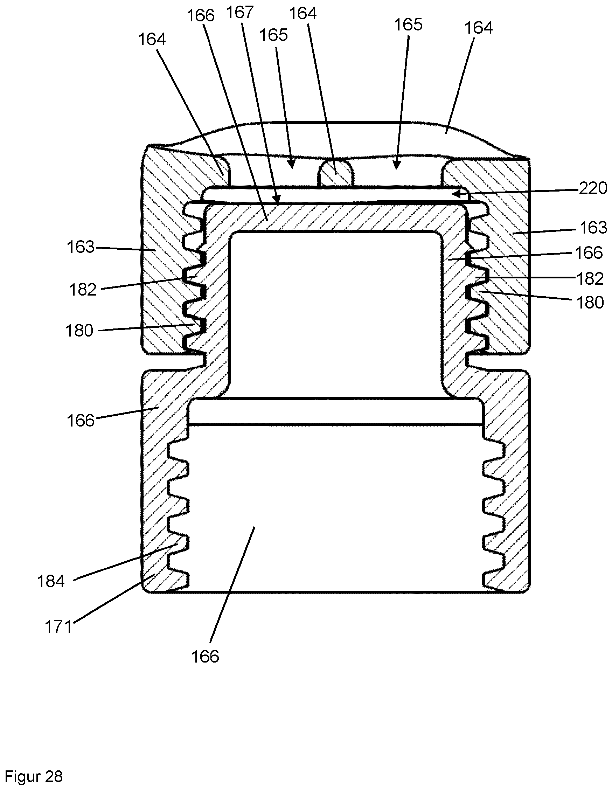

Durch Einschrauben des Ventilkörpers 166 in den Ventilsitz 163 bis zum Anschlag können die ersten Durchführungen 165 und die zweiten Durchführungen 168 in Überdeckung zueinander gebracht werden. Das Ventil befindet sich dann im geöffneten Zustand. Ein Knochenzementteig kann in diesem geöffneten Zustand (siehe Figuren 22 bis 24) durch die ersten Durchführungen 165 und durch die zweiten Durchführungen 168 fließen. Durch eine Vierteldrehung (um 90°) des Ventilkörpers 166 gegen den Ventilsitz 163, das heißt durch ein Herausschrauben des Ventilkörpers 166 aus dem Ventilsitz 163, können die ersten Durchführungen 165 und die zweiten Durchführungen 168 gegeneinander versetzt werden, so dass die Dichtfläche 167 des Ventilkörpers 166 die ersten Durchführungen 165 des Ventilsitzes 163 abdeckt und die geschlossenen Bereiche der Kopfseite 164 des Ventilsitzes 163 die zweiten Durchführungen 168 des Ventilkörpers 166 abdeckt. Das Ventil befindet sich dann im geschlossenen Zustand (siehe Figuren 25 bis 28). Durch den geringen Hub des Ventilkörpers 166 gegen den Ventilsitz 163 bei einer Vierteldrehung, ist der zwischen dem Ventilkörper 166 und dem Ventilsitz 163 entstehende Spalt 220 so schmal (weniger als 1 mm breit), dass ein normaler und erst recht ein hochviskoser Knochenzementteig nicht durch den Spalt 220 dringen können (siehe Figur 28). Dies gilt insbesondere, weil der Knochenzementteig im Spalt 220 von seiner eigentlichen Flussrichtung um 90° abgelenkt wird. Ein Vorsprung 216 kann vorgesehen sein, um eine stabilere und drehsichere Verbindung des Ventilsitzes 163 zu der Gießform zu gewährleisten.

In der Rückseite des Ventilkörpers 166 kann im Anschluss 171 ein Innengewinde 184 angeordnet sein. Ein Adapterelement (nicht gezeigt) kann so in den Anschluss 171 des Ventilkörpers 166 eingeschraubt werden. Das Innengewinde 180 des Ventilsitzes 163, das Außengewinde 182 des Ventilkörpers 166 und das Innengewinde 184 des Ventilkörpers 166 können alle den gleichen Drehsinn aufweisen, das heißt, dass alle diese Gewinde Rechtsgewinde oder Linksgewinde sind. Dadurch kann das Ventil durch Einschrauben eines Adapterelements in den Anschluss 171 und gleichsinniges Weiterdrehen des Adapterelements geöffnet werden. Gleichzeitig dichtet auch der Ventilkörper 166 gegen den Ventilsitz 163 ab.

Des Weiteren kann ein Hebel 198 am Ventilkörper 166 angeordnet sein. Der Ventilkörper 166 kann mit dem Hebel 198 im Ventilsitz 163 gedreht werden. Dadurch kann das Ventil mit Hilfe des Hebels 198 manuell von außen vom geöffneten Zustand in den geschlossenen Zustand beziehungsweise vom geschlossenen Zustand in den geöffneten Zustand überführt werden.

### Bezugszeichenliste

- 1, 61: Gießform
- 2, 62: Einfüllöffnung
- 3, 63, 163: Ventilsitz
- 4, 64, 164: Kopfseite
- 5, 65, 165: Durchführung
- 6, 66, 166: Ventilkörper
- 7, 67, 167: Dichtfläche
- 8, 68, 168: Durchführung
- 9, 69: Adapterelement
- 10: Knochenzementkartusche
- 11,71,171: Anschluss
- 12: Austragsöffnung
- 13, 73: Dichtung
- 14, 74: Flansch
- 15, 75: Entlüftungselement
- 16, 76: Metallkern
- 17, 77: Porenfilter
- 18, 78: Haltestift
- 19, 79: Entlüftungsöffnung
- 20, 80, 180: Innengewinde
- 21, 81: Halterung
- 22, 82, 182: Außengewinde
- 23, 83: Ringscheibe
- 24, 84, 184: Innengewinde
- 25, 85: Schraube
- 26, 86: Außengewinde
- 27, 87: Stutzen
- 28, 88: Rastmittel
- 29, 89: Stift
- 30: Gegenrastung
- 31, 91: Schraube
- 32, 92: Hohlform
- 33, 93: Stutzen
- 34, 94: Schaftform
- 35, 95: Flansch
- 36: Mischer
- 37: Austragsrohr
- 38: Hebel
- 39, 99: Ausnehmung
- 40, 100: Form für Kavitäten
- 44, 104: Vakuumanschluss
- 46: Kolben
- 47: Dichtung
- 48: Dichtung
- 50: Knochenzementteig
- 52: Porenscheibe
- 102: Adapterelement
- 103: Innengewinde
- 105: Hohlzylinder
- 107: Außengewinde
- 120, 130: Spacer
- 122, 132: Kopf
- 124, 134: Schaft
- 126, 136: Hals
- 216: Vorsprung
- 220: Spalt

## Patentansprüche

1. Vorrichtung zum Herstellen eines Spacers (120, 130) durch Aushärten von Knochenzementteig (50), wobei der Spacer (120, 130) dazu vorgesehen ist, im medizinischen Bereich ein Gelenk oder einen Teil eines Gelenks umfassend eine artikulierende Oberfläche eines Kopfs des Gelenks temporär zu ersetzen, insbesondere eines Hüftgelenks oder eines Schultergelenks temporär zu ersetzen, die Vorrichtung aufweisend
eine Gießform (1, 61) zum Formen des Spacers (120, 130) aus Knochenzementteig (50), wobei die Gießform (1, 61) eine Schaftform (34, 94) zum Formen eines Schafts (124, 134) und eines Halses (126, 136) aufweist und die Gießform (1, 61) eine Hohlform (32, 92) mit einer kugelflächenförmigen Innenfläche zum Formen einer Gleitfläche eines Kopfs (122, 132) des Spacers (120, 130) aufweist, wobei die Schaftform (34, 94) und die Hohlform (32, 92) einen gemeinsamen Innenraum begrenzen, so dass der Kopf (122, 132) eines mit der Gießform (1, 61) geformten Spacers (120, 130) über den Hals (126, 136) mit dem Schaft (124, 134) einteilig verbunden ist,
wenigstens eine Einfüllöffnung (2, 62) zum Einpressen eines Knochenzementteigs (50) in die Gießform (1, 61),
wenigstens ein Entlüftungselement (15, 75), das den gemeinsamen Innenraum der Gießform (1, 61) gasdurchlässig mit der Umgebung der Gießform (1, 61) verbindet, wobei die Schaftform (34, 94) bei einem Einpressen von Knochenzementteig (50) in die Gießform (1, 61) formstabil ist, **dadurch gekennzeichnet, dass** die Hohlform (32, 92) bei dem Einpressen von Knochenzementteig (50) in die Gießform (1, 61) zumindest im Bereich der kugelflächenförmigen Innenfläche durch einen vom eingepressten Knochenzementteig (50) vermittelten Druck expandierbar ist.

2. Vorrichtung nach Anspruch 1 **dadurch gekennzeichnet, dass**
das wenigstens eine Entlüftungselement (15, 75) für Gase durchlässig ist und für Knochenzementteig (50) undurchlässig ist, insbesondere für Polymethylmethacrylat-Knochenzementteig (50) (PMMA-Knochenzementteig) undurchlässig ist, und/oder das wenigstens eine Entlüftungselement (15, 75) in der Hohlform (32, 92) angeordnet ist oder das wenigstens eine Entlüftungselement (15, 75) mehrere Entlüftungselemente sind, wobei zumindest eines der mehreren Entlüftungselemente in der Hohlform (32, 92) angeordnet ist und zumindest eines der mehreren Entlüftungselemente in der Schaftform (34, 94) angeordnet ist.

3. Vorrichtung nach Anspruch 1 oder 2, **dadurch gekennzeichnet, dass**
die Hohlform (32, 92) zumindest im Bereich der kugelflächenförmigen Innenfläche durch den vom eingepressten Knochenzementteig (50) vermittelten Druck radial expandierbar ist, bevorzugt radial und gleichförmig expandierbar ist, und/oder
die Hohlform (32, 92) zumindest im Bereich der kugelflächenförmigen Innenfläche durch den vom eingepressten Knochenzementteig (50) vermittelten Druck elastisch expandierbar ist, bevorzugt gummielastisch expandierbar ist.

4. Vorrichtung nach einem der vorangehenden Ansprüche, **dadurch gekennzeichnet, dass**
die Schaftform (34, 94) und die Hohlform (32, 92) über einen Flansch (14, 74) oder ein Adapterelement (102) miteinander flüssigkeitsdicht verbunden oder verbindbar sind, wobei bevorzugt ein expandierbarer Teil der Hohlform (32, 92), der die kugelflächenförmige Innenfläche umfasst, mit einer ringförmigen Halterung (21, 81) auf einem Flansch (35, 95) der Schaftform (34, 94) oder des Adapterelements (102) befestigt oder befestigbar ist, so dass eine randseitige Ringscheibe (23, 83) des expandierbaren Teils der Hohlform (32, 92) zwischen der ringförmigen Halterung (21, 81) und dem Flansch (35, 95) angeordnet ist und die Verbindung abdichtet, wobei besonders bevorzugt die ringförmige Halterung (21, 81) mit dem Flansch (35, 95) der Schaftform (34, 94) verschraubt oder verschraubbar ist, und/oder
die Vorrichtung ein Befestigungselement zur Befestigung der Hohlform (32, 92) an der Schaftform (34, 94) aufweist, wobei bevorzugt das Befestigungselement lösbar ist und besonders bevorzugt das Befestigungselement mehrere Schrauben (25, 85) sind oder mehrere Schrauben (25, 85) aufweist, wobei das Befestigungselement Klemmplatten aufweist, wobei bevorzugt das Befestigungselement zusätzlich Schrauben (25, 85), Flügelschrauben, Muttern und/oder Gewindestangen aufweist.

5. Vorrichtung nach einem der vorangehenden Ansprüche, **dadurch gekennzeichnet, dass**
die wenigstens eine Einfüllöffnung (2, 62) auf einer von der Gießform (1, 61) abgewandten Seite mit einem Anschluss (11, 71, 171) zum flüssigkeitsdichten Verbinden einer Knochenzementkartusche (10) verbunden ist, wobei bevorzugt der Anschluss (11, 71, 171) zum druckdichten Verbinden einer Knochenzementkartusche (10) geeignet ist, wobei besonders bevorzugt der Anschluss (11, 71, 171) ein Gewinde aufweist und ganz besonders bevorzugt ein umlaufende Dichtung (13, 73) und/oder eine umlaufende Dichtfläche oder eine umlaufende Dichtkante aufweist, und/oder sich die Hohlform (32, 92) zumindest im Bereich der kugelflächenförmigen Innenfläche durch Einpressen von weiterem Knochenzementteig (50) in die bereits vollständig mit Knochenzementteig (50) gefüllte Gießform (1, 61) ausdehnt, während die Schaftform (34, 94) keinen zusätzlichen Knochenzementteig (50) aufnimmt und formstabil bleibt.

6. Vorrichtung nach einem der vorangehenden Ansprüche, **gekennzeichnet durch** einen Ventilsitz (3, 63, 163), der im Bereich der wenigstens einen Einfüllöffnung (2, 62) mit der Gießform (1, 61) verbunden ist, wobei der Ventilsitz (3, 63, 163) eine bereichsweise geschlossene Kopfseite (4, 64, 164) mit mindestens einer ersten Durchführung (5, 65, 165) aufweist, wobei die mindestens eine erste Durchführung (5, 65, 165) in die wenigstens eine Einfüllöffnung (2, 62) mündet,
einen Ventilkörper (6, 66, 166), der drehbar gegen den Ventilsitz (3, 63, 163) gelagert ist und der eine Dichtfläche (7, 67, 167) aufweist, wobei die Dichtfläche (7, 67, 167) in Richtung der bereichsweise geschlossenen Kopfseite (4, 64, 164) des Ventilsitzes (3, 63, 163) ausgerichtet ist, wobei in der Dichtfläche (7, 67, 167) mindestens eine zweite Durchführung (8, 68, 168) angeordnet ist;
wobei der Ventilsitz (3, 63, 163) und der Ventilkörper (6, 66, 166) zusammen ein Ventil bilden, wobei das Ventil durch Drehen des Ventilkörpers (6, 66, 166) gegen den Ventilsitz (3, 63, 163) reversibel in eine geöffnete Stellung und reversibel in eine geschlossene Stellung überführbar ist, wobei in der geöffneten Stellung des Ventils die mindestens eine erste Durchführung (5, 65, 165) des Ventilsitzes (3, 63, 163) und die mindestens eine zweite Durchführung (8, 68, 168) des Ventilkörpers (6, 66, 166) zumindest bereichsweise übereinanderliegen und eine für Knochenzementteig (50) durchlässige Verbindung durch das Ventil in die Gießform (1, 61) bereitstellen, wobei in der geschlossenen Stellung des Ventils die mindestens eine erste Durchführung (5, 65, 165) des Ventilsitzes (3, 63, 163) durch die Dichtfläche (7, 67, 167) des Ventilkörpers (6, 66, 166) abgedeckt ist, wobei die wenigstens eine Einfüllöffnung (2, 62) der Gießform (1, 61) in der geschlossenen Stellung des Ventils für Knochenzementteig (50) abgedeckt ist.

7. Vorrichtung nach Anspruch 6, **dadurch gekennzeichnet, dass**
das Ventil auf der von der Gießform (1, 61) abgewandten Seite mit einem Anschluss (11, 71, 171) zum flüssigkeitsdichten Verbinden einer Knochenzementkartusche (10) verbunden ist oder das Ventil einen solchen Anschluss (11, 71, 171) aufweist und/oder der Ventilsitz (3, 63, 163) nicht verdrehbar gegen die Gießform (1, 61) mit der Gießform (1, 61) verbunden ist, bevorzugt der Ventilsitz (3, 63, 163) fest und/oder starr mit der Gießform (1, 61) verbunden ist, und/oder
das Ventil manuell bedienbar ist, bevorzugt manuell von außerhalb der Vorrichtung manuell bedienbar ist, wobei besonders bevorzugt der Ventilkörper (6, 66, 166) manuell gegen den Ventilsitz (3, 63, 163) drehbar ist und durch die Drehung das Ventil von der geschlossenen Stellung in die geöffnete Stellung und von der geöffneten Stellung in die geschlossene Stellung überführbar ist.

8. Vorrichtung nach einem der Ansprüche 6 oder 7, **dadurch gekennzeichnet, dass**
der Ventilsitz (3, 63, 163) ein Innengewinde (20, 80, 180) an der Innenseite aufweist und der Ventilkörper (6, 66, 166) ein passendes Außengewinde (22, 82, 182) an der Außenseite aufweist, so dass der Ventilkörper (6, 66, 166) in den Ventilsitz (3, 63, 163) schraubbar ist und/oder
der Anschluss (11, 71, 171) zum flüssigkeitsdichten Verbinden einer Knochenzementkartusche (10) ein Innengewinde (24, 84, 184) im Ventilkörper (6, 66, 166) oder ein Außengewinde am Ventilkörper (6, 66, 166) umfasst, wobei vorzugsweise ein Adapterelement (9, 69) der Knochenzementkartusche (10) oder an der Knochenzementkartusche (10) ein zu dem Innengewinde (24, 84, 184) oder zu dem Außengewinde passendes Gegengewinde aufweist.

9. Vorrichtung nach einem der vorangehenden Ansprüche, **dadurch gekennzeichnet, dass**
die Gießform (1, 61) ausgehend von einem Innenraum der Gießform (1, 61) mindestens drei oder vier Kavitäten zur Aufnahme von Haltestiften aufweist, wobei bevorzugt die Kavitäten in der Schaftform (34, 94) angeordnet sind und besonders bevorzugt die Schaftform (34, 94) zweiteilig oder dreiteilig ist und die Kavitäten in Rändern oder in Längsflanschen (14, 74) zumindest eines Teils der zweiteiligen Schaftform (34, 94) angeordnet sind, und
die Vorrichtung einen Metallkern (16, 76) aufweist, der in der Gießform (1, 61) anzuordnen ist, wobei bevorzugt der Metallkern (16, 76) Bohrungen zur Aufnahme von Haltestiften (18, 78) besitzt, wobei besonders bevorzugt die Bohrungen (18, 78) innerhalb des Teils des Metallkerns (16, 76) angeordnet sind, die in der Schaftform (34, 94) anzuordnen sind, und/oder
die kugelflächenförmige Innenfläche der Hohlform (32, 92) im nicht expandierten Zustand einen Durchmesser von mindestens 35 mm oder von mindestens 40 mm hat, bevorzugt zwischen 40 mm und 50 mm hat, und im maximal expandierten Zustand einen Durchmesser von höchstens 70 mm hat, bevorzugt zwischen 60 mm und 70 mm, wobei der Durchmesser der kugelflächenförmigen Innenfläche der Hohlform (32, 92) im nicht expandierten Zustand kleiner ist als der Durchmesser der kugelflächenförmigen Innenfläche der Hohlform (32, 92) im expandierten Zustand.

10. Vorrichtung nach einem der vorangehenden Ansprüche, **dadurch gekennzeichnet, dass**
die Hohlform (32, 92) zumindest im Bereich der kugelflächenförmigen Innenfläche aus einem gummielastischen Material besteht, vorzugsweise aus Kautschuk, Silikonkautschuk, einem Synthesekautschuk oder einem Ethylen-Propylen-Dien-Kautschuk (EPDM) besteht, und/oder
die wenigstens eine Einfüllöffnung (2, 62) ein Absperrelement enthält, das in einem geschlossenen Zustand ein Ausfließen des Knochenzementteigs (50) aus der Gießform (1, 61) durch die wenigstens eine Einfüllöffnung (2, 62) verhindert.

11. Vorrichtung nach einem der vorangehenden Ansprüche, **dadurch gekennzeichnet, dass**
die Schaftform (94) ein in der Länge variables Adapterelement (102) umfasst, mit dem die Länge des Halses (126, 136) des Spacers (120, 130), der den Schaft (124, 134) mit dem Kopf (122, 132) des Spacers (120, 130) verbindet, variierbar ist, wobei vorzugsweise das Adapterelement (102) durch eine Schraubverbindung in der Länge variierbar ist.

12. Vorrichtung nach einem der vorangehenden Ansprüche, **dadurch gekennzeichnet, dass**
die Vorrichtung mehrere formstabile Gegenformen aufweist, die zur Aufnahme der Hohlform (32, 92) geeignet sind, wobei die formstabilen Gegenformen eine unterschiedlich weite Expansion der Hohlform (32, 92) ermöglichen, so dass die Expansion der Hohlform (32, 92) zumindest in dem Bereich der kugelflächenförmigen Innenfläche durch die formstabilen Gegenformen bis hin zu unterschiedlichen Durchmessern begrenzt ist, wenn die Hohlform (32, 92) in die jeweilige formstabile Gegenform eingesetzt ist, wobei bevorzugt die formstabilen Gegenformen durch zumindest eine Blisterpackung oder eine Kunststoffschale mit einer oder mit mehreren Vertiefungen als die formstabilen Gegenformen realisiert ist, und/oder die Vorrichtung eine Prüflehre oder eine Schieblehre zum Messen des aktuellen Durchmessers der kugelflächenförmigen Innenfläche der Hohlform (32, 92) aufweist, wobei die Prüflehre oder die Schieblehre außen an der Hohlform (32, 92) anlegbar oder angeordnet ist und wobei vorzugsweise der Durchmesser der kugelflächenförmigen Innenfläche der Hohlform (32, 92) unmittelbar ablesbar ist.

13. Verfahren zur Herstellung eines Spacers (120, 130) zum temporären Ersetzen eines Gelenks oder eines Teils eines Gelenks, insbesondere eines Hüftgelenks oder eines Schultergelenks, umfassend eine artikulierende Oberfläche des Gelenks, wobei das Verfahren mit einer Vorrichtung nach einem der Ansprüche 1 bis 12 durchgeführt wird, das Verfahren aufweisend die folgenden chronologischen Schritte:
A) Einpressen von Knochenzementteig (50) durch die wenigstens eine Einfüllöffnung (2, 62) in die Gießform (1, 61) und gleichzeitig Verdrängen von Luft aus der Gießform (1, 61) durch das wenigstens eine Entlüftungselement (15, 75) durch das Einpressen des Knochenzementteigs (50);
B) Weiteres Einpressen von Knochenzementteig (50) durch die wenigstens eine Einfüllöffnung (2, 62) in die Gießform (1, 61), wobei durch das Einpressen des Knochenzementteigs (50) die Hohlform (32, 92) zumindest im Bereich der kugelflächenförmigen Innenfläche expandiert, während die Schaftform (34, 94) formstabil bleibt;
C) Aushärten des Knochenzementteigs (50) in der Gießform (1, 61); und
D) Entnehmen des so geformten und ausgehärteten Spacers (120, 130) aus der Gießform (1, 61).

14. Verfahren nach Anspruch 13, **dadurch gekennzeichnet, dass**
vor Schritt A) ein flüssigkeitsdichtes Verbinden einer Knochenzementkartusche (10) mit einem Anschluss (11, 71, 171) der Vorrichtung erfolgt, wobei der Anschluss (11, 71, 171) mit der wenigstens einen Einfüllöffnung (2, 62) flüssigkeitsdurchlässig verbunden ist und in Schritt A) der Knochenzementteig (50) aus der Knochenzementkartusche (10) in die Gießform (1, 61) gepresst wird,
wobei vorzugsweise eine Vorrichtung gemäß einem der Ansprüche 6 bis 8 mit einem Ventil verwendet wird, wobei das Einpressen von Knochenzementteig (50) in Schritt A) durch das Ventil in der geöffneten Stellung hindurch in die Gießform (1, 61) erfolgt, wobei nach Schritt B) und vor Schritt C) ein Schritt B1) erfolgt:
B1) Drehen des Ventilkörpers (6, 66, 166) gegen den Ventilsitz (3, 63, 163) und dadurch Überführen des Ventils in die geschlossene Stellung und Abscheren des Knochenzementteigs (50) an der mindestens einen ersten Durchführung (5, 65, 165) in der bereichsweise geschlossenen Kopfseite (4, 64, 164) des Ventilsitzes (3, 63, 163) durch die Drehung des Ventilkörpers (6, 66, 166) gegen den Ventilsitz (3, 63, 163), wobei vorzugsweise anschließend eine Knochenzementkartusche (10) von einem Anschluss (11, 71, 171) gelöst wird, der mit der wenigstens einen Einfüllöffnung (2, 62) flüssigkeitsdurchlässig verbunden ist.

15. Verfahren nach Anspruch 14, **dadurch gekennzeichnet, dass** nach Schritt B1) und vor Schritt C) die folgenden Zwischenschritte erfolgen:
B2) Flüssigkeitsdichtes Verbinden einer neuen Knochenzementkartusche (10) mit dem Anschluss (11, 71, 171) der Vorrichtung, wobei in der neuen Knochenzementkartusche (10) Knochenzementteig (50) oder Ausgangskomponenten zur Herstellung des Knochenzementteigs (50) enthalten ist oder sind;
B3) Drehen des Ventilkörpers (6, 66, 166) gegen den Ventilsitz (3, 63, 163) und dadurch Überführen des Ventils in die geöffnete Stellung;
B4) Einpressen des Knochenzementteigs (50) aus der neuen Knochenzementkartusche (10) durch das Ventil in der geöffneten Stellung hindurch in die Gießform (1, 61);
B5) Drehen des Ventilkörpers (6, 66, 166) gegen den Ventilsitz (3, 63, 163) und dadurch Überführen des Ventils in die geschlossene Stellung und Abscheren des Knochenzementteigs (50) an der mindestens einen ersten Durchführung (5, 65, 165) in der bereichsweise geschlossenen Kopfseite (4, 64, 164) des Ventilsitzes (3, 63, 163) durch die Drehung des Ventilkörpers (6, 66, 166) gegen den Ventilsitz (3, 63, 163); und B6) Lösen der neuen Knochenzementkartusche (10) von dem Anschluss (11, 71, 171); wobei vorzugsweise die Schritte B2) bis B6) einmal oder mehrfach mit jeweils neuen Knochenzementkartuschen, die Knochenzementteig (50) oder dessen Ausgangskomponenten enthalten, wiederholt werden, bis die Gießform (1, 61) vollständig mit Knochenzementteig (50) gefüllt ist und darüber hinaus bis die Hohlform (32, 92) zumindest im Bereich der kugelflächenförmigen Innenfläche mit Hilfe des Knochenzementteigs (50) bis zur gewünschten Größe expandiert ist.

16. Verfahren nach einem der Ansprüche 13 bis 15, **dadurch gekennzeichnet, dass**
die Hohlform (32, 92) der Gießform (1, 61) in eine von mehreren formstabile Gegenformen eingesetzt wird, wobei die formstabilen Gegenformen eine unterschiedlich weite Expansion der Hohlform (32, 92) ermöglichen, so dass die Expansion der Hohlform (32, 92) zumindest in dem Bereich der kugelflächenförmigen Innenfläche durch die verwendete formstabile Gegenform bis hin zu einem bestimmten Durchmessern begrenzt wird, während der Knochenzementteig (50) zum Expandieren der Hohlform (32, 92) in die Gießform (1, 61) gepresst wird, und/oder
eine Prüflehre oder eine Schieblehre zum Messen des aktuellen Durchmessers der kugelflächenförmigen Innenfläche der Hohlform (32, 92) dazu verwendet wird, den aktuellen Durchmesser der kugelflächenförmigen Innenfläche der Hohlform (32, 92) abzulesen, wobei vorzugsweise das Einpressen des Knochenzementteigs (50) in die Gießform (1, 61) gestoppt wird, wenn ein gewünschter Durchmesser erreicht wird.

## Claims

1. A device for producing a spacer (120, 130) by curing bone cement paste (50), wherein the spacer (120, 130) is provided in the medical field for temporarily replacing a joint or part of a joint comprising an articulating surface of a head of the joint, in particular for temporarily replacing a hip joint or a shoulder joint, the device having
a casting mold (1, 61) for molding the spacer (120, 130) from bone cement paste (50), wherein the casting mold (1, 61) has a stem mold (34, 94) for molding a stem (124, 134) and a neck (126, 136) and the casting mold (1, 61) has a mold cavity (32, 92) with a spherical surface-shaped inner surface for molding a sliding surface of a head (122, 132) of the spacer (120, 130), wherein the stem mold (34, 94) and the mold cavity (32, 92) delimit a common interior, such that the head (122, 132) of a spacer (120, 130) molded with the casting mold (1, 61) is connected as one part via the neck (126, 136) to the stem (124, 134),
at least one filling opening (2, 62) for injecting a bone cement paste (50) into the casting mold (1, 61),
at least one vent element (15, 75) which connects the common interior of the casting mold (1, 61) in a gas-permeable manner to the surroundings of the casting mold (1, 61),
wherein the stem mold (34, 94) is dimensionally stable on injection of bone cement paste (50) into the casting mold (1, 61), **characterized in that**
on injection of bone cement paste (50) into the casting mold (1, 61), the mold cavity (32, 92) is expandable, at least in the region of the spherical surface-shaped inner surface, by pressure imparted by the injected bone cement paste (50).

2. The device according to Claim 1, **characterized in that**
the at least one vent element (15, 75) is permeable to gases and is impermeable to bone cement paste (50), in particular to polymethyl methacrylate bone cement paste (50) (PMMA bone cement paste), and/or
the at least one vent element (15, 75) is arranged in the mold cavity (32, 92) or the at least one vent element (15, 75) is a plurality of vent elements, wherein at least one of the plurality of vent elements is arranged in the mold cavity (32, 92) and at least one of the plurality of vent elements is arranged in the stem mold (34, 94).

3. The device according to Claim 1 or 2, **characterized in that**
the mold cavity (32, 92) is radially expandable, and preferably radially and uniformly expandable, at least in the region of the spherical surface-shaped inner surface, by the pressure imparted by the injected bone cement paste (50), and/or
the mold cavity (32, 92) is elastically expandable, and preferably rubber-elastically expandable, at least in the region of the spherical surface-shaped inner surface, by the pressure imparted by the injected bone cement paste (50).

4. The device according to any one of the preceding claims, **characterized in that** the stem mold (34, 94) and the mold cavity (32, 92) are connected or connectable to one another in a liquid-tight manner via a flange (14, 74) or an adapter element (102), wherein an expandable part of the mold cavity (32, 92), which comprises the spherical surface-shaped inner surface, is preferably fastened or fastenable with an annular mount (21, 81) to a flange (35, 95) of the stem mold (34, 94) or the adapter element (102), such that a peripheral annular disk (23, 83) of the expandable part of the mold cavity (32, 92) is arranged between the annular mount (21, 81) and the flange (35, 95) and seals the connection, wherein the annular mount (21, 81) is particularly preferably screwed or screwable to the flange (35, 95) of the stem mold (34, 94), and/or the device has a fastening element for fastening the mold cavity (32, 92) to the stem mold (34, 94), wherein the fastening element is preferably detachable and, particularly preferably, the fastening element is a plurality of screws (25, 85) or has a plurality of screws (25, 85), wherein the fastening element has clamping plates, wherein the fastening element preferably additionally has screws (25, 85), thumb screws, nuts and/or threaded rods.

5. The device according to any one of the preceding claims, **characterized in that**
the at least one filling opening (2, 62) is connected on a side remote from the casting mold (1, 61) to a port (11, 71, 171) for liquid-tight connection of a bone cement cartridge (10), wherein the port (11, 71, 171) is preferably suitable for pressure-tight connection of a bone cement cartridge (10), wherein the port (11, 71, 171) particularly preferably has a thread and very particularly preferably has a circumferential seal (13, 73) and/or a circumferential sealing face or a circumferential sealing edge, and/or
at least in the region of the spherical surface-shaped inner surface, the mold cavity (32, 92) expands by injection of further bone cement paste (50) into the casting mold (1, 61) already completely filled with bone cement paste (50), while the stem mold (34, 94) receives no additional bone cement paste (50) and remains dimensionally stable.

6. The device according to any one of the preceding claims, **characterized by** a valve seat (3, 63, 163), which is connected to the casting mold (1, 61) in the region of the at least one filling opening (2, 62), wherein the valve seat (3, 63, 163) has an in places closed head side (4, 64, 164) with at least one first feed-through (5, 65, 165), wherein the at least one first feed-through (5, 65, 165) opens into the at least one filling opening (2, 62),
a valve body (6, 66, 166) which is mounted so as to be rotatable relative to the valve seat (3, 63, 163) and which has a sealing face (7, 67, 167), wherein the sealing face (7, 67, 167) is oriented in the direction of the in places closed head side (4, 64, 164) of the valve seat (3, 63, 163), wherein at least one second feed-through (8, 68, 168) is arranged in the sealing face (7, 67, 167);
wherein the valve seat (3, 63, 163) and the valve body (6, 66, 166) together form a valve, wherein the valve is reversibly transferable into an open position and a closed position by rotation of the valve body (6, 66, 166) relative to the valve seat (3, 63, 163), wherein, in the open position of the valve, the at least one first feed-through (5, 65, 165) of the valve seat (3, 63, 163) and the at least one second feed-through (8, 68, 168) of the valve body (6, 66, 166) are located above one another at least in places and provide a connection permeable to bone cement (50) through the valve into the casting mold (1, 61), wherein, in the closed position of the valve, the at least one first feed-through (5, 65, 165) of the valve seat (3, 63, 163) is covered by the sealing face (7, 67, 167) of the valve body (6, 66, 166), wherein, in the closed position of the valve, the at least one filling opening (2, 62) of the casting mold (1, 61) is covered for bone cement paste (50).

7. The device according to Claim 6, **characterized in that**
the valve is connected on the side remote from the casting mold (1, 61) to a port (11, 71, 171) for liquid-tight connection of a bone cement cartridge (10) or the valve has such a port (11, 71, 171) and/or
the valve seat (3, 63, 163) is connected to the casting mold (1, 61) so as not to be rotatable relative to the casting mold (1, 61), preferably the valve seat (3, 63, 163) is firmly and/or rigidly connected to the casting mold (1, 61), and/or
the valve is manually operable, preferably manually operable from outside the device, wherein the valve body (6, 66, 166) is particularly preferably manually rotatable relative to the valve seat (3, 63, 163) and the valve is transferable by rotation from the closed position into the open position and from the open position into the closed position.

8. The device according to any one of Claims 6 or 7, **characterized in that** the valve seat (3, 63, 163) has an inner thread (20, 80, 180) on the inside and the valve body (6, 66, 166) has a matching outer thread (22, 82, 182) on the outside, such that the valve body (6, 66, 166) can be screwed into the valve seat (3, 63, 163) and/or the port (11, 71, 171) comprises, for liquid-tight connection of a bone cement cartridge (10), an inner thread (24, 84, 184) in the valve body (6, 66, 166) or an outer thread on the valve body (6, 66, 166), wherein an adapter element (9, 69) of the bone cement cartridge (10) or on the bone cement cartridge (10) preferably has a mating thread matching the inner thread (24, 84, 184) or the outer thread.

9. The device according to any one of the preceding claims, **characterized in that**
the casting mold (1, 61) has at least three or four cavities, starting from an inner chamber of the casting mold (1, 61), for receiving retaining pins, wherein the cavities are preferably arranged in the stem mold (34, 94) and the stem mold (34, 94) is particularly preferably in two parts or three parts and the cavities are arranged in edges or in longitudinal flanges (14, 74) of at least one part of the two-part stem mold (34, 94), and
the device has a metal core (16, 76) which is to be arranged in the casting mold (1, 61), wherein the metal core (16, 76) preferably has bores for receiving retaining pins (18, 78), wherein those bores (18, 78) which are to be arranged in the stem mold (34, 94) are particularly preferably arranged within the part of the metal core (16, 76), and/or the spherical surface-shaped inner surface of the mold cavity (32, 92) has, in the unexpanded state, a diameter of at least 35 mm or of at least 40 mm and preferably of between 40 mm and 50 mm, and/or, in the maximally expanded state, has a diameter of at most 70 mm and preferably of between 60 mm and 70 mm, wherein, in the unexpanded state, the diameter of the spherical surface-shaped inner surface of the mold cavity (32, 92) is smaller than the diameter of the spherical surface-shaped inner surface of the mold cavity (32, 92) in the expanded state.

10. The device according to any one of the preceding claims, **characterized in that**
the mold cavity (32, 92) consists, at least in the region of the spherical surface-shaped inner surface, of a rubber-elastic material, preferably of rubber, silicone rubber, a synthetic rubber or an ethylene-propylene-diene rubber (EPDM), and/or
the at least one filling opening (2, 62) contains a shut-off element which, in a closed state, prevents bone cement paste (50) from flowing out of the casting mold (1, 61) through the at least one filling opening (2, 62).

11. The device according to any one of the preceding claims, **characterized in that**
the stem mold (94) comprises a length-variable adapter element (102) with which the length of the neck (126, 136) of the spacer (120, 130) is able to be varied, said neck connecting the stem (124, 134) to the head (122, 132) of the spacer (120, 130), wherein the adapter element (102) is preferably able to be varied in length by a screw connection.

12. The device according to any one of the preceding claims, **characterized in that**
the device has a plurality of dimensionally stable mating molds which are suitable for receiving the mold cavity (32, 92), wherein the dimensionally stable mating molds enable a different degree of expansion of the mold cavity (32, 92), such that, at least in the region of the spherical surface-shaped inner surface, expansion of the mold cavity (32, 92) is limited by the dimensionally stable mating molds to different diameters when the mold cavity (32, 92) is inserted into the respective dimensionally stable mating mold, wherein the dimensionally stable mating molds are preferably embodied by at least one blister pack or a plastics shell with one or a plurality of indentations as the dimensionally stable mating molds, and/or
the device has a check gage or a vernier caliper for measuring the current diameter of the spherical surface-shaped inner surface of the mold cavity (32, 92), wherein the check gage or vernier caliper may be placed or is arranged on the outside of the mold cavity (32, 92) and wherein the diameter of the spherical surface-shaped inner surface of the mold cavity (32, 92) is preferably directly readable.

13. A method for producing a spacer (120, 130) for temporarily replacing a joint or part of a joint, in particular a hip joint or a shoulder joint, comprising an articulating surface of the joint, wherein the method is carried out with the device according to any one of Claims 1 to 12, the method having the following chronological steps:
A) injecting bone cement paste (50) through the at least one filling opening (2, 62) into the casting mold (1, 61) and simultaneously displacing air from the casting mold (1, 61) through the at least one vent element (15, 75) by injection of the bone cement paste (50);
B) further injecting bone cement paste (50) though the at least one filling opening (2, 62) into the casting mold (1, 61), wherein injection of the bone cement paste (50) expands the mold cavity (32, 92) at least in the region of the spherical surface-shaped inner surface, while the stem mold (34, 94) remains dimensionally stable;
C) curing the bone cement paste (50) in the casting mold (1, 61); and
D) removing the resultant molded and cured spacer (120, 130) from the casting mold (1,61).

14. The method according to Claim 13, **characterized in that**,
prior to step A), a bone cement cartridge (10) is connected in liquid-tight manner to a port (11, 71, 171) of the device, wherein the port (11, 71, 171) is connected to the at least one filling opening (2, 62) in a liquid-permeable manner and in step A) the bone cement paste (50) is pressed out of the bone cement cartridge (10) into the casting mold (1, 61),
wherein preferably a device according to any one of Claims 6 to 8 with a valve is used, wherein bone cement paste (50) is injected in step A) through the valve in the open position into the casting mold (1, 61), wherein a step B1) proceeds after step B) and before step C):
B1) rotating the valve body (6, 66, 166) relative to the valve seat (3, 63, 163) and so transferring the valve into the closed position and shearing off the bone cement paste (50) at the at least one first feed-through (5, 65, 165) in the in places closed head side (4, 64, 164) of the valve seat (3, 63, 163) by rotation of the valve body (6, 66, 166) relative to the valve seat (3, 63, 163), wherein a bone cement cartridge (10) is then preferably detached from a port (11, 71, 171) which is connected to the at least one filling opening (2, 62) in a liquid-permeable manner.

15. The method according to Claim 14, **characterized in that** the following intermediate steps proceed after step B1) and before step C):
B2) connecting a new bone cement cartridge (10) to the port (11, 71, 171) of the device in a liquid-tight manner, wherein bone cement paste (50) or starting components for producing the bone cement paste (50) is or are present in the new bone cement cartridge (10);
B3) rotating the valve body (6, 66, 166) relative to the valve seat (3, 63, 163) and so transferring the valve into the open position;
B4) injecting the bone cement paste (50) from the new bone cement cartridge (10) through the valve in the open position into the casting mold (1, 61);
B5) rotating the valve body (6, 66, 166) relative to the valve seat (3, 63, 163) and so transferring the valve into the closed position and shearing off the bone cement paste (50) at the at least one first feed-through (5, 65, 165) in the in places closed head side (4, 64, 164) of the valve seat (3, 63, 163) by rotation of the valve body (6, 66, 166) relative to the valve seat (3, 63, 163); and
B6) detaching the new bone cement cartridge (10) from the port (11, 71, 171); wherein steps B2) to B6) are preferably repeated once or multiple times with in each case new bone cement cartridges which contain bone cement paste (50) or the starting components thereof until the casting mold (1, 61) is completely filled with bone cement paste (50) and furthermore until, with the assistance of the bone cement paste (50), the mold cavity (32, 92) is expanded to the desired size at least in the region of the spherical surface-shaped inner surface.

16. The method according to any one of Claims 13 to 15, **characterized in that**
the mold cavity (32, 92) of the casting mold (1, 61) is inserted into one of a plurality of dimensionally stable mating molds, wherein the dimensionally stable mating molds enable a different degree of expansion of the mold cavity (32, 92), such that, at least in the region of the spherical surface-shaped inner surface, expansion of the mold cavity (32, 92) is limited by the dimensionally stable mating mold used to a specific diameter while the bone cement paste (50) is pressed into the casting mold (1, 61) in order to expand the mold cavity (32, 92), and/or
a check gage or a vernier caliper for measuring the current diameter of the spherical surface-shaped inner surface of the mold cavity (32, 92) is used to read off the current diameter of the spherical surface-shaped inner surface of the mold cavity (32, 92), wherein injection of the bone cement paste (50) into the casting mold (1, 61) is preferably stopped once a desired diameter is reached.

## Revendications

1. Dispositif pour la fabrication d'un espaceur (120, 130) par le durcissement de pâte de ciment osseux (50), l'espaceur (120, 130) étant prévu pour remplacer temporairement, dans le domaine médical, une articulation ou une partie d'une articulation comprenant une surface articulaire d'une tête de l'articulation, en particulier une articulation de la hanche ou une articulation de l'épaule, le dispositif présentant
un moule de coulée (1, 61) pour la formation de l'espaceur (120, 130) en pâte de ciment osseux (50), le moule de coulée (1, 61) présentant une forme de tige (34, 94) pour la formation d'une tige (124, 134) et d'un col (126, 136) et le moule de coulée (1, 61) présentant une forme creuse (32, 92) comprenant une surface interne de forme sphérique pour la formation d'une surface de glissement d'une tête (122, 132) de l'espaceur (120, 130), la forme de tige (34, 94) et la forme creuse (32, 92) limitant un espace interne commun, de telle sorte que la tête (122, 132) d'un espaceur (120, 130) formé avec le moule de coulée (1, 61) soit connectée d'une pièce à la tige (124, 134) à travers le col (126, 136), au moins une ouverture de remplissage (2, 62) pour l'injection d'une pâte de ciment osseux (50) dans le moule de coulée (1, 61),
au moins un élément de ventilation (15, 75) qui connecte l'espace interne du moule de coulée (1, 61) de manière perméable aux gaz à l'environnement du moule de coulée (1, 61), la forme de tige (34, 94) étant de forme stable lors d'une injection de pâte de ciment osseux (50) dans le moule de coulée (1, 61), **caractérisé en ce que,**
lors de l'injection de la pâte de ciment osseux (50) dans le moule de coulée (1, 61), la forme creuse (32, 92) est expansible au moins dans la zone de la surface interne de forme sphérique sous une pression transmise par la pâte de ciment osseux (50) injectée.

2. Dispositif selon la revendication 1, **caractérisé en ce que**
l'au moins un élément de ventilation (15, 75) est perméable pour des gaz et imperméable pour de la pâte de ciment osseux (50), en particulier est imperméable pour de la pâte de ciment osseux en poly(méthacrylate de méthyle) (50) (pâte de ciment osseux en PMMA), et/ou
l'au moins un élément de ventilation (15, 75) est disposé dans la forme creuse (32, 92) ou l'au moins un élément de ventilation (15, 75) sont plusieurs éléments de ventilation, au moins un des plusieurs éléments de ventilation étant disposé dans la forme creuse (32, 92) et au moins un des plusieurs éléments de ventilation étant disposé dans la forme de tige (34, 94).

3. Dispositif selon la revendication 1 ou 2, **caractérisé en ce que**
la forme creuse (32, 92) est expansible radialement, est de préférence expansible radialement et uniformément, au moins dans la zone de la surface interne de forme sphérique sous une pression transmise par la pâte de ciment osseux (50) injectée, et/ou la forme creuse (32, 92) est expansible de manière élastique, est de préférence expansible de manière élastique comme du caoutchouc, au moins dans la zone de la surface interne de forme sphérique sous une pression transmise par la pâte de ciment osseux (50) injectée.

4. Dispositif selon l'une quelconque des revendications précédentes, **caractérisé en ce que** la forme de tige (34, 94) et la forme creuse (32, 92) sont ou peuvent être connectées l'une à l'autre de manière étanche aux liquides à travers une bride (14, 74) ou un élément adaptateur (102), une partie expansible de la forme creuse (32, 92), qui comprend la surface interne de forme sphérique, étant ou pouvant être de préférence fixée à un support annulaire (21, 81) sur une bride (35, 95) de la forme de tige (34, 94) ou de l'élément adaptateur (102), de telle sorte qu'une rondelle annulaire (23, 83) côté bord de la partie expansible de la forme creuse (32, 92) étant disposée entre le support annulaire (21, 81) et la bride (35, 95) et étanchéifiant la connexion, le support annulaire (21, 81) étant ou pouvant été encore plus préférablement vissé à la bride (35, 95) de la forme de tige (34, 94), et/ou le dispositif présente un élément de fixation pour la fixation de la forme creuse (32, 92) à la forme de tige (34, 94), l'élément de fixation étant de préférence amovible et l'élément de fixation encore plus préférablement étant plusieurs vis (25, 85) ou présentant plusieurs vis (25, 85), l'élément de fixation présentant des plaques de serrage, l'élément de fixation présentant de préférence en plus des vis (25, 85), des vis à oreilles, des écrous et/ou des tiges filetées.

5. Dispositif selon l'une quelconque des revendications précédentes, **caractérisé en ce que** l'au moins une ouverture de remplissage (2, 62) est connectée sur un côté opposé au moule de coulée (1, 61) à un raccord (11, 71, 171) pour la connexion étanche aux liquides d'une cartouche de ciment osseux (10), le raccord (11, 71, 171) étant de préférence approprié pour la connexion étanche à la pression d'une cartouche de ciment osseux (10), le raccord (11, 71, 171) présentant encore plus préférablement un filetage et de manière particulièrement préférée un joint périphérique (13, 73) et/ou une surface d'étanchéité périphérique ou un bord d'étanchéité périphérique, et/ou la forme creuse (32, 92) se dilate au moins dans la zone de la surface interne de forme sphérique par l'injection de pâte de ciment osseux (50) supplémentaire dans le moule de coulée (1, 61) déjà rempli de pâte de ciment osseux (50), tandis que la forme de tige (34, 94) ne reçoit par de pâte de ciment osseux (50) supplémentaire et reste de forme stable.

6. Dispositif selon l'une quelconque des revendications précédentes, **caractérisé par** un siège de soupape (3, 63, 163), lequel est connecté au moule de coulée (1, 61) dans la zone de l'au moins une ouverture de remplissage (2, 62), le siège de soupape (3, 63, 163) présentant un côté de tête (4, 64, 164) fermé par zones comprenant au moins une première conduite (5, 65, 165), l'au moins une première conduite (5, 65, 165) débouchant dans l'au moins une ouverture de remplissage (2, 62),
un corps de soupape (6, 66, 166), lequel est monté tournant par rapport au siège de soupape (3, 63, 163) et lequel présente une surface d'étanchéité (7, 67, 167), la surface d'étanchéité (7, 67, 167) étant orientée dans la direction du côté de tête (4, 64, 164) fermé par zones du siège de soupape (3, 63, 163), au moins une deuxième conduite (8, 68, 168) étant disposée dans la surface d'étanchéité (7, 67, 167) ;
le siège de soupape (3, 63, 163) et le corps de soupape (6, 66, 166) formant ensemble une soupape, la soupape pouvant être passée, par la rotation du corps de soupape (6, 66, 166) par rapport au siège de soupape (3, 63, 163), de manière réversible dans une position ouverte et de manière réversible dans une position fermée, dans la position ouverte de la soupape, l'au moins une première conduite (5, 65, 165) du siège de soupape (3, 63, 163) et l'au moins une deuxième conduite (8, 68, 168) du corps de soupape (6, 66, 166) étant placées au moins par zones l'une au-dessus de l'autre et fournissant une connexion perméable à la pâte de ciment osseux (50) à travers la soupape dans le moule de coulée (1, 61), dans la position fermée de la soupape, l'au moins une première conduite (5, 65, 165) du siège de soupape (3, 63, 163) étant couverte par la surface d'étanchéité (7, 67, 167) du corps de soupape (6, 66, 166), l'au moins une ouverture de remplissage (2, 62) du moule de coulée (1, 61) étant couverte pour la pâte de ciment osseux (50) dans la position fermée de la soupape.

7. Dispositif selon la revendication 6, **caractérisé en ce que**
la soupape est connectée sur le côté opposé au moule de coulée (1, 61) à un raccord (11, 71, 171) pour la connexion étanche aux liquides d'une cartouche de ciment osseux (10) ou que la soupape présente un tel raccord (11, 71, 171) et/ou le siège de soupape (3, 63, 163) est connecté au moule de coulée (1, 61) de manière non rotative par rapport au moule de coulée (1, 61), le siège de soupape (3, 63, 163) est de préférence connecté de manière fixe et/ou rigide au moule de coulée (1, 61), et/ou la soupape peut être actionnée manuellement, peut de préférence être actionnée manuellement depuis l'extérieur du dispositif, le corps de soupape (6, 66, 166) pouvant de manière particulièrement préférée tourner manuellement par rapport au siège de soupape (3, 63, 163) et la soupape pouvant être transférée, par la rotation, de la position fermée à la position ouverte et de la position ouverte à la position fermée.

8. Dispositif selon l'une quelconque des revendications 6 ou 7, **caractérisé en ce que** le siège de soupape (3, 63, 163) présente un filetage interne (20, 80, 180) sur la face interne et le corps de soupape (6, 66, 166) présente un filetage externe (22, 82, 182) correspondant sur la face externe, de sorte que le corps de soupape (6, 66, 166) puisse être vissé dans le siège de soupape (3, 63, 163) et/ou
le raccord (11, 71, 171) pour la connexion étanche aux liquides d'une cartouche de ciment osseux (10) comprend un filetage interne (24, 84, 184) dans le corps de soupape (6, 66, 166) ou un filetage externe sur le corps de soupape (6, 66, 166), un élément adaptateur (9 69) de la cartouche de ciment osseux (10) ou sur la cartouche de ciment osseux (10) présentant de préférence un contre-filetage correspondant au filetage interne (24, 84, 184) ou au filetage externe.

9. Dispositif selon l'une quelconque des revendications précédentes, **caractérisé en ce que** le moule de coulée (1, 61) présente, à partir d'un espace interne du moule de coulée (1, 61), au moins trois ou quatre cavités pour la réception de chevilles, les cavités étant de préférence disposées dans la forme de tige (34, 94) et la forme de tige (34, 94) étant constituée de deux ou de trois parties et les cavités étant disposées dans les bords ou dans les brides longitudinales (14, 74) d'au moins une partie de la forme de tige (34, 94) constituée de deux parties, et
le dispositif présente un noyau métallique (16, 76), lequel doit être disposé dans le moule de coulée (1, 61), le noyau métallique (16, 76) possédant de préférence des alésages pour la réception de chevilles (18, 78), les alésages (18, 78) qui doivent être disposés dans la forme de tige (34, 94) étant encore plus préférablement disposés à l'intérieur de la partie du noyau métallique (16, 76), et/ou
la surface interne de forme sphérique de la forme creuse (32, 92) a, à l'état non dilaté, un diamètre d'au moins 35 mm ou d'au moins 40 mm, de préférence situé entre 40 mm et 50 mm, et a, à l'état dilaté au maximum, un diamètre d'au plus 70 mm, de préférence situé entre 60 mm et 70 mm, le diamètre de la surface interne de forme sphérique de la forme creuse (32, 92) à l'état non dilaté étant inférieur au diamètre de la surface interne de forme sphérique de la forme creuse (32, 92) à l'état dilaté.

10. Dispositif selon l'une quelconque des revendications précédentes, **caractérisé en ce que** la forme creuse (32, 92) est constituée, au moins dans la zone de la surface interne de forme sphérique, d'un matériau élastique comme du caoutchouc, de préférence de caoutchouc, de caoutchouc de silicone, d'un caoutchouc de synthèse ou d'un caoutchouc éthylène-propylène-diène (EPDM), et/ou
l'au moins une ouverture de remplissage (2, 62) contient un élément de verrouillage, lequel empêche, à l'état fermé, une fuite de la pâte de ciment osseux (50) du moule de coulée (1, 61) à travers l'au moins une ouverture de remplissage (2, 62).

11. Dispositif selon l'une quelconque des revendications précédentes, **caractérisé en ce que** la forme de tige (94) comprend un élément adaptateur (102) variable dans la longueur, au moyen duquel la longueur du col (126, 136) de l'espaceur (120, 130), qui connecte la tige (124, 134) à la tête (122, 132) de l'espaceur (120, 130), peut être variée, l'élément adaptateur (102) pouvant de préférence être varié dans la longueur au moyen d'un raccord vissé.

12. Dispositif selon l'une quelconque des revendications précédentes, **caractérisé en ce que** le dispositif présente plusieurs contre-formes de forme stable, lesquelles sont adaptées pour la réception de la forme creuse (32, 92), les contre-formes de forme stable permettant une dilatation de différente largeur de la forme creuse (32, 92), de telle sorte que la dilatation de la forme creuse (32, 92) est limitée, au moins dans la zone de la surface interne de forme sphérique, jusqu'à des diamètres différents par les contre-formes de forme stable, lorsque la forme creuse (32, 92) est insérée dans la contre-forme de forme stable respective, les contre-formes de forme stable étant de préférence réalisées par au moins un emballage blister ou une coque en matière plastique comprenant une ou plusieurs cavités en tant que contre-formes de forme stable, et/ou le dispositif présente un calibre de contrôle ou un pied à coulisse pour la mesure du diamètre actuel de la surface interne de forme sphérique de la forme creuse (32, 92), le calibre de contrôle ou le pied à coulisse pouvant être appuyé contre ou étant disposé sur l'extérieur de la forme creuse (32, 92) et le diamètre de la surface interne de forme sphérique de la forme creuse (32, 92) étant de préférence directement lisible.

13. Procédé de fabrication d'un espaceur (120, 130) pour le remplacement temporaire d'une articulation ou d'une partie d'une articulation, en particulier une articulation de la hanche ou une articulation de l'épaule, comprenant une surface articulaire de l'articulation, le procédé étant exécuté avec un dispositif selon l'une quelconque des revendications 1 à 12, le procédé présentant les étapes chronologiques suivantes :
A) injection de pâte de ciment osseux (50) à travers l'au moins une ouverture de remplissage (2, 62) dans le moule de coulée (1, 61) et simultanément expulsion d'air du moule de coulée (1, 61) à travers l'au moins un élément de ventilation (15, 75) par l'injection de la pâte de ciment osseux (50) ;
B) injection supplémentaire de pâte de ciment osseux (50) à travers l'au moins une ouverture de remplissage (2, 62) dans le moule de coulée (1, 61), la forme creuse (32, 92) se dilatant au moins dans la zone de la surface interne de forme sphérique dû à l'injection de la pâte de ciment osseux (50), tandis que la forme de tige (34, 94) reste de forme stable ;
C) durcissement de la pâte de ciment osseux (50) dans le moule de coulée (1, 61) ; et
D) retrait de l'espaceur (120, 130) ainsi formé et durci du moule de coulée (1, 61).

14. Procédé selon la revendication 13, **caractérisé en ce que**
avant l'étape A), une connexion étanche aux liquides d'une cartouche de ciment osseux (10) à un raccord (11, 71, 171) du dispositif est effectuée, le raccord (11, 71, 171) étant connecté de manière perméable aux liquides à l'au moins une ouverture de remplissage (2, 62) et dans l'étape A), la pâte de ciment osseux (50) est injectée à partir de la cartouche de ciment osseux (10) dans le moule de coulée (1, 61),
un dispositif selon l'une quelconque des revendications 6 à 8 comprenant une soupape étant de préférence utilisé, l'injection de pâte de ciment osseux (50) dans l'étape A) étant effectuée à travers la soupape en position ouverte dans le moule de coulée (1, 61), une étape B1) étant effectuée après l'étape B) et avant l'étape C) :
B1) rotation du corps de soupape (6, 66, 166) par rapport au siège de soupape (3, 63, 163) et ainsi transfert de la soupape à la position fermée et découpe de la pâte de ciment osseux (50) au niveau de l'au moins une première conduite (5, 65, 165) dans le côté de tête (4, 64, 164) au moins fermé par zones du siège de soupape (3, 63, 163) par la rotation du corps de soupape (6, 66, 166) par rapport au siège de soupape (3, 63, 163), une cartouche de ciment osseux (10) étant de préférence ensuite détachée d'un raccord (11, 71, 171), lequel est connecté de manière perméable aux liquides à l'au moins une ouverture de remplissage (2, 62).

15. Procédé selon la revendication 14, **caractérisé en ce que**
les étapes intermédiaires suivantes sont effectuées après l'étape B1) et avant l'étape C) :
B2) connexion étanche aux liquides d'une nouvelle cartouche de ciment osseux (10) au raccord (11, 71, 171) du dispositif, de la pâte de ciment osseux (50) ou des composants initiaux pour la fabrication de pâte de ciment osseux (50) étant contenus dans la nouvelle cartouche de ciment osseux (10) ;
B3) rotation du corps de soupape (6, 66, 166) par rapport au siège de soupape (3, 63, 163) et ainsi transfert de la soupape dans la position ouverte ;
B4) injection de la pâte de ciment osseux (50) de la nouvelle cartouche de ciment osseux (10) à travers la soupape dans la position ouverte dans le moule de coulée (1, 61) ;
B5) rotation du corps de soupape (6, 66, 166) par rapport au siège de soupape (3, 63, 163) et ainsi transfert de la soupape dans la position fermée et découpe de la pâte de ciment osseux (50) au niveau de l'au moins une première conduite (5, 65, 165) dans le côté de tête (4, 64, 164) fermé par zones du siège de soupape (3, 63,163) par la rotation du corps de soupape (6, 66, 166) par rapport au siège de soupape (3, 63, 163) ; et B6) détachement de la nouvelle cartouche de ciment osseux (10) du raccord (11, 71, 171), les étapes B2) à B6) étant de préférence répétées une ou plusieurs fois avec respectivement de nouvelles cartouches de ciment osseux, qui contiennent de la pâte de ciment osseux (50) ou ses composants initiaux, jusqu'à ce que le moule de coulée (1, 61) soit complètement rempli de pâte de ciment osseux (50) et en outre jusqu'à ce que la forme creuse (32, 92) se soit dilatée au moins dans la zone de la surface interne de forme sphérique jusqu'à la taille souhaitée à l'aide de la pâte de ciment osseux (50).

16. Procédé selon l'une quelconque des revendications 13 à 15, **caractérisé en ce que** la forme creuse (32, 92) du moule de coulée (1, 61) est insérée dans une de plusieurs contre-formes de forme stable, les contre-formes de forme stable permettant une dilatation de différente largeur de la forme creuse (32, 92), de telle sorte que la dilatation de la forme creuse (32, 92) au moins dans la zone de la surface interne de forme sphérique soit limitée jusqu'à un diamètre déterminé par la contre-forme de forme stable pendant que la pâte de ciment osseux (50) est injectée dans le moule de coulée (1, 61) pour faire dilater la forme creuse (32, 92), et/ou
un calibre de contrôle ou un pied à coulisse pour la mesure du diamètre actuel de la surface interne de forme sphérique de la forme creuse (32, 92) est utilisé pour lire le diamètre actuel de la surface interne de forme sphérique de la forme creuse (32, 92), l'injection de la pâte de ciment osseux (50) dans le moule de coulée (1, 61) étant de préférence arrêtée lorsque le diamètre souhaité est atteint.
